# EUROPEAN PATENT APPLICATION

(11) **EP 3 799 054 A1**
(43) Date of publication of application: **31.03.2021**
(21) Application number: 20197191.8
(22) Date of filing: 21.09.2020
(51) Int. Cl.: G16H 10/40, G06Q 10/06, G16H 10/60

(54) **METHOD FOR MANAGING TEST REQUEST BY COMPUTER, MANAGEMENT DEVICE, MANAGEMENT COMPUTER PROGRAM, AND MANAGEMENT SYSTEM**

(30) Priority: 30.09.2019 JP 2019180791
(71) Applicant: SYSMEX CORPORATION, Kobe-shi Hyogo 651-0073 (JP)
(72) Inventor: Takahata, Takayuki, Hyogo, 651-0073 (JP); Ikeda, Koji, Hyogo, 651-0073 (JP); Suzuki, Kenichiro, Hyogo, 651-0073 (JP); Onoe, Hiroko, Hyogo, 651-0073 (JP)
(74) Representative: Hoffmann Eitle

(57) **Abstract**

Disclosed is a method for managing a test request for gene panel testing by a computer, the method including: acquiring, for each of a plurality of test requests, information regarding the test request and an attribute of a test result in the gene panel testing; and outputting display information for displaying a plurality of the test requests and the attribute in association with each other.

## Description

### FIELD OF THE INVENTION

The present specification discloses a method for managing a test request by a computer, a management device for managing a test request for gene panel testing, a computer program for managing a test request for gene panel testing, and a management system for managing a test request for gene panel testing.

### BACKGROUND

In recent years, in cancer treatment, research on cancer genomic medicine is being promoted in which, for each patient, gene panel testing is performed that can comprehensively investigate many gene mutations at once with a next-generation sequencer and the like, to formulate a treatment strategy suitable for each patient based on a result.

However, in general, a patient's electronic medical record, pathological image, and various test results of gene panel testing that serve as a reference in formulating a treatment strategy suitable for each patient are individually managed by different systems in a medical facility. WO2017/042396 discloses an information platform that supports formulation of a treatment plan for a patient by aggregating an electronic medical record, a pathological image, a test result such as of gene panel testing, and the like dispersed in a medical facility.

In genomic medicine, a treatment strategy is formulated by holding an expert meeting including a genetic counselor, a molecular genetics researcher, a clinical technologist, a bioinformatician, and the like, in addition to a doctor in charge of a patient and a pathologist. In the expert meeting, different treatment strategies may be proposed depending on what mutation has been detected or has not been detected in the gene panel testing. When there are a plurality of test requests, urgency of treatment may differ depending on test results.

Under such circumstances, the number of requests is expected to increase further in the future, and therefore a system for improving efficiency of the entire operation of genomic medicine that handles a plurality of test requests is required.

Therefore, an object is to provide a method for improving efficiency of the entire operation of genomic medicine that handles a plurality of test requests.

### SUMMARY OF THE INVENTION

One embodiment of the present invention relates to (1) a method for managing a test request for gene panel testing by a computer. The method includes: acquiring, for each of a plurality of test requests, information regarding the test request and attribute information of a test result in the gene panel testing; and outputting display information for displaying a plurality of the test requests and the attribute information in association with each other.

Such a configuration makes it possible to integrate and display information regarding a test request and attribute information of a test result, which have been managed by different computers before, on one graphical user interface.

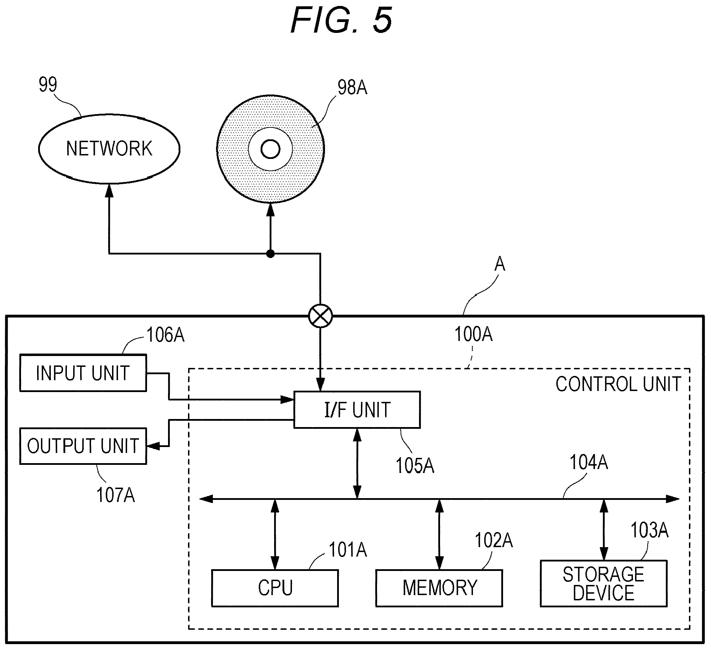

One embodiment of the present invention relates to (2) a management device (A) for managing a test request for gene panel testing. The management device (A in FIG. 5) includes a control unit (100A in FIG. 5). The control unit (100A in FIG. 5) acquires, for each of a plurality of test requests, information regarding the test request and attribute information of a test result in the gene panel testing, and outputs display information for displaying a plurality of the test requests and the attribute information in association with each other.

One embodiment of the present invention relates to (3) a non-transitory computer-readable storage medium storing a computer program for managing a test request for gene panel testing, the computer program. The computer program, which when read and executed, causes a computer to perform operations comprising: acquiring, for each of a plurality of test requests, information regarding the test request, and attribute information of a test result in gene panel testing corresponding to the test request (step ST21 of FIG. 19 and ST35 of FIG. 20); and outputting display information for displaying the plurality of the test requests and the attribute information in association with each other (step ST40 in FIG. 21).

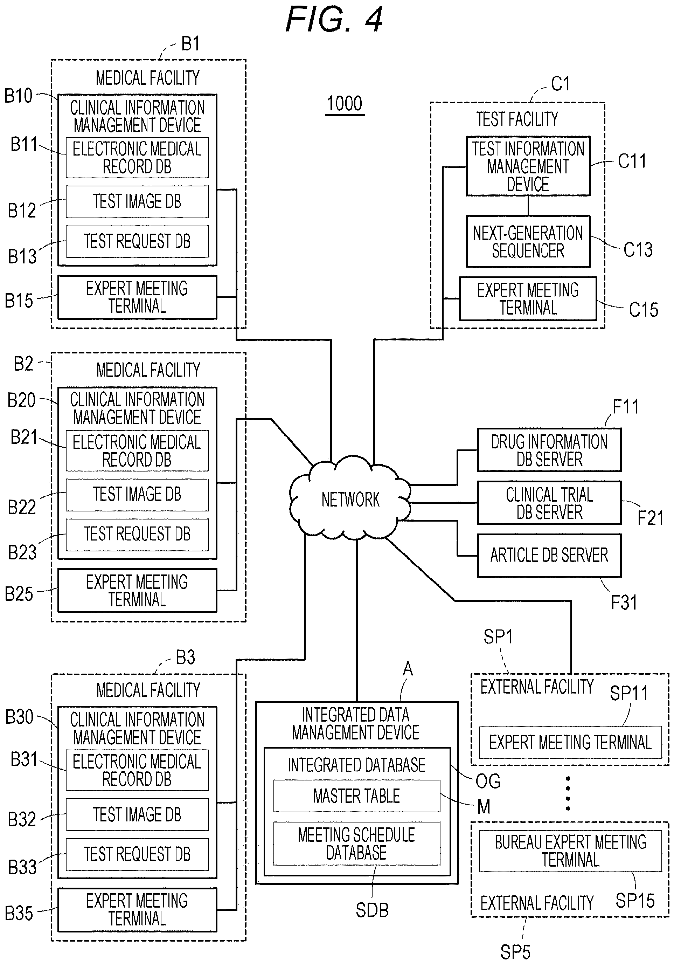

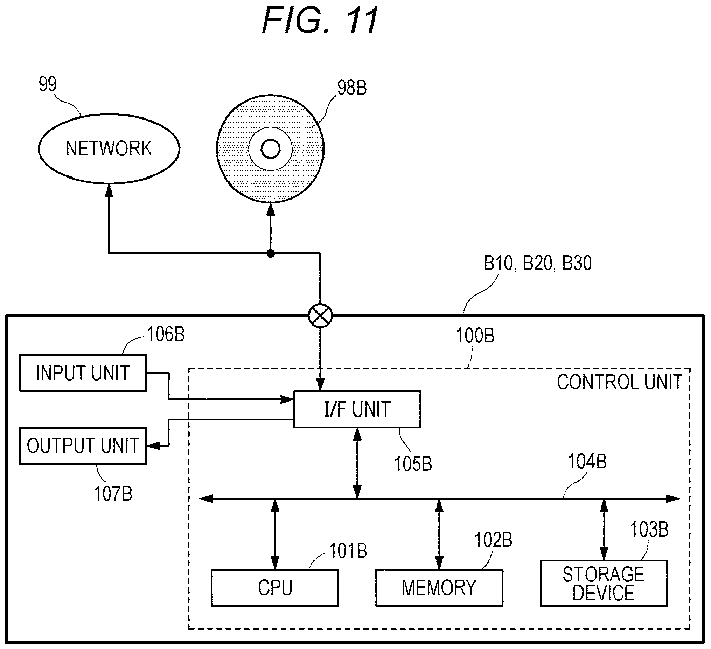

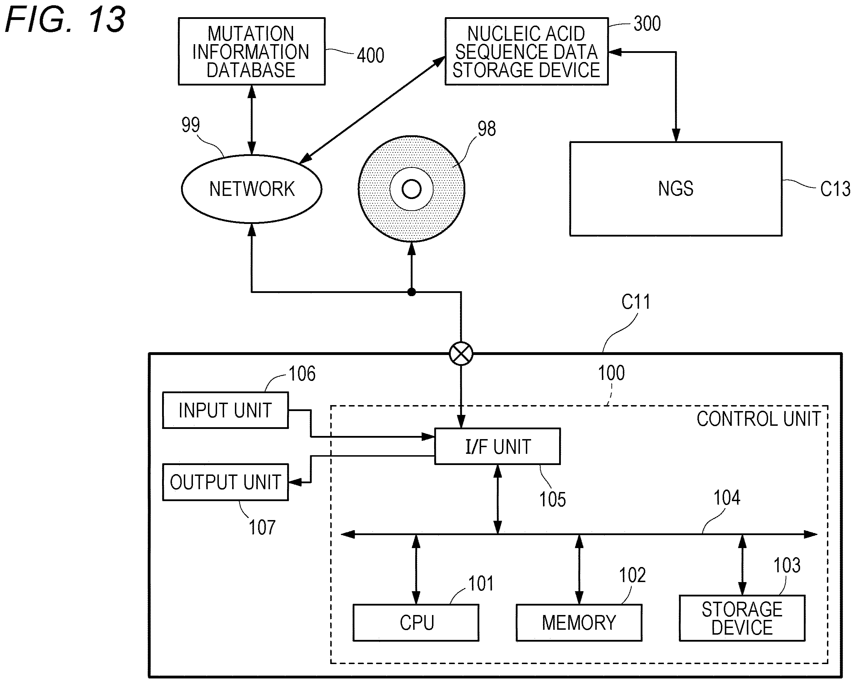

One embodiment of the present invention relates to (4) a management system (1000 in FIG. 4) for managing a test request for gene panel testing. The management system (1000 in FIG. 4) includes: a first computer (B10 in FIG. 11) including a control unit (100B in FIG. 11); a second computer (C11 in FIG. 13) including a control unit (100 in FIG. 13); and a management device (A in FIG. 5) including a control unit (100A in FIG. 5). The control unit of the first computer transmits information regarding a plurality of test requests to the management device, the control unit of the second computer transmits attribute information of a test result in the gene panel testing to the management device, and the control unit of the management device outputs display information for displaying a plurality of the test requests and the attribute information that have been received, in association with each other.

The configurations of the above (1) to (4) make it possible to integrate and display information regarding a test request and attribute information of a test result, which have been managed by different computers before, on one graphical user interface.

It is possible to improve efficiency of the entire operation of genomic medicine that handles a plurality of test requests.

### BRIEF DESCRIPTION OF THE DRAWINGS

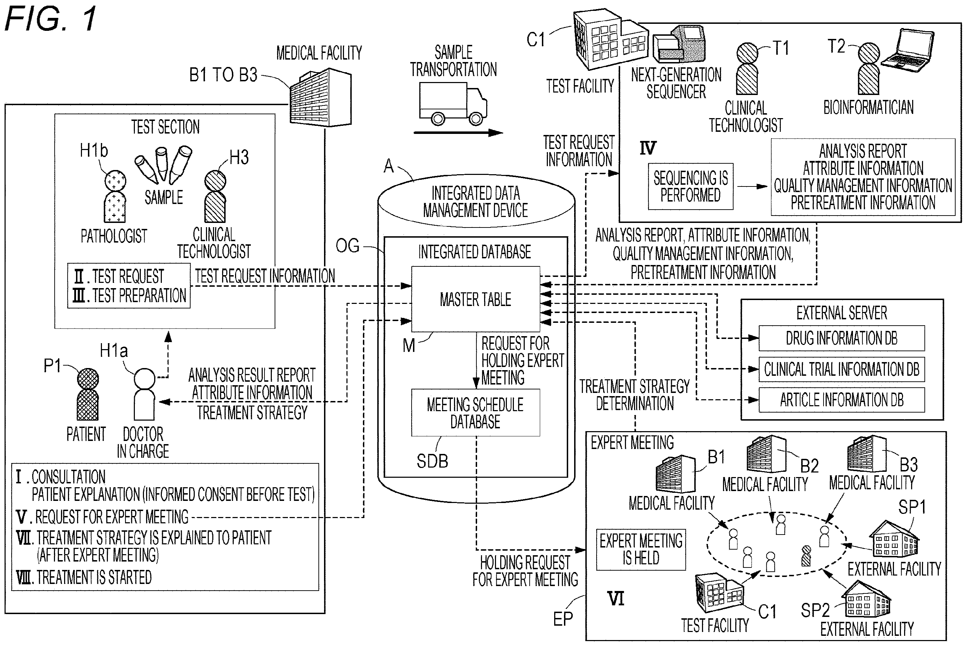

Fig. 1 shows a flow of gene panel testing.

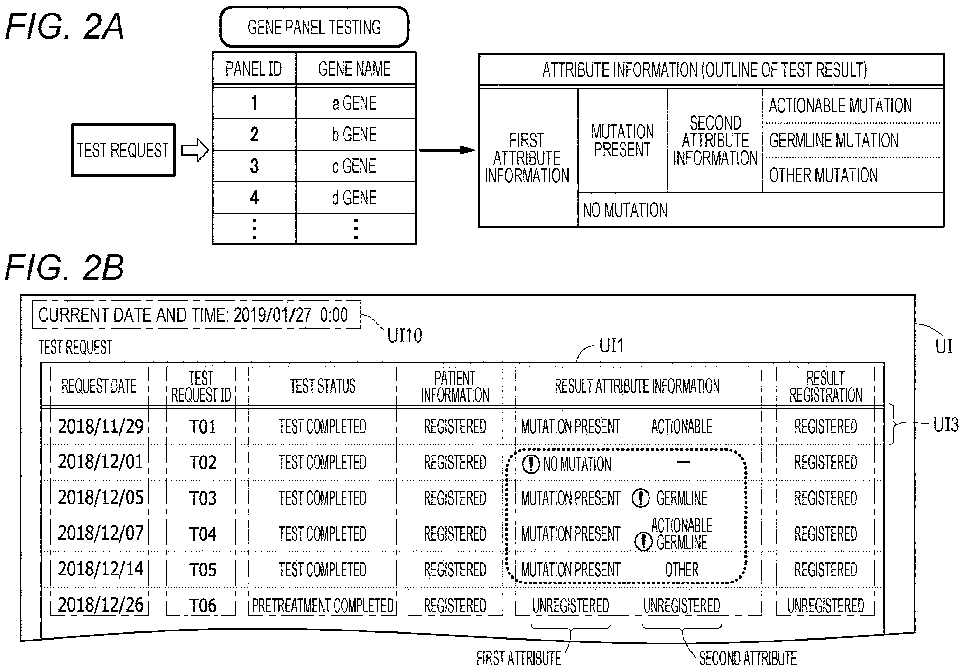

Fig. 2A shows a flow from a gene panel testing request to acquisition of attribute information. Fig. 2B shows a display example of a test request list.

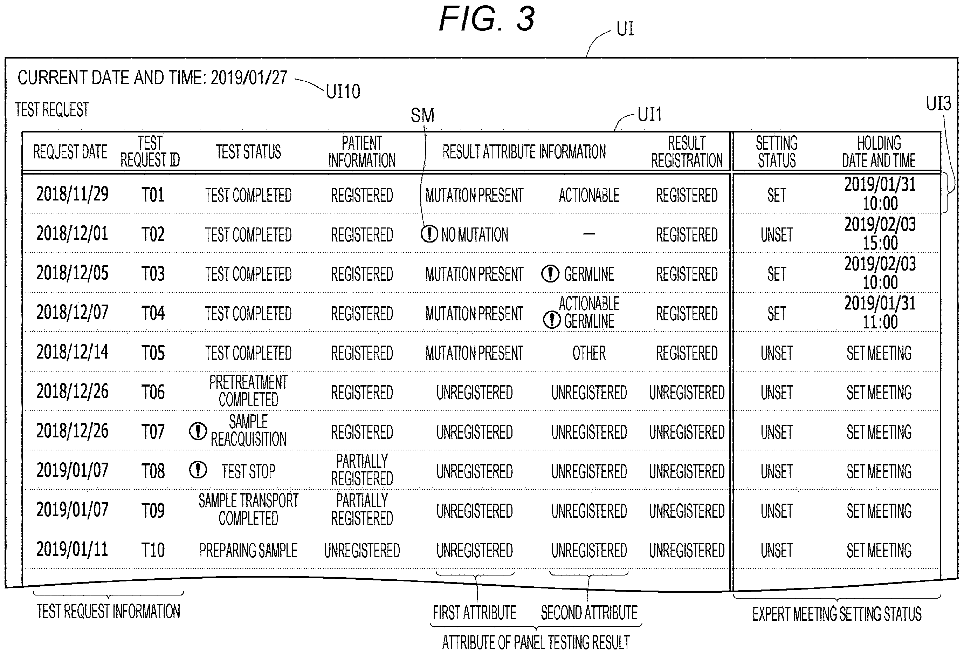

Fig. 3 shows a display example of a test request list.
Fig. 4 shows a hardware configuration of a system 1000.
Fig. 5 shows a hardware configuration of an integrated data management device A.

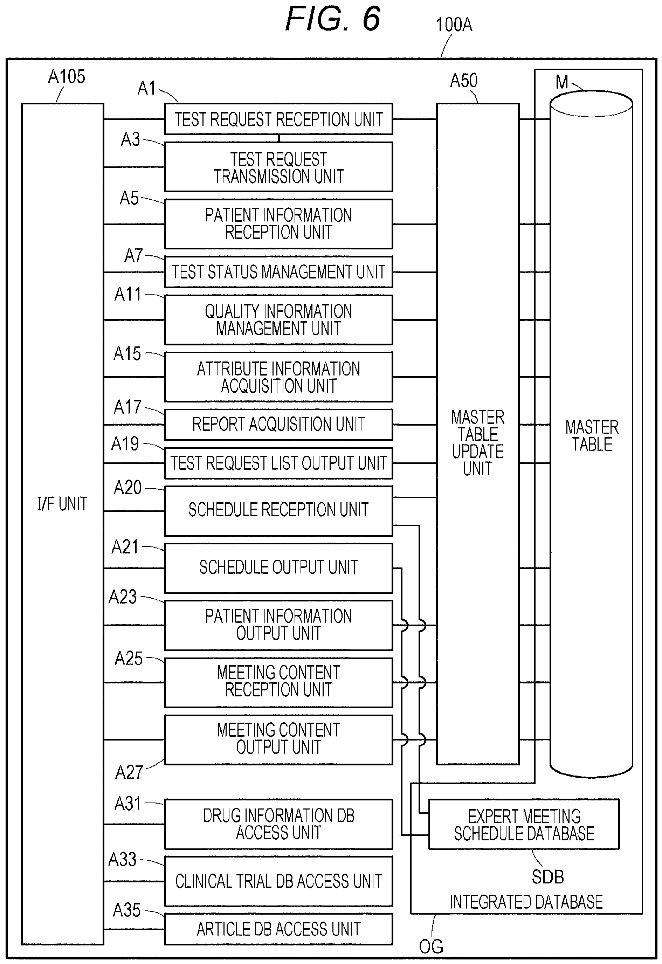

Fig. 6 shows functional blocks of a control unit of the integrated data management device A.

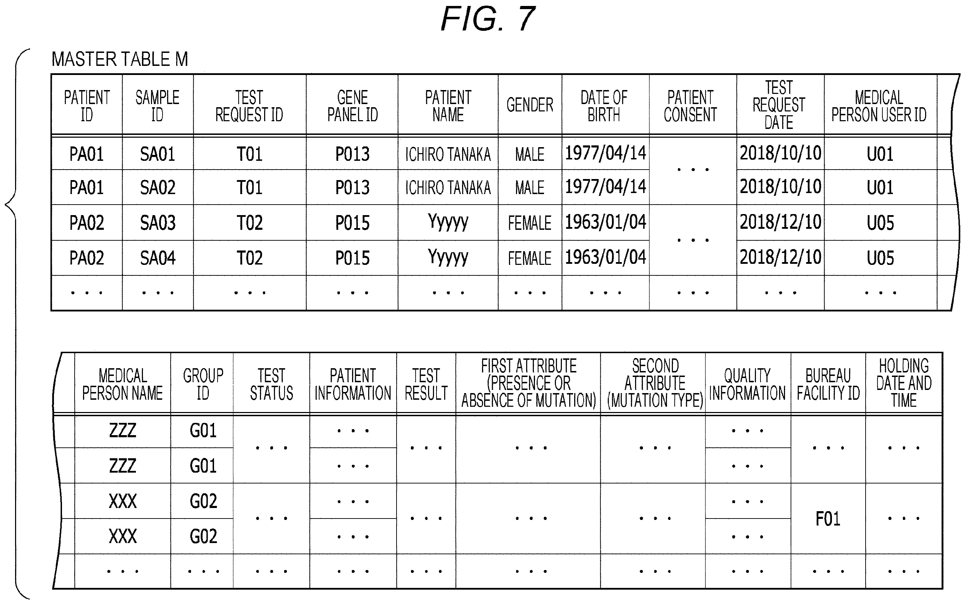

Fig. 7 shows an outline of a master table M.

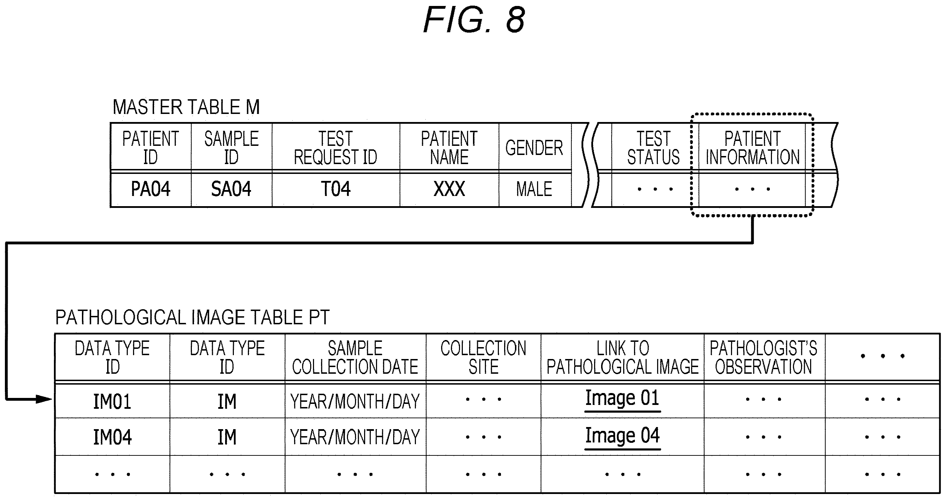

Fig. 8 shows an outline of a pathological image table PT linked to a "patient information" field of the master table M.

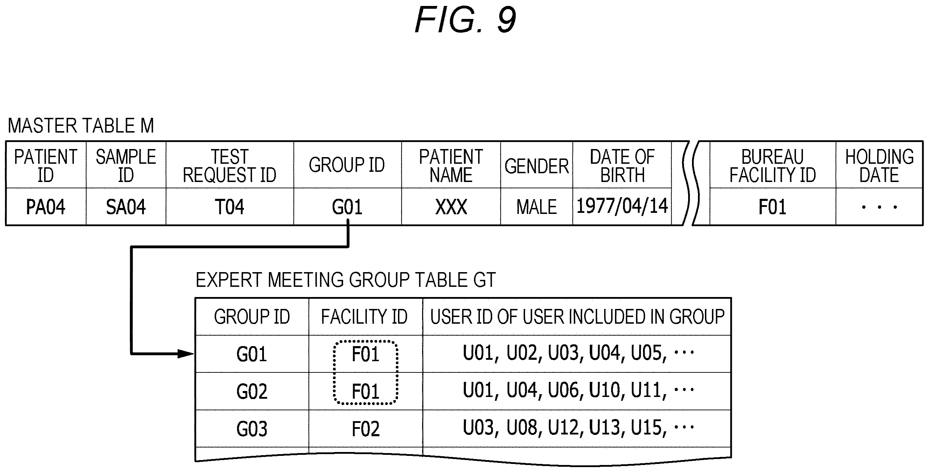

Fig. 9 shows an outline of an expert meeting group table GT linked to a "group ID" field of the master table M.

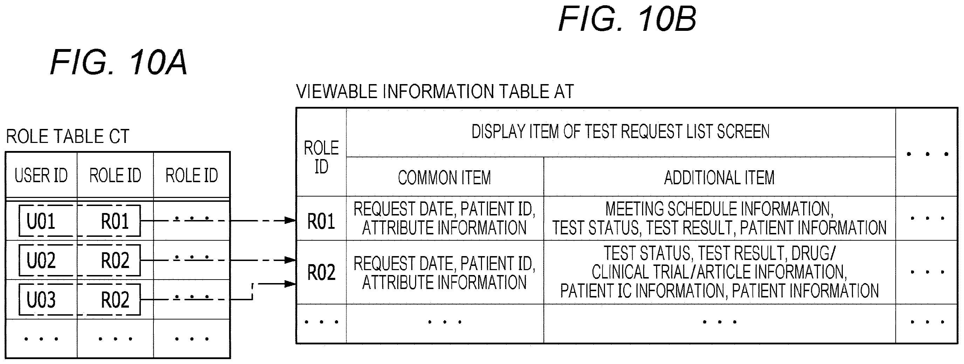

Fig. 10A shows an outline of a role table CT. Fig. 10B shows an outline of a viewable information table AT.
Fig. 11 shows a hardware configuration of clinical information management devices B10, B20, and B30.

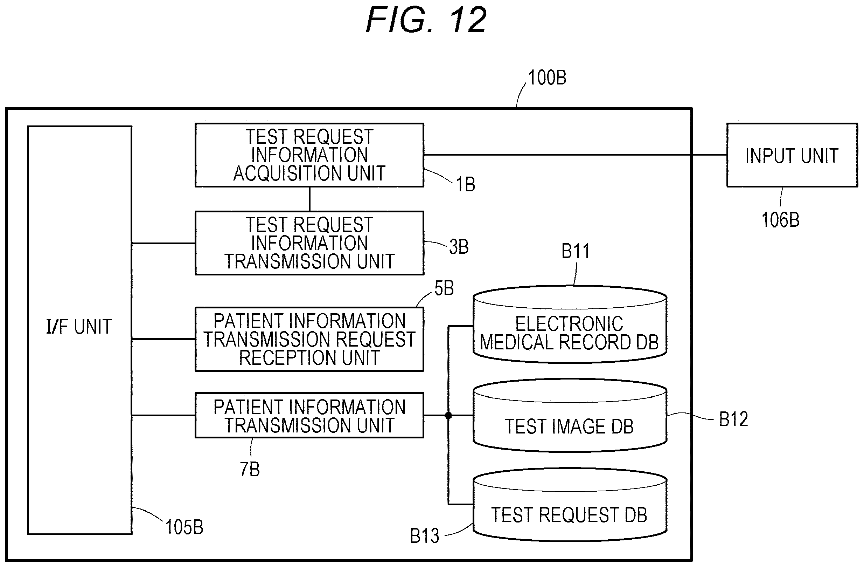

Fig. 12 shows functional blocks of a control unit of the clinical information management devices B10, B20, and B30.
Fig. 13 shows a hardware configuration of a test information management device C11.

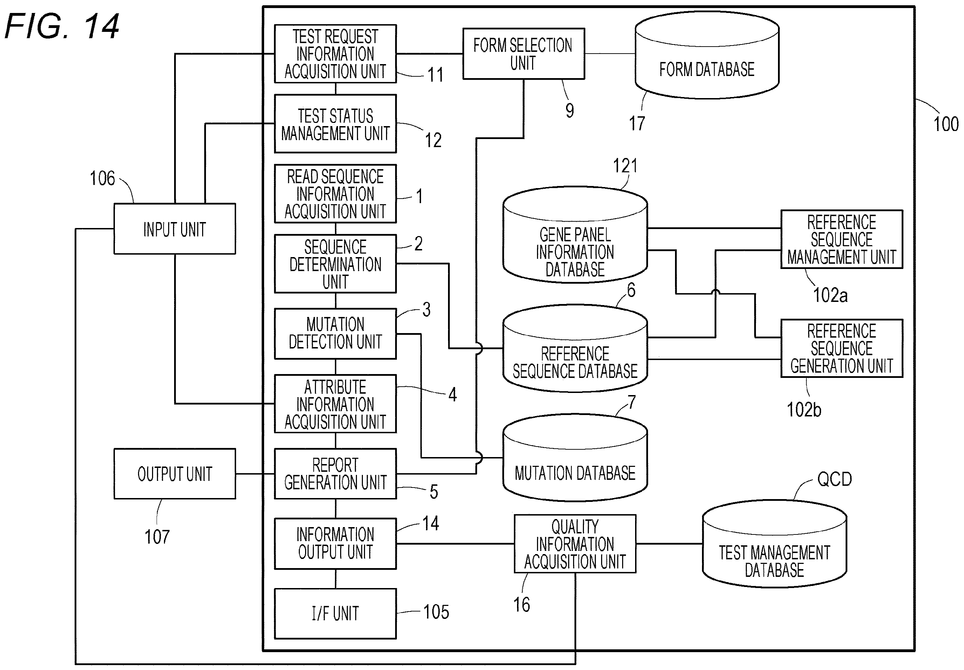

Fig. 14 shows functional blocks of a control unit of the test information management device C11.

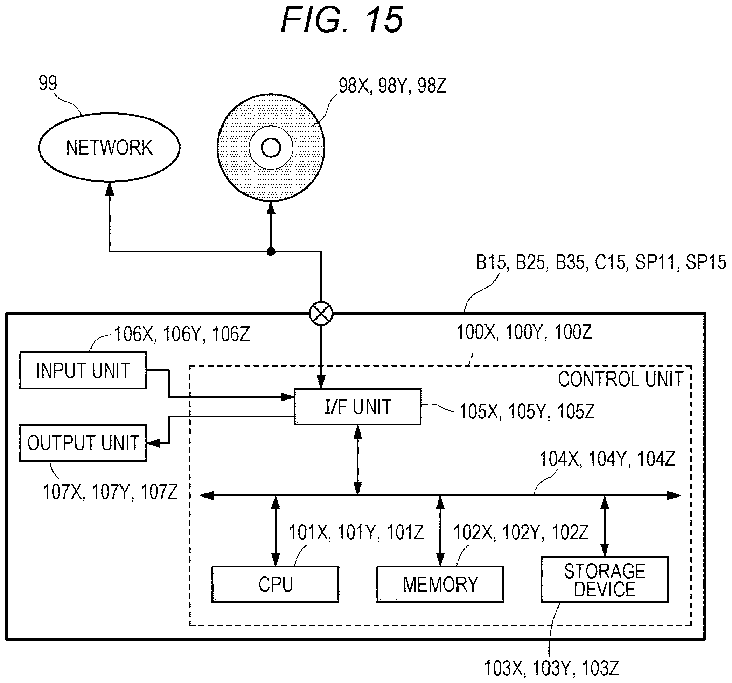

Fig. 15 shows a hardware configuration of each of expert meeting terminal B15, B25, B35, C15, SP11, and SP15.

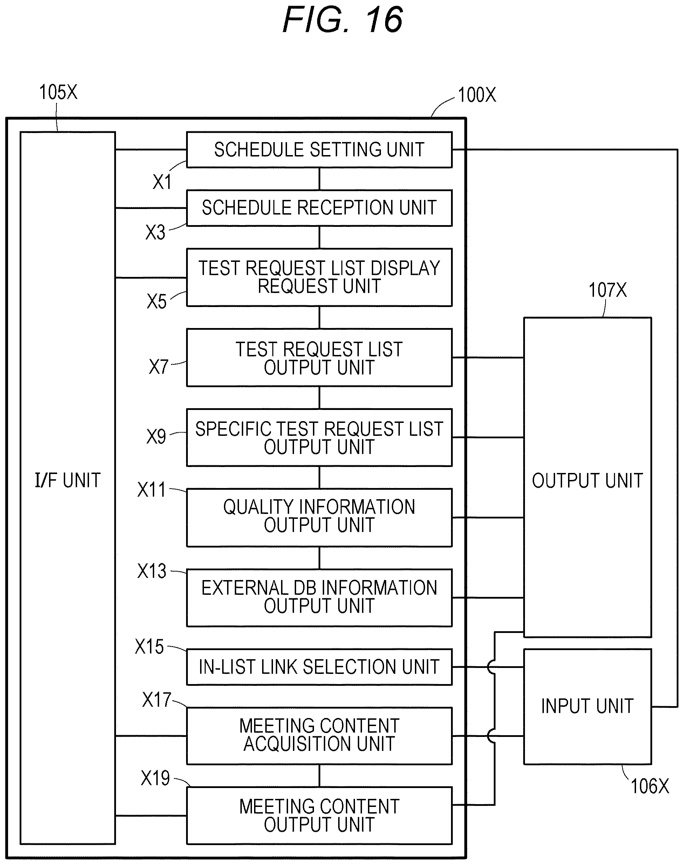

Fig. 16 shows functional blocks of a control unit of the expert meeting terminals B15, B25, and B35 of medical facilities.

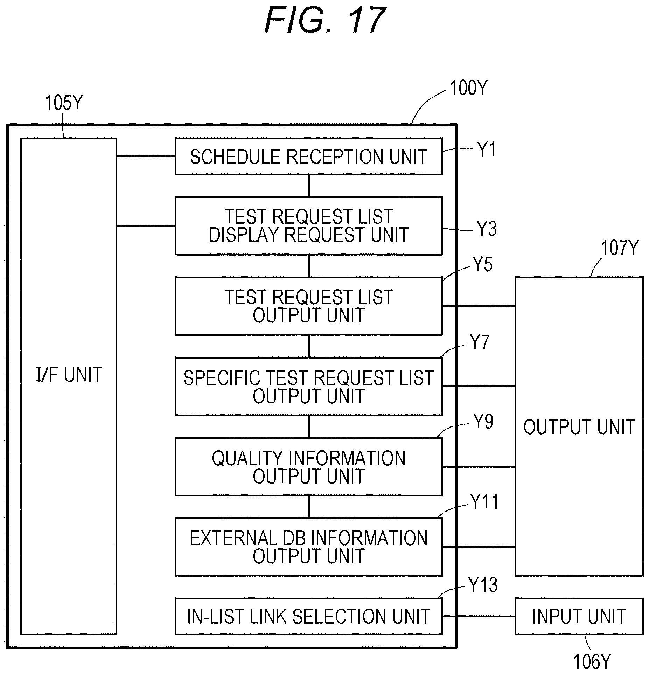

Fig. 17 shows functional blocks of a control unit of the expert meeting terminal C15 of a test facility and the expert meeting terminal SP11 of an external organization.

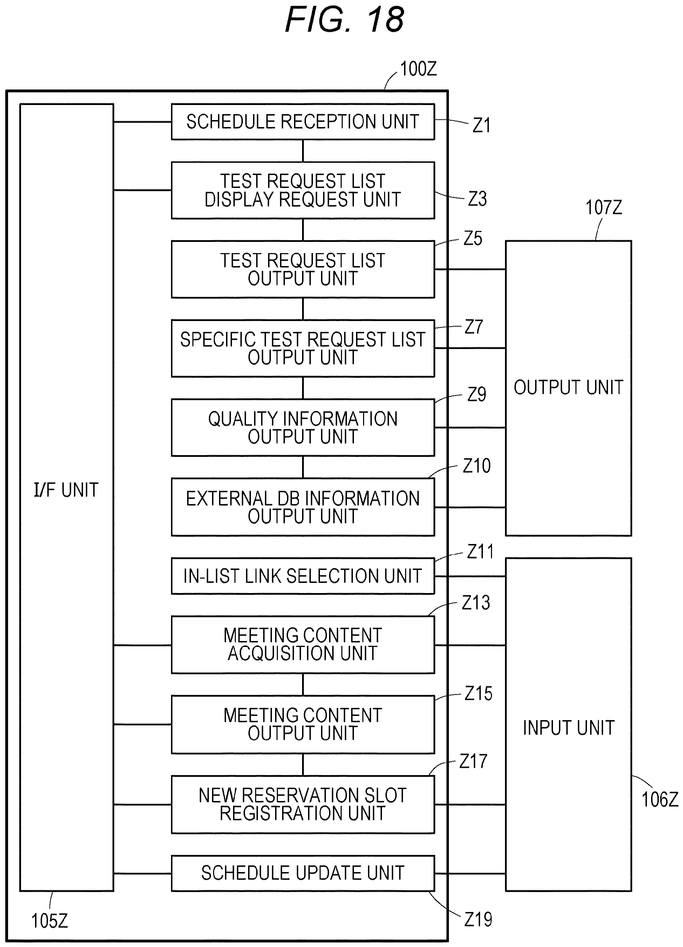

Fig. 18 shows functional blocks of a control unit of the bureau expert meeting terminal SP15.

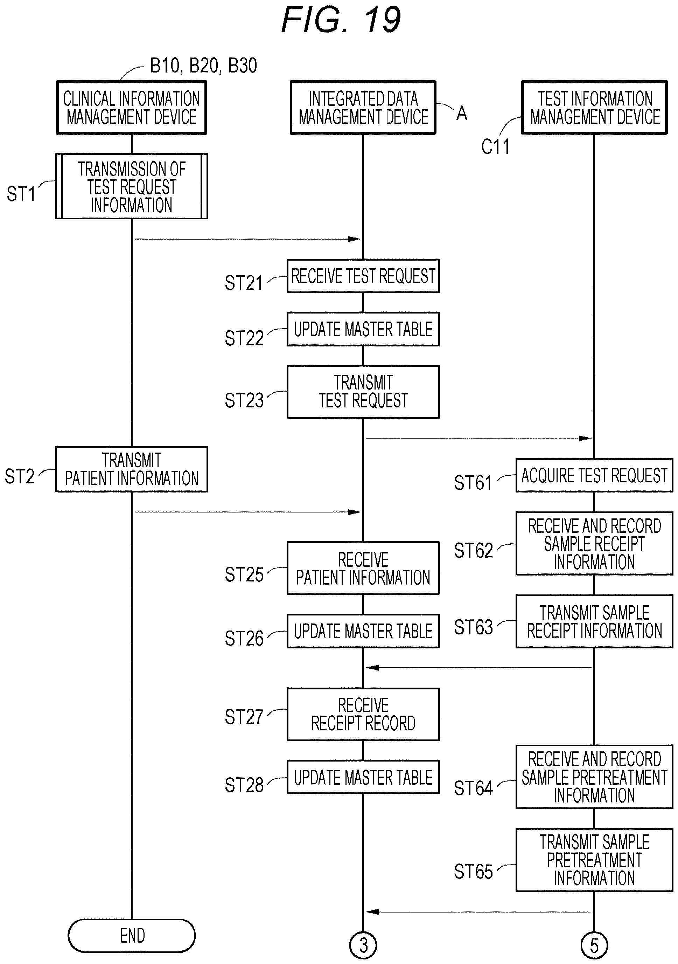

Fig. 19 is a flowchart showing a part of an operation of the system 1000.

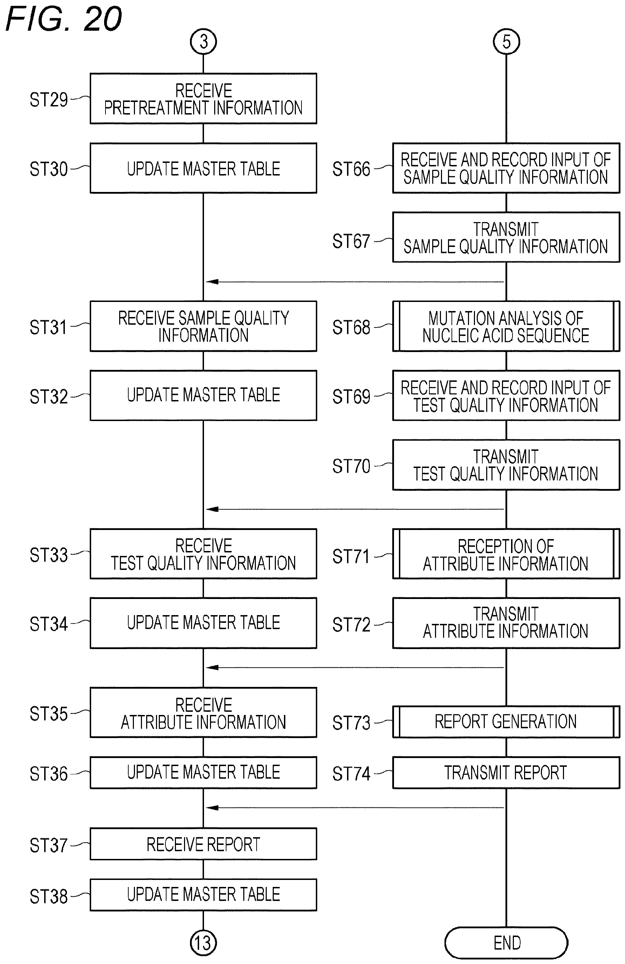

Fig. 20 is a flowchart showing a part of an operation of the system 1000.

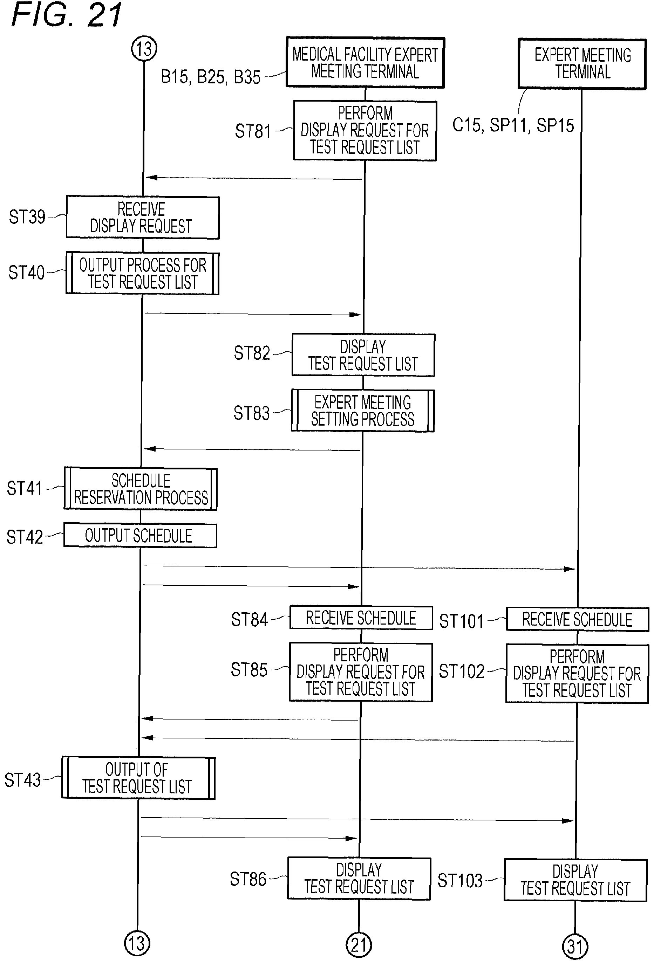

Fig. 21 is a flowchart showing a part of an operation of the system 1000.

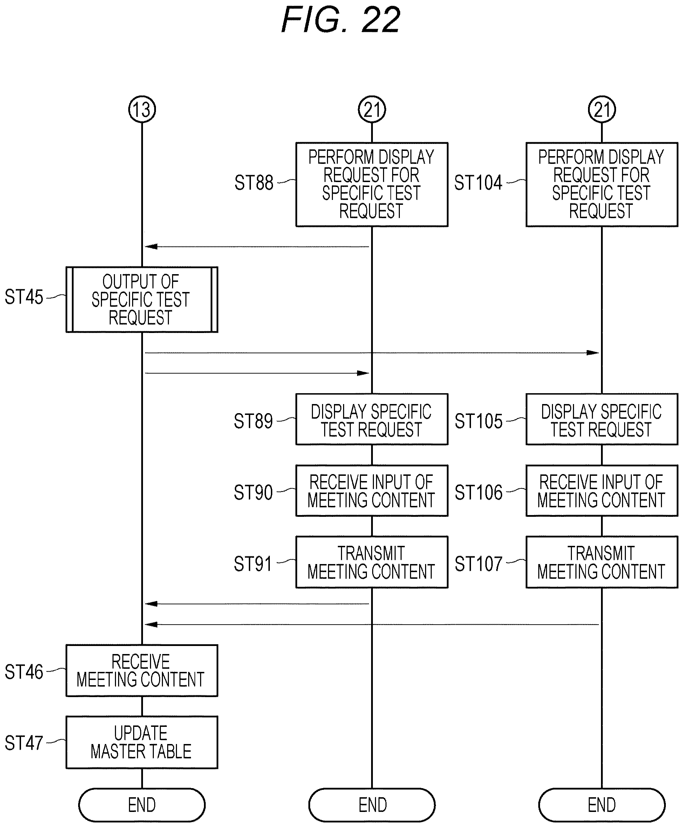

Fig. 22 is a flowchart showing a part of the operation of the system 1000.

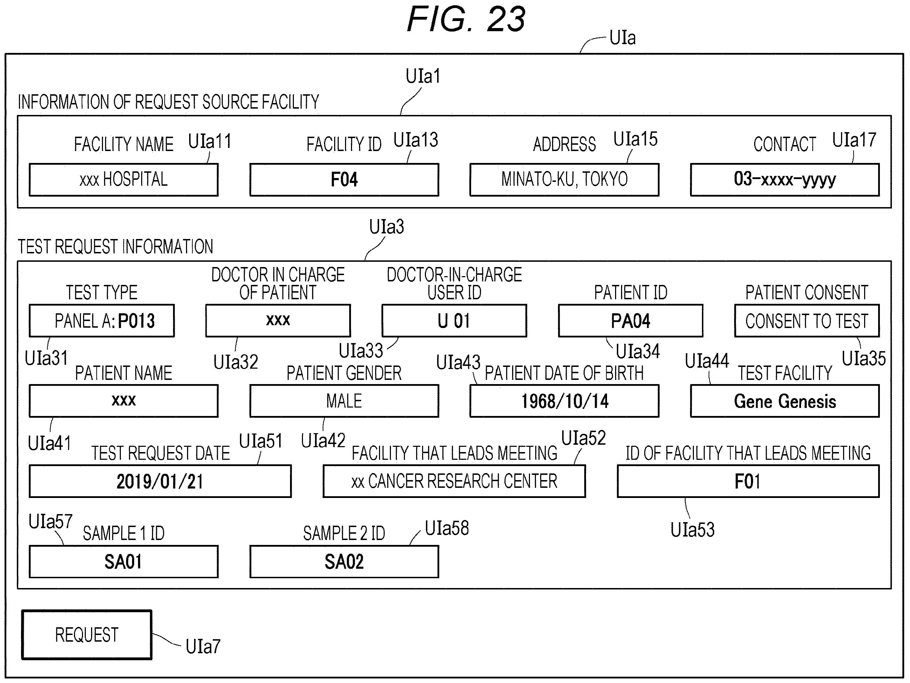

Fig. 23 shows an example of a graphical user interface UIa that is for making a test request.

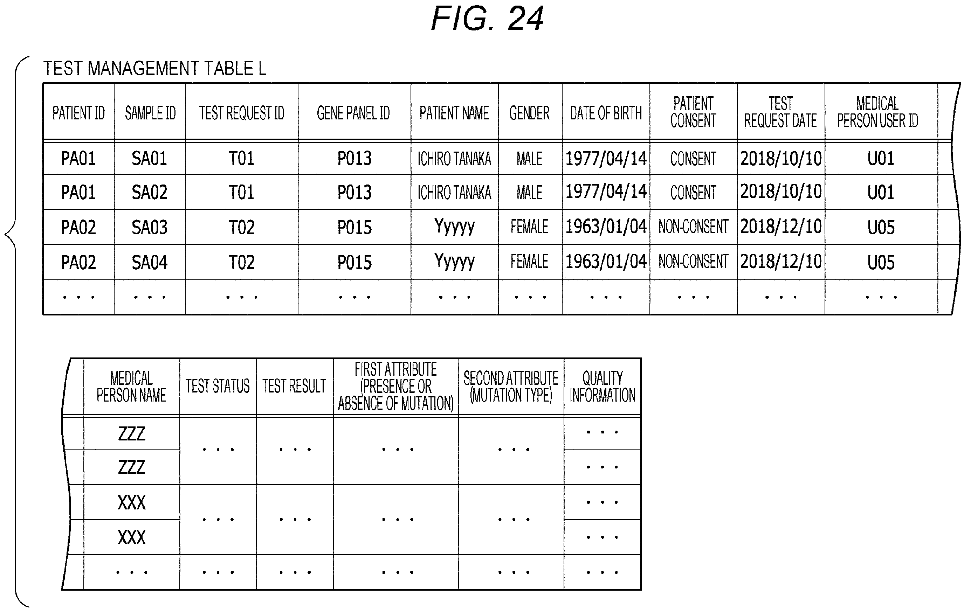

Fig. 24 shows an outline of a test management table L.

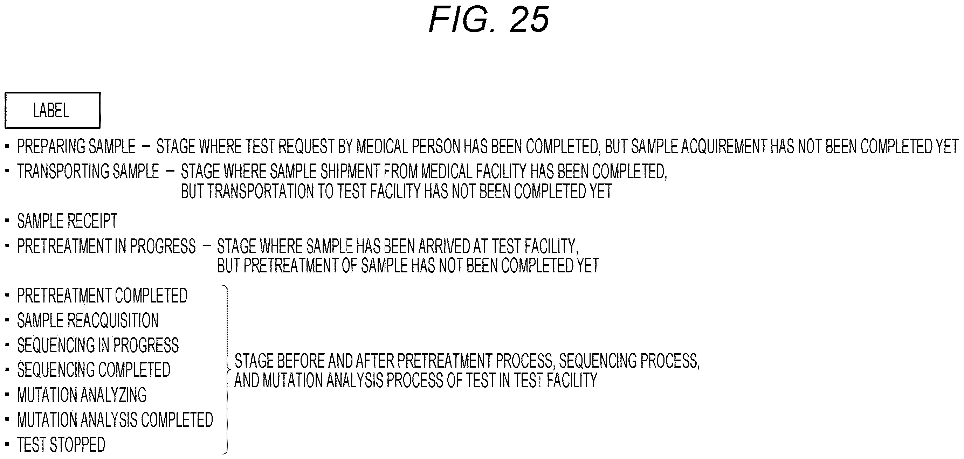

Fig. 25 is an example of a label showing progress information of a test.

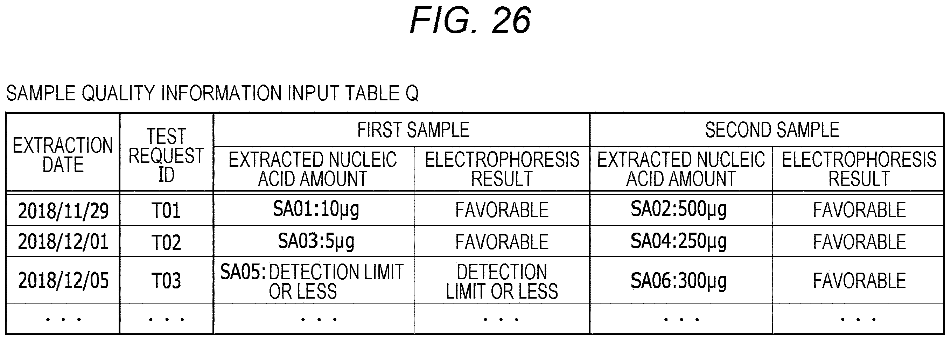

Fig. 26 shows an outline of a sample quality information input table Q in which sample quality information is recorded.

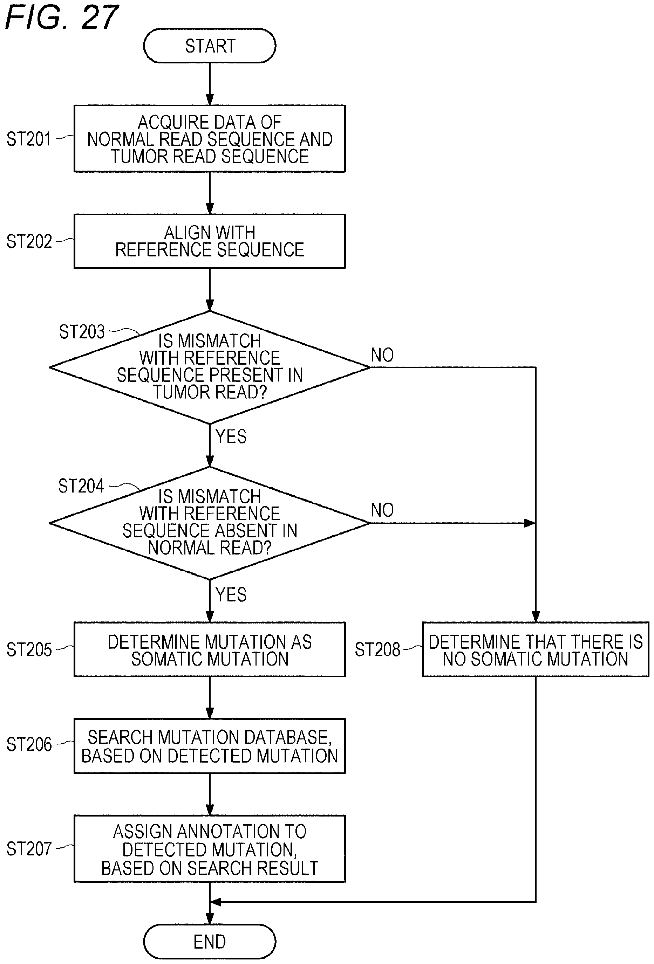

Fig. 27 shows a flowchart of mutation analysis.

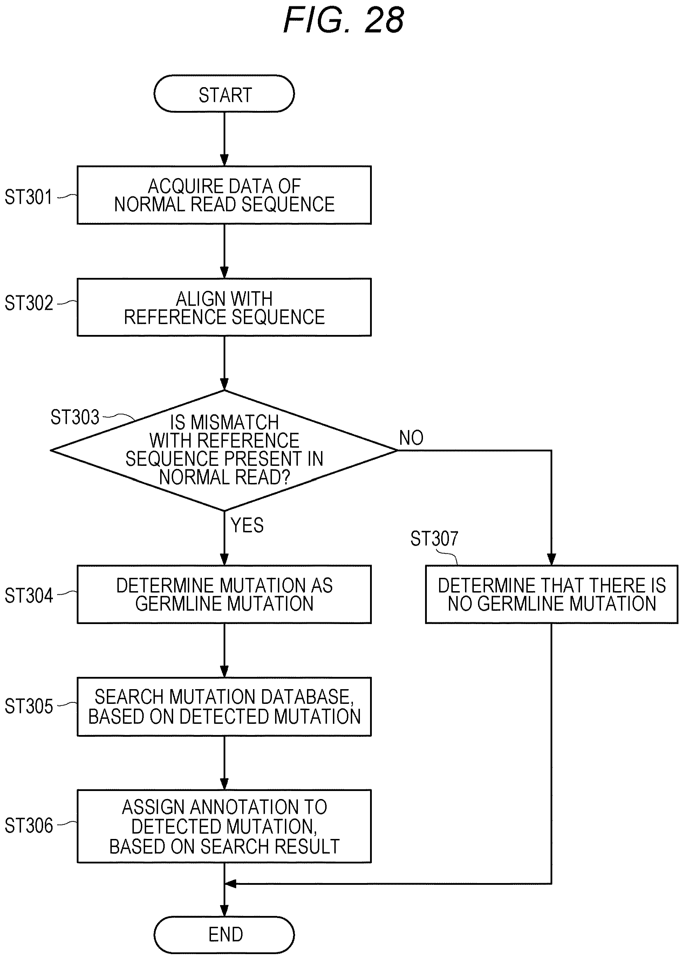

Fig. 28 shows a flowchart of mutation analysis.

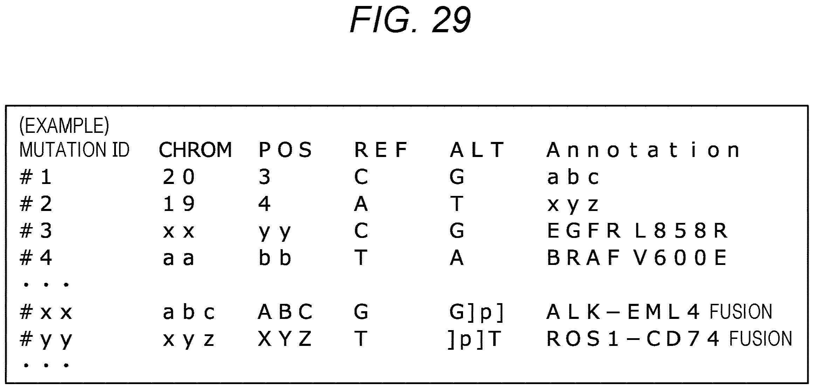

Fig. 29 shows an example of a test result outputted in mutation analysis.

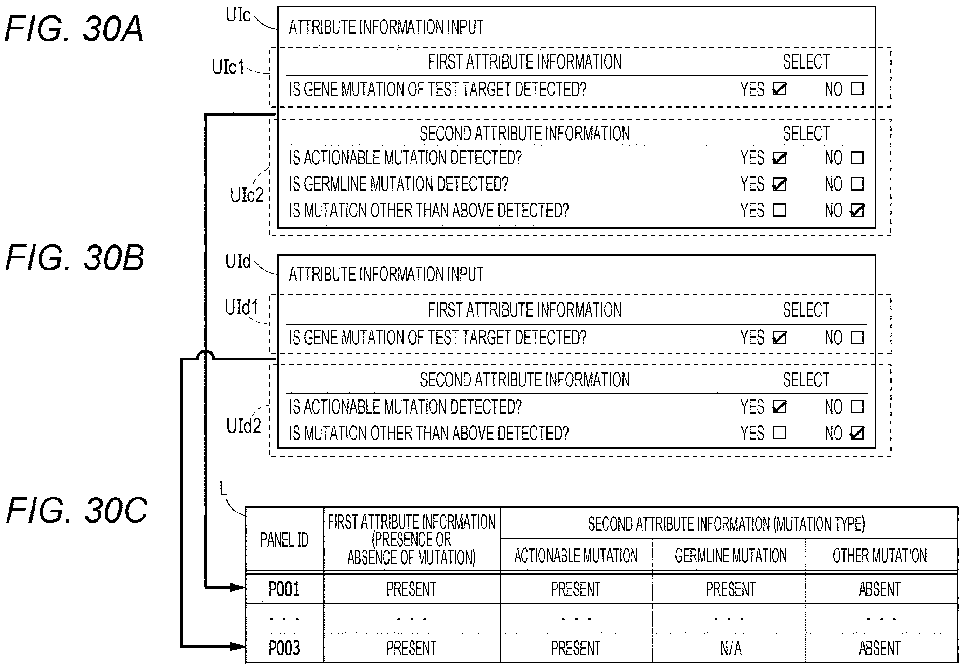

Fig. 30A shows an example of a graphical user interface UIc that is for acquiring attribute information. Fig. 30B shows an example of a graphical user interface UId that is for acquiring attribute information. Fig. 30C shows an example of the test management table L to which attribute information has been inputted.

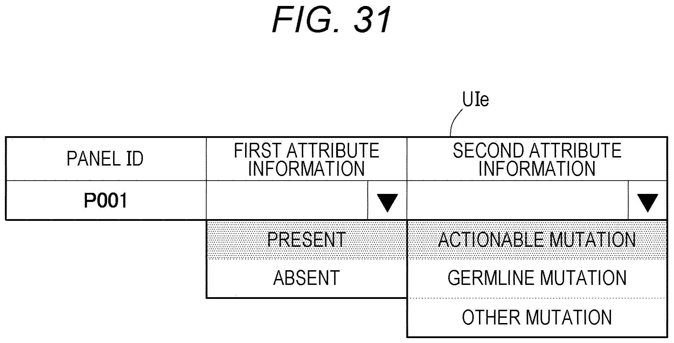

Fig. 31 shows an example of a list for acquiring attribute information.

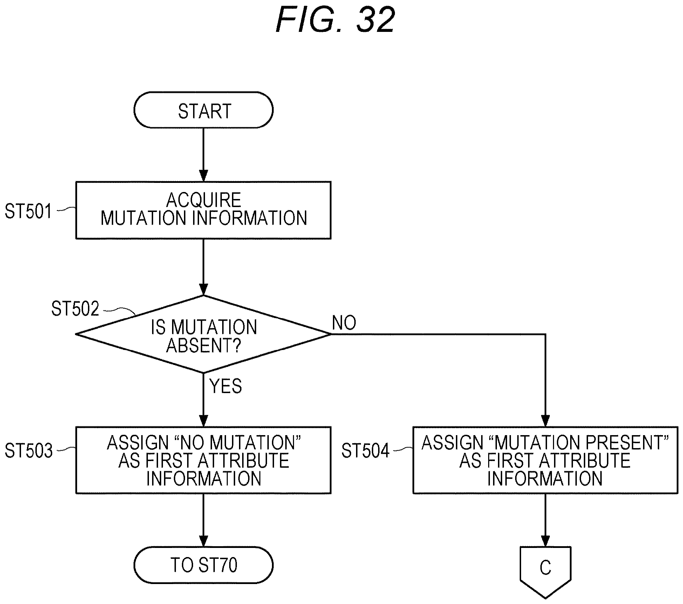

Fig. 32 shows a flowchart for the test information management device C11 to determine attribute information.

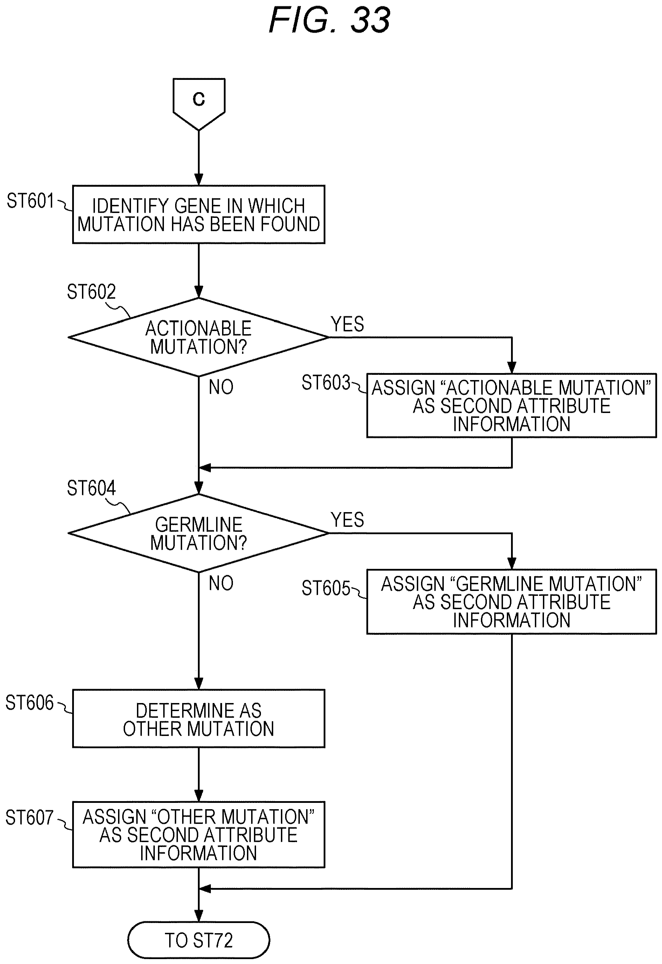

Fig. 33 shows a flowchart for the test information management device C11 to determine attribute information.

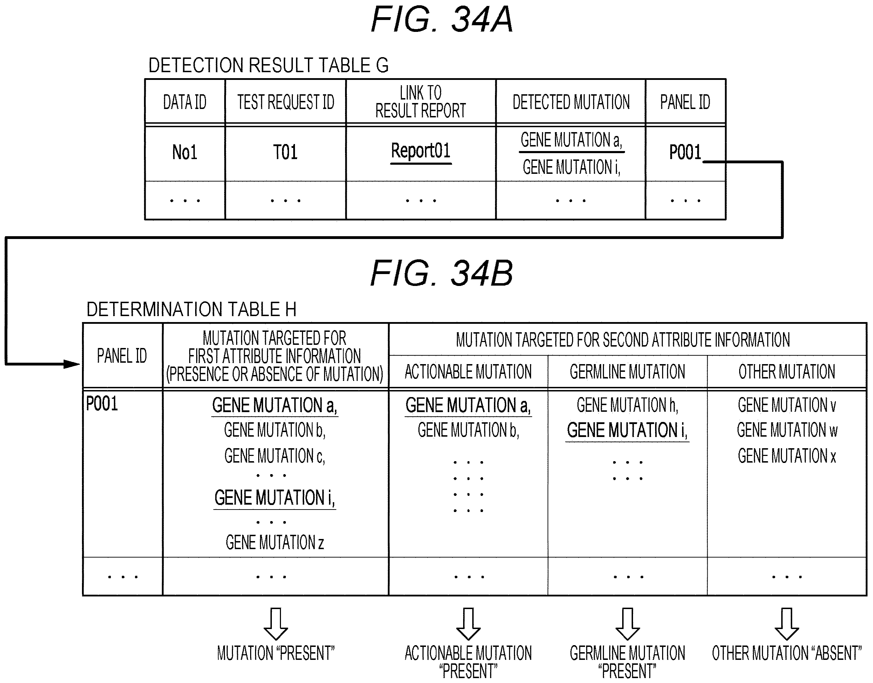

Fig. 34A shows an outline of a detection result table G. Fig. 34B shows an outline of a determination table H.

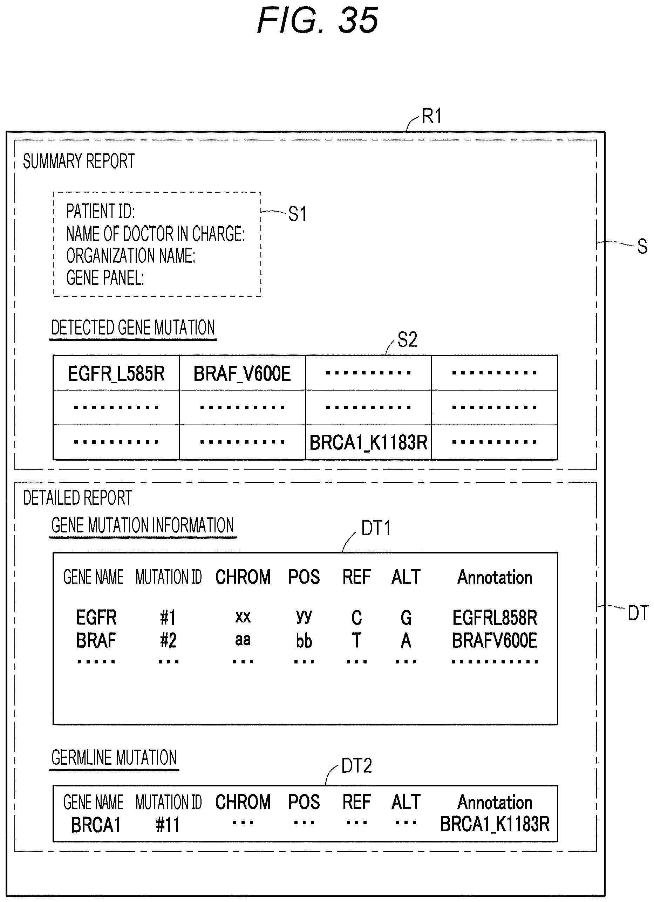

Fig. 35 shows a report format.

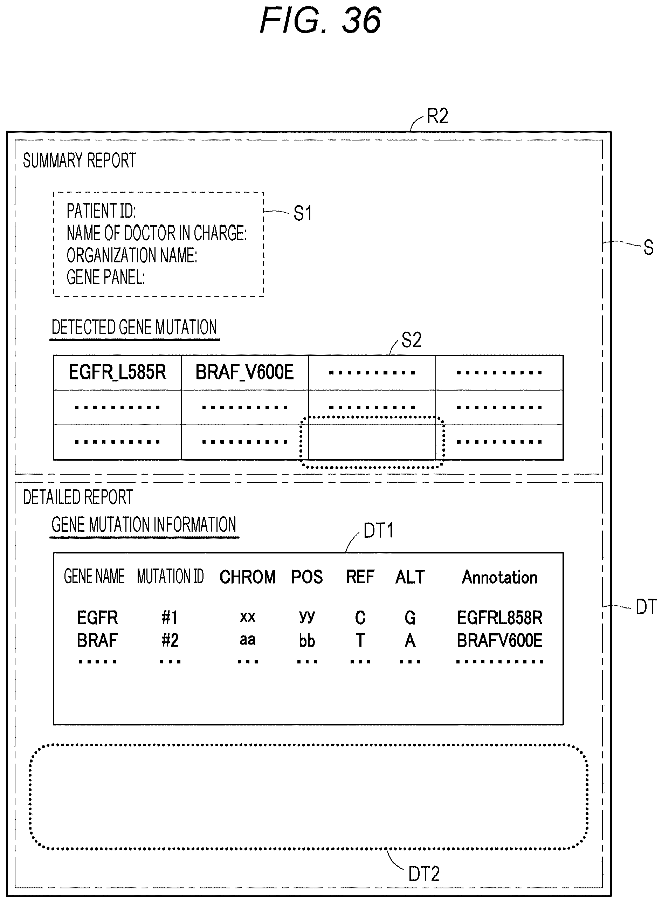

Fig. 36 shows a report format when it is necessary to hide an incidental finding.

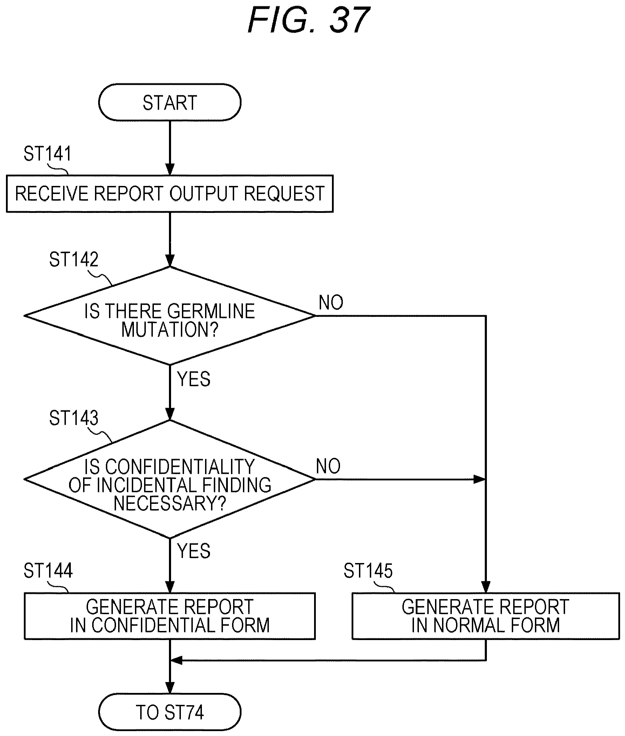

Fig. 37 shows a flowchart of a report generation process.

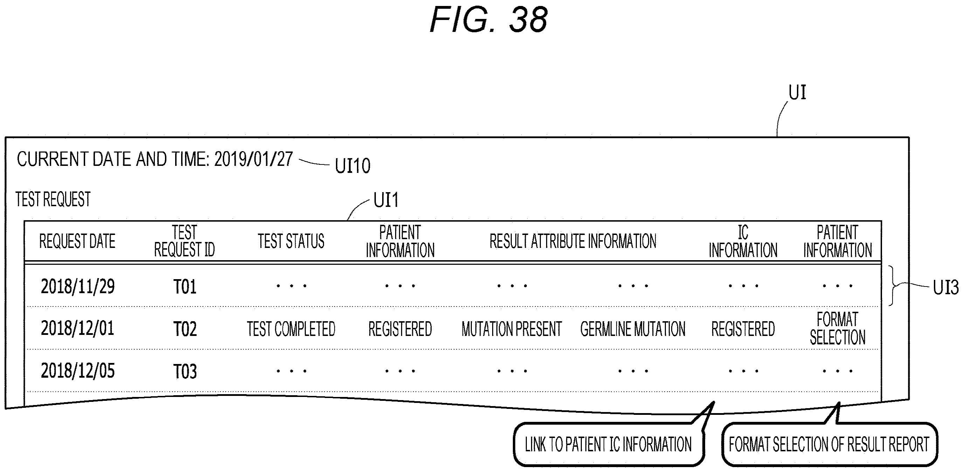

Fig. 38 shows an example of a test request list area UI1 for selection of a report format from a test request list outputted from the integrated data management device A.

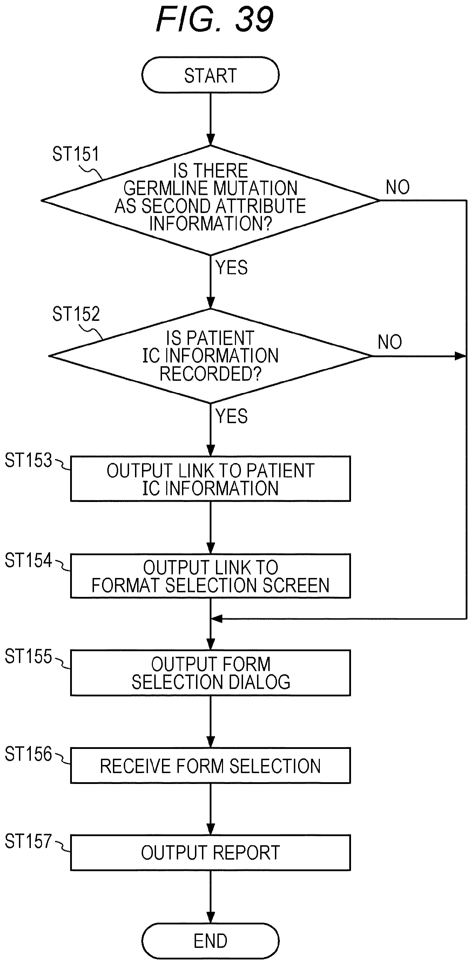

Fig. 39 shows a flowchart of the report generation process in the integrated data management device A.

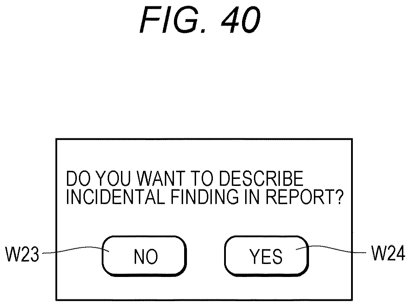

Fig. 40 shows an example of dialog displayed to allow selection of a report format.

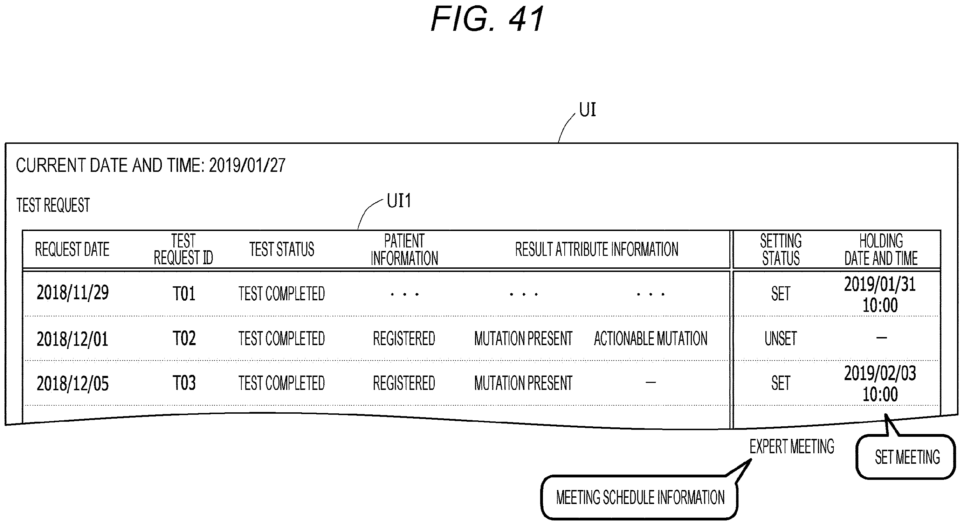

Fig. 41 shows an example of the test request list area UI1 when setting an expert meeting from a test request list outputted from the integrated data management device A.

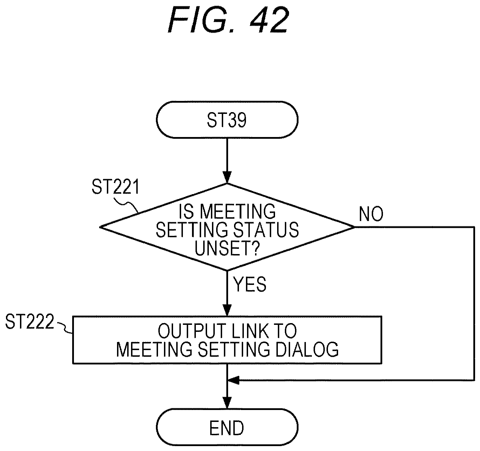

Fig. 42 is a flowchart of a process for displaying a link for setting an expert meeting on the graphical user interface UI, in the integrated data management device A.

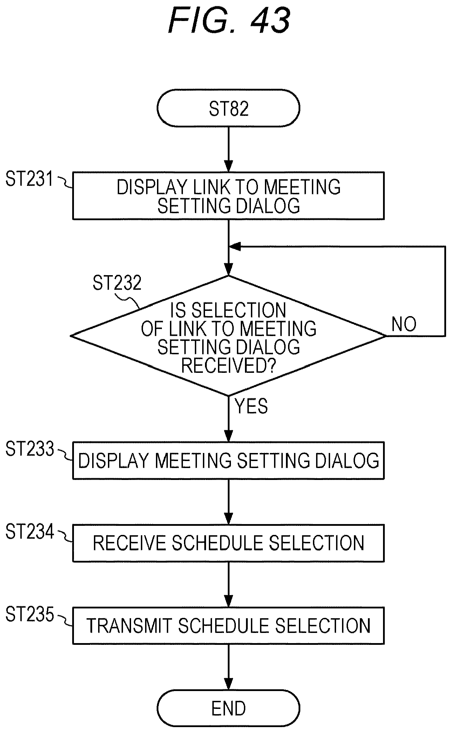

Fig. 43 shows a flowchart of a display process in the expert meeting terminals B15, B25, and B35 of medical facilities.

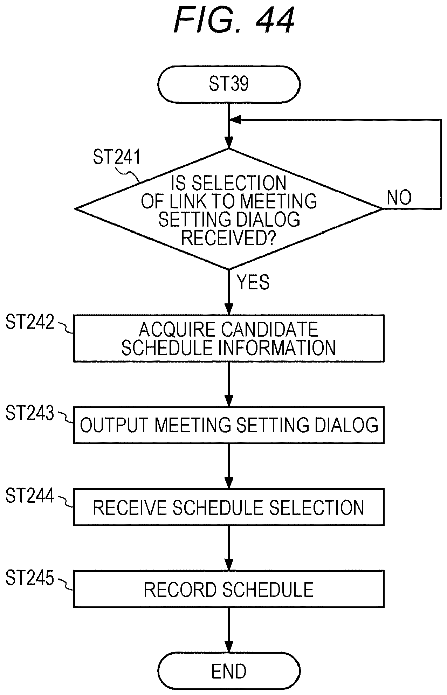

Fig. 44 shows a flowchart of a process for setting an expert meeting in the integrated data management device A.

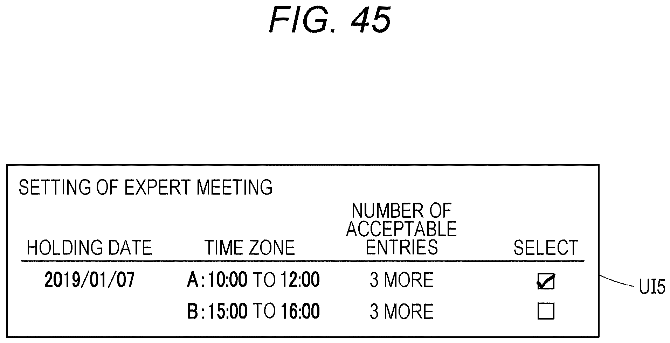

Fig. 45 shows an example of dialog UI5 that is for displaying a meeting schedule.

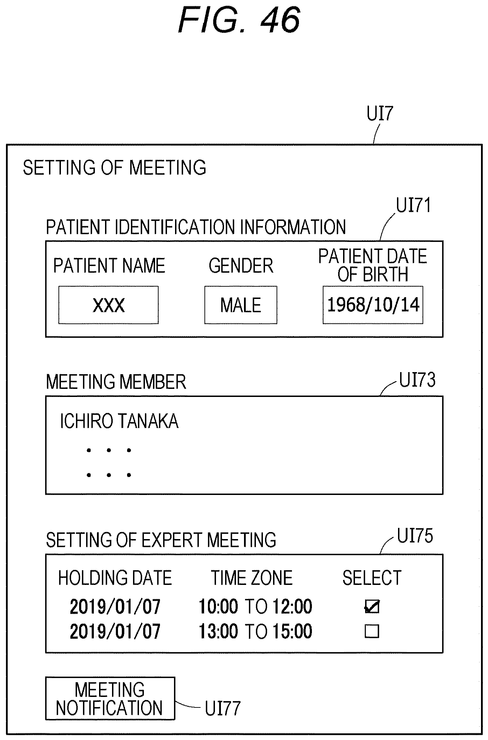

Fig. 46 shows an example of dialog UI7 that is for displaying a meeting schedule.

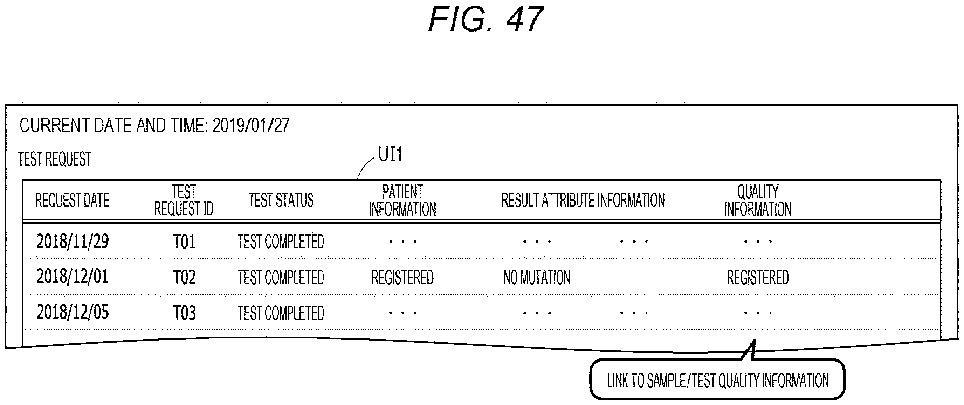

Fig. 47 shows an example of the test request list area UI1 for display of quality information from the test request list outputted from the integrated data management device A.

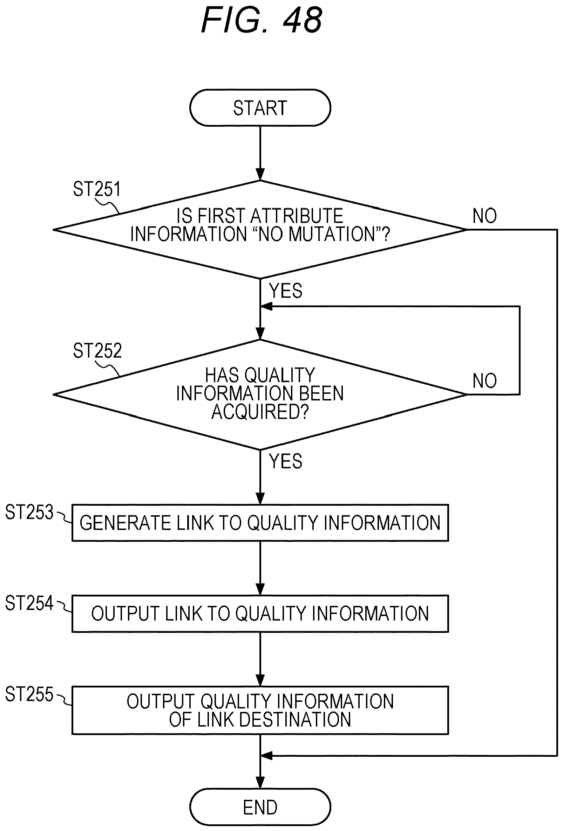

Fig. 48 is a flowchart of a process for displaying a link to quality information on the graphical user interface UI, in the integrated data management device A.

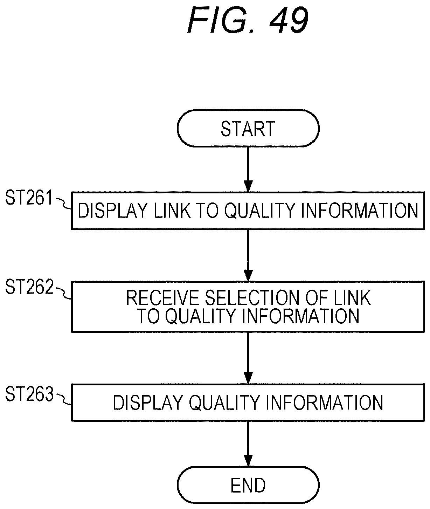

Fig. 49 shows a flowchart of a display process in the expert meeting terminal C15 of the test facility C1.

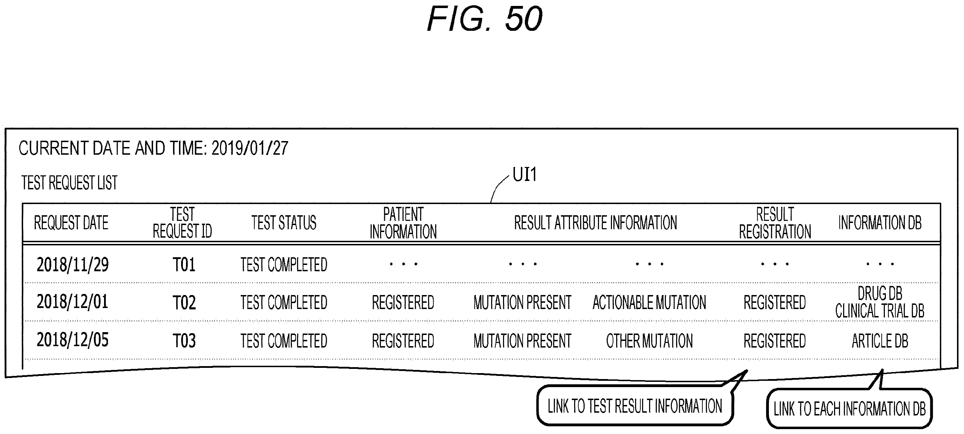

Fig. 50 shows an example of the test request list area UI1 when an external database is displayed from the test request list outputted from the integrated data management device A.

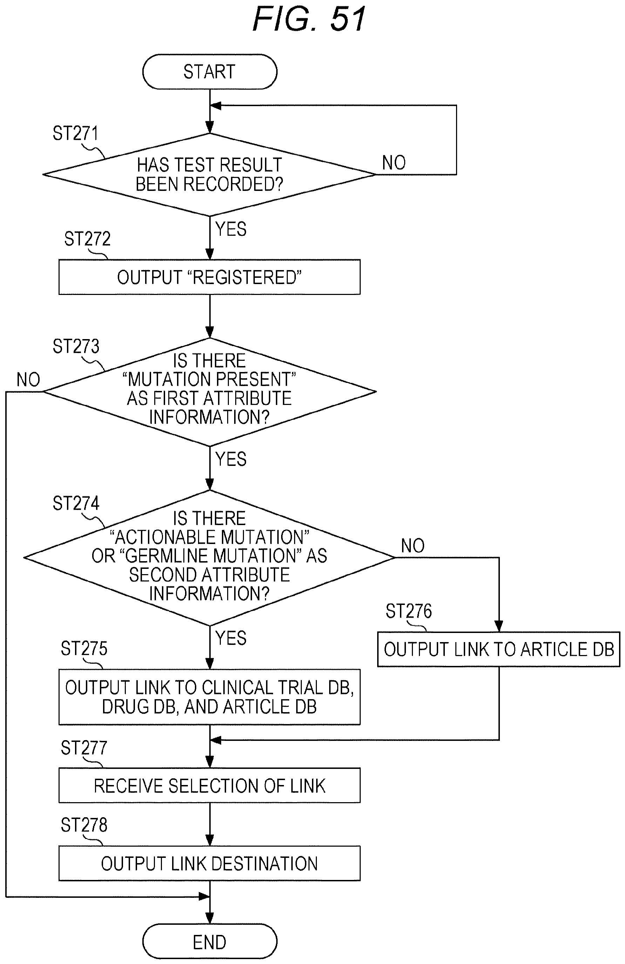

Fig. 51 shows a flowchart of a process for displaying a link to an external database on the graphical user interface UI, in the integrated data management device A.

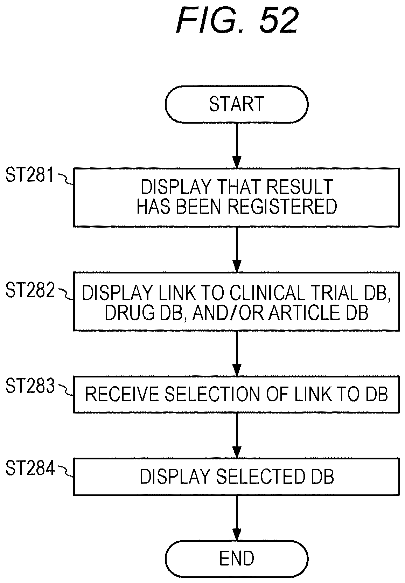

Fig. 52 shows a flowchart of a display process in the expert meeting terminal SP11 of an external facility SP1 and the bureau expert meeting terminal SP15.

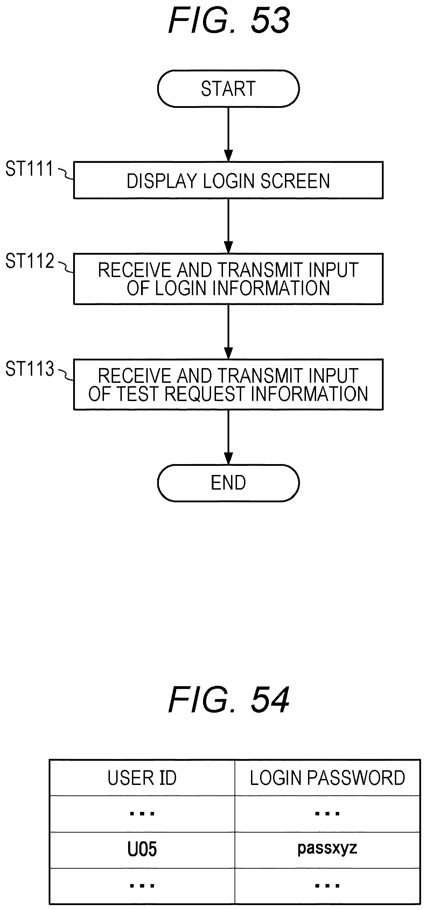

Fig. 53 shows an example of an authentication process when a test request is made.
Fig. 54 shows an example of a login information table P.

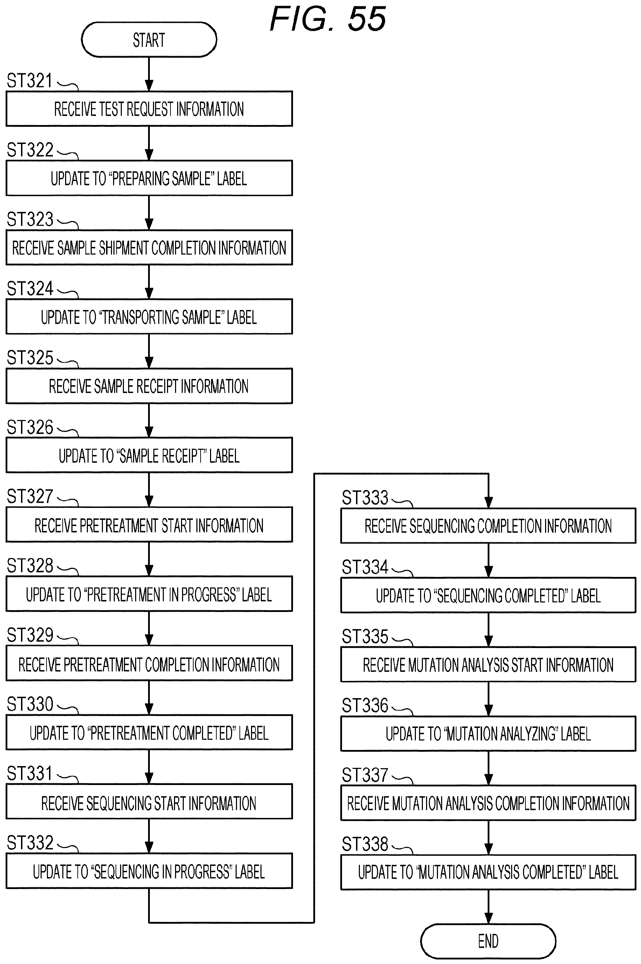

Fig. 55 shows an update process of test progress information in the integrated data management device A.

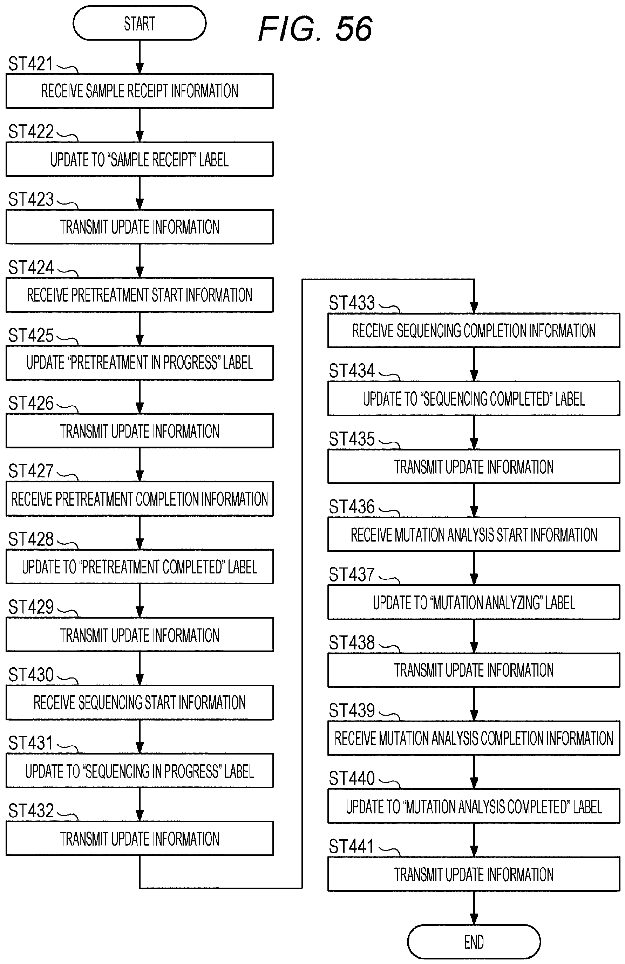

Fig. 56 shows an update process of test progress information in the test information management device C11.

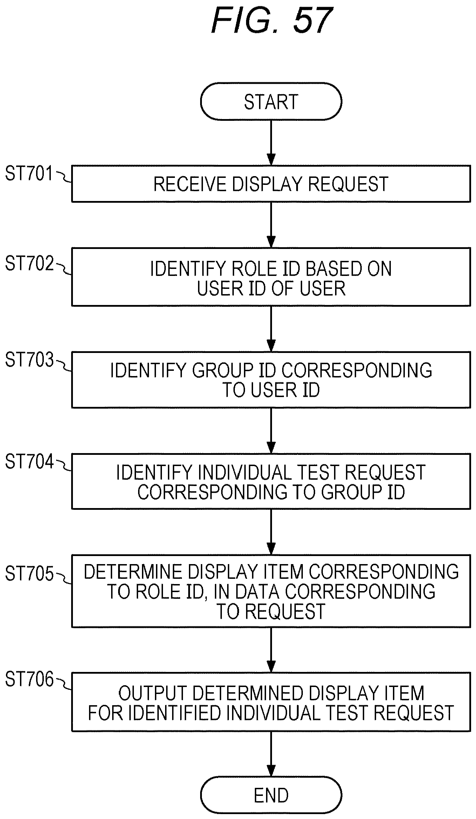

Fig. 57 is a flowchart showing a display process for a test request list according to a role ID.

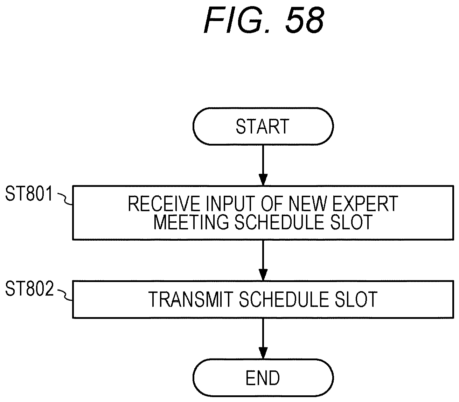

Fig. 58 shows a registration process of a new expert meeting schedule slot in the bureau expert meeting terminal SP15.

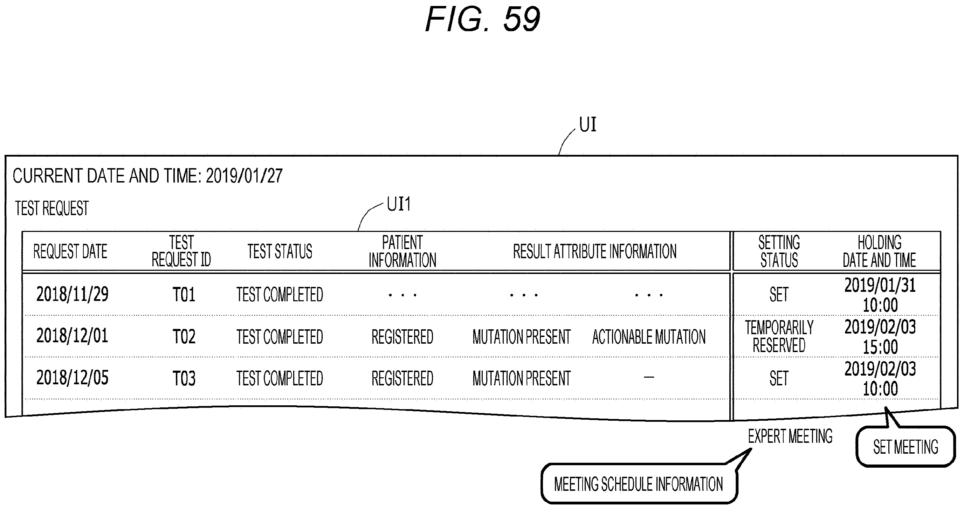

Fig. 59 shows a modified example of the test request list area UI1 when setting an expert meeting from the test request list outputted from the integrated data management device A.

### DETAILED DESCRIPTION OF THE PREFERRED EMBODIMENTS

Hereinafter, an embodiment for carrying out the invention will be described in detail with reference to the accompanying drawings. In the following description and drawings, the same reference numerals denote the same or similar components, and thus the description of the same or similar components will be omitted.

### I. Outline of embodiment

One embodiment relates to a method for managing a test request for gene panel testing by a computer.

First, an outline of the present embodiment will be described with reference to Figs. 1 to 3A to 3C.

Analysis of a nucleic acid sequence of a patient sample is performed, for example, for the purpose of detecting a mutation in a nucleic acid sequence present in a tumor cell, in order to predict an effect of an anticancer drug on the tumor cell and a prognosis.

In the present specification, "nucleic acid sequence mutation" is a concept including nucleotide substitution, insertion, deletion, gene fusion, and the like. The nucleic acid sequence mutation may include a synonymous mutation that does not affect an amino acid sequence and a non-synonymous mutation that affects an amino acid sequence. The mutation to be detected is desirably a non-synonymous mutation. The non-synonymous mutation is a mutation that causes a structural abnormality of protein. The non-synonymous mutation is considered to be associated with tumorigenesis of a cell.

Mutations may be classified into two types depending on whether the mutation has occurred in a germ cell before fertilization or after fertilization. A mutation that has occurred in a somatic cell is called a somatic mutation, and a mutation that has occurred in a germ cell is called a germline mutation. Unlike the somatic mutation, the germline mutation may be inherited to the next generation of an individual. Therefore, when a patient to be applied with the method of the present embodiment inherits the germline mutation from a parental generation, even a sample prepared from a somatic cell also contains the germline mutation.

In addition to the classification nucleic acid sequence mutations as described above, mutations can be classified in accordance with reactivity to anticancer drug treatment. For example, even if there is a somatic mutation or a germline mutation that causes a disease, a mutation may be generally called an actionable mutation when the mutation can be expected to have therapeutic efficacy of 3A or more shown in "Clinical practice guidance for next-generation sequencing in cancer diagnosis and treatment" published jointly by the Japanese Society of Medical Oncology, the Japan Society of Clinical Oncology, and the Japanese Cancer Association.

Analysis of a nucleic acid sequence of a cancer-related gene by using a patient's cancer tissue sample is important for identification of an effective anticancer drug or the like against a cancer held by the patient. In gene panel testing, it is possible to simultaneously analyze several tens to several hundreds of genes in one test. A result of gene panel testing is not interpreted by a doctor in charge of the patient alone, but is interpreted by an expert meeting (also called an expert panel) including a pathologist who performs tissue diagnosis, a clinical technologist and/or a bioinformatician who conducts gene panel testing, a genetic counselor and/or a molecular genetics researcher who is an expert in gene mutation interpretation, and the like, in addition to the doctor in charge. The expert meeting determines an appropriate treatment strategy for the patient subjected to the gene panel testing.

A test request for gene panel testing is made from each medical facility, but the expert meeting is often held by one base institution in each region. Therefore, results of gene panel testing for which test requests have been made individually and for each patient by multiple medical facilities in the region will be aggregated in one expert meeting.

Therefore, in order to efficiently manage a large number of requests for gene panel testing, one embodiment provides a method for managing a test request for gene panel testing. The method includes: using a computer to acquire, for each of a plurality of test requests for gene panel testing, information regarding the test request, and an attribute indicating an outline of a test result in the gene panel testing; and outputting display information for displaying a plurality of the test requests and the attribute in association with each other.

Fig. 1 shows a flow of gene panel testing in the present embodiment.

In Fig. 1, the flow of the gene panel testing will be described with use of three organizations as an example. A first organization involved in the gene panel testing is a medical facility B1 such as a hospital where a cancer patient actually visits. A second organization is a test facility C1 in which the gene panel testing is actually conducted. A third organization is an expert meeting EP. The three organizations may share information through an integrated data management device A that is for sharing information in each organization.

In gene panel testing (hereinafter, also simply referred to as a test), a patient P1 having a tumor visits the medical facility B1, and a doctor in charge H1a explains details and a flow of gene panel testing. In the explanation, informed consent is also acquired regarding whether the patient P1 or a his/her family wishes to be informed of a result in case of incidental finding such as finding of a germline mutation in the test (I in Fig. 1).

When the patient P1 consents to carry out the gene panel testing, the doctor in charge H1a requests the gene panel testing (II in Fig. 1). Information regarding the test request is transmitted to the integrated data management device A and recorded in a master table M together with patient information. The information regarding the test request may include information such as a test request date, test identification information (ID), a test panel number, a sample type, and information regarding a patient (patient information). The patient information may include patient identification information (ID), a patient name, gender, age, a pathological diagnosis result, a patient medical history, a patient family history, and the like.

When the test is requested, a sample required for the test is collected at the medical facility B1 (III in Fig. 1). As the sample, a sample containing a tumor cell and a sample containing a non-tumor cell are usually collected for one patient. The sample may be collected by a pathologist H1b or a clinical technologist H3. The collected sample is transported to the test facility C1.

At the test facility C1, a clinical technologist T1 performs pretreatment of the sample, performs sequencing of a nucleic acid sequence contained in the sample by using a next-generation sequencer, to carry out the gene panel testing (IV in Fig. 1). After the test is carried out, the clinical technologist T1 and a bioinformatician T2 cooperate with each other to create a test report (also referred to as a report). In addition to this, information regarding a test status such as pretreatment information indicating progress of the test, quality information regarding quality of the test and the sample, and attribute information indicating an outline of a test result (hereinafter, may be simply referred to as "attribute information" or "attribute") is generated. The attribute information may include first attribute information regarding the presence or absence of a mutation relating to a predetermined gene, and second attribute information relating to a mutation type. The test result of the gene panel testing, the pretreatment information, the test and sample quality information, and the attribute information are transmitted to the integrated data management device A, and recorded in the master table M in association with the information regarding the test request and the patient information. The test report may be returned to the medical facility B1 that has requested the test in a paper medium.

Fig. 2B shows an example of a graphical user interface UI including display information outputted from the integrated data management device A. In Fig. 2A, when a test request is made, gene panel testing included in information regarding the test request is conducted. Genes to be tested in the gene panel testing include predetermined genes such as a gene, b gene, c gene, d gene..., for example. Attribute information is attached to a result of the gene panel testing. In Fig. 2B, the graphical user interface UI includes a test request list area UI1 that displays a test request list and an area UI10 that indicates a display date and time. The test request list area UI1 includes an individual test request display area UI3 that displays each test request. The test request list area UI1 may include a plurality of individual test request display areas UI3. The test request list area UI1 may include a column area showing a request date of each test, a test request ID, a test status, patient information, result attribute information, result registration status, and the like. For example, in the test request ID area, a label indicating a test request ID for identification of each test is given as T01, T02,.... For example, each ID has a link to information regarding each test request registered in the master table M. The test status area displays labels indicating a test status such as "test completed" and "pretreatment completed" transmitted from the test facility C1. The patient information area displays a label of "registered" or "unregistered". The "registered" label has a link to patient information associated with each test request registered in the master table M. The result attribute information may display a label indicating whether or not there is a mutation as the first attribute information transmitted from test facility C1, a label indicating a mutation type as the second attribute information, and a label indicating "actionable mutation", "germline mutation", or "other". The result registration area may display labels of "registered" and "unregistered" indicating whether or not a result of gene panel testing transmitted from the test facility C1 is registered in the master table M.

The information displayed in the test request list area UI1 serves as display information for displaying a plurality of the test requests and an attribute in association with each other.

Returning to Fig. 1, in the medical facility B1, the doctor in charge H1a accesses the individual test request display area UI3 of the patient P1 who has been subjected to the gene panel testing, from the test request list outputted from the integrated data management device A. Then, the doctor in charge H1a requests to hold an expert meeting (V in Fig. 1). For example, when the doctor in charge H1a accesses the integrated data management device A from the expert meeting terminal provided in the medical facility B1, the graphical user interface UI shown in Fig. 3 may be displayed. The test request list area UI1 of the graphical user interface UI displays a setting status area indicating a setting status of an expert meeting and a holding date and time area. The setting status area is an area indicating whether or not a meeting is set. When an expert meeting is set, the holding date and time field displays a schedule. When no expert meeting is set, an "unset" label is displayed. The doctor in charge H1a can set a schedule of an expert meeting by selecting an unset label from the individual test request display area UI3 of the patient P1. This causes a request for an expert meeting regarding a result of gene panel testing of the patient P1.

In general, when gene panel testing is conducted, an expert meeting is held for almost all patients regarding the result, to determine a treatment strategy. Almost all is intended to exclude, for example, a case where quality of the test or the sample does not bear the test, or a case where the patient dies.

Return to Fig. 1. An expert meeting will be held at a date and time set by the doctor in charge H1a for the gene panel testing of the patient P1 (VI in Fig. 1). The expert meeting may be generally held in a web meeting or the like, by using an expert meeting terminal or the like. At the expert meeting, a tumor treatment strategy for the patient P1 is determined, and the result is recorded in the master table M.

The doctor in charge H1a explains the treatment strategy indicated in the expert meeting to the patient P1 (VII in Fig. 1). When the patient P1 consents to the treatment strategy, the treatment is started (VIII in Fig. 1).

There may be multiple medical facilities. In this case, individual medical facilities are represented such as by reference numerals B1, B2, and B3.

A mutation of a nucleic acid sequence can be detected by a method including, by using a sample containing a nucleic acid derived from a tumor cell and a nucleic acid extracted from a sample containing a nucleic acid derived from a non-tumor cell, (process 1) acquiring first nucleic acid sequence data derived from a tumor cell collected from a patient, and second nucleic acid sequence data derived from a non-tumor cell collected from the same patient; and (process 2) detecting a somatic mutation on the basis of the first nucleic acid sequence data, or the first nucleic acid sequence data and the second nucleic acid sequence data; or (process 2') detecting a germline mutation on the basis of the second nucleic acid sequence data.

A tumor may include a benign epithelial tumor, a benign non-epithelial tumor, a malignant epithelial tumor, and a malignant non-epithelial tumor. An origin of the tumor is not limited. Examples of the origin of the tumor include respiratory system tissue such as a trachea, a bronchus, or a lung; digestive tract tissue such as a nasopharynx, an esophagus, a stomach, a duodenum, a jejunum, an ileum, a cecum, an appendix, an ascending colon, a transverse colon, a sigmoid colon, a rectum, or an anus; a liver; a pancreas; urinary system tissue such as a bladder, a ureter, or a kidney; female reproductive system tissue such as an ovary, a fallopian tube, and a uterus; a mammary gland; male reproductive system tissue such as a prostate gland; skin; endocrine system tissue such as hypothalamus, a pituitary gland, a thyroid gland, a parathyroid gland, and an adrenal gland; central nervous system tissue; bone and soft part tissue; hematopoietic system tissue such as bone marrow and a lymph node; a blood vessel; and the like.

The sample is a test sample containing a nucleic acid derived from a tumor cell, such as tissue, body fluid, excrement collected from a patient, and a test sample prepared from these. The body fluid is, for example, blood, bone marrow fluid, ascitic fluid, pleural effusion, spinal fluid, or the like. The excrement is, for example, stool and urine. A liquid obtained after washing a part of the patient's body, such as an intraperitoneal lavage fluid or a colon lavage fluid, may be used.

An amount of a nucleic acid contained in the sample is not limited as long as a nucleic acid sequence can be detected. When acquiring nucleic acid sequence data derived from a non-tumor cell, a sample containing a nucleic acid derived from a non-tumor cell is used. Concentration of the non-tumor cell contained in the tissue, body fluid, and the like is not limited as long as the nucleic acid sequence present in the non-tumor cell can be detected. When the tumor cell is derived from a solid tumor, for example, it is possible to use peripheral blood, oral mucosa tissue, skin tissue, and the like as the sample containing a non-tumor cell. When the tumor cell is derived from hematopoietic system tissue, it is possible to use oral mucosa tissue, skin tissue, and the like as the sample containing a non-tumor cell.

The sample can be collected from fresh tissue, fresh frozen tissue, paraffin-embedded tissue, or the like. The sample can be collected in accordance with a known method.

The sample containing a nucleic acid derived from a tumor cell and the sample containing a nucleic acid derived from a non-tumor cell are collected from the same patient. The test sample containing a nucleic acid derived from the non-tumor cell and the test sample containing a nucleic acid derived from the tumor cell may be collected at the same timing or may be collected at different timing. The nucleic acid may be DNA or RNA.

A gene whose nucleic acid sequence is to be analyzed is not limited as long as the gene exists on a human genome. The gene is desirably associated with tumor onset, prognosis, and therapeutic efficacy.

The germline mutation may be a disease-related mutation or gene sequence polymorphism. "Polymorphism" of a gene includes single nucleotide polymorphism (SNV), variable nucleotide of tandem repeat (VNTR), short tandem repeat polymorphism (STRP), and the like. A left column of Table 1 shows an example of genes from which a germline mutation may be detected. The genes listed in the left column of Table 1 are respectively related to diseases shown in a right column of the table.

**[Table 1]**

| Gene | Phenotype |
|---|---|
| BRCA1, BRCA2 | Hereditary Breast and Ovarian Cancer |
| TP53 | Li-Fraumeni Syndrome |
| STK11/LKB1 | Peutz-Jeghers Syndrome |
| MLH1, MSH2 | Lynch Syndrome |
| APC | Familial Adenomatous Polyposis |
| VHL | Von Hippel-Lindau Syndrome |
| RET | Multiple Endocrine Neoplasia Type 2 |
| | Familial Medullary Thyroid Cancer (FMTC) |
| PTEN | PTEN Hamartoma Tumor Syndrome |
| RB1 | Retinoblastoma |
| TSC1 | Tuberous Sclerosis Complex |
| SMAD4 | Juvenile Polyposis |

The nucleic acid sequence data is not limited as long as the data reflects a nucleic acid sequence. The nucleic acid sequence data may be nucleic acid sequence information itself, and may be data indicating a structure of the nucleic acid sequence or the presence or absence of a mutation in the nucleic acid sequence, or data indicating a structure of protein derived from the nucleic acid sequence. Preferably, the nucleic acid sequence data is the nucleic acid sequence information itself.

Acquisition of the nucleic acid sequence data is not limited as long as the method can acquire mutation information. For the acquisition of the nucleic acid sequence data, the nucleic acid sequence information itself may be acquired with use of a next-generation sequencer described later. In addition, by a PCR-Invader method, a PCR-RFLP method, a PCR-SSCP method, a Southern blotting method, a Northern blotting method, a Western blotting method, a FISH method, a microarray method, an immunostaining method, or the like, data indicating a structure of the nucleic acid sequence or the presence or absence of a mutation in the nucleic acid sequence, or data indicating a structure of protein derived from the nucleic acid sequence may be acquired as the nucleic acid sequence data. These methods for acquiring the nucleic acid sequence data are known. A method for acquiring the first nucleic acid sequence data derived from a tumor cell and a method for acquiring the second nucleic acid sequence data derived from a non-tumor cell are desirably the same method.

Detection of a somatic mutation and a germline mutation can be performed by comparing reference sequence data reported as a general sequence, with the first nucleic acid sequence data and the second nucleic acid sequence data. For example, in comparing the reference sequence data and the first nucleic acid sequence data, a mutation in the first nucleic acid sequence data can be detected by detecting a sequence in the first nucleic acid sequence data that is different from a sequence in the reference sequence data. Similarly, in comparing the reference sequence data and the second nucleic acid sequence data, a mutation in the second nucleic acid sequence data can be detected by detecting a sequence in the second nucleic acid sequence data that is different from a sequence in the reference sequence data.

In Fig. 2B and Figs. 3A to 3C, all test requests are displayed as a list, but list display may be changed in accordance with, for example, a test date, attribute information, the presence or absence of a setting of an expert meeting, and the like.

### II. Test request management system for gene panel testing

### 1. System configuration

With reference to Fig. 4, a description is given to a configuration of a management system 1000 (hereinafter, simply referred to as a system 1000) for managing, with a computer, a test request for gene panel testing. There may be multiple medical facilities connected to the system 1000. Here, an example is shown in which three medical facilities, a medical facility B 1, a medical facility B2, and a medical facility B3 are connected. The system 1000 includes a clinical information management device B10 and an expert meeting terminal B 15 that are installed in the medical facility B1, a clinical information management device B20 and an expert meeting terminal B25 that are installed in the medical facility B2, and a clinical information management device B30 and an expert meeting terminal B35 that are installed in the medical facility B3. The clinical information management device B10, the expert meeting terminal B15, the clinical information management device B20, the expert meeting terminal B25, the clinical information management device B30, and the expert meeting terminal B35 are communicably connected to the integrated data management device A via a wired or wireless network. The clinical information management device B10, B20, and B30 are management devices that integrally manage medical record information such as a test request, a test result, prescription information, meal information, and surgery information in a medical facility. The clinical information management devices B10, B20, and B30 are individually and communicably connected to electronic medical record databases (electronic medical record DBs) B1 1, B21, and B31, test image databases (test image DBs) B12, B22, and B32, and test request databases (test request DBs) B13, B23, and B33 via a wired or wireless network. The expert meeting terminals B15, B25, and B35 of individual medical facilities are used for displaying a graphical user interface UI outputted from the integrated data management device A, requesting to hold an expert meeting, and the like. In the present embodiment, in the clinical information management device B10, the expert meeting terminal B 15, the clinical information management device B20, the expert meeting terminal B25, the clinical information management device B30, and the expert meeting terminal B35, a dedicated application for accessing the integrated data management device A is installed.

The system 1000 includes a test information management device C11, a next-generation sequencer C13 connected to the test information management device C11, and an expert meeting terminal C15 of the test facility C1, that are installed in the test facility C1. The test information management device C11 and the expert meeting terminal C15 of the test facility C1 are communicably connected to the integrated data management device A via a wired or wireless network. The test information management device C11 analyzes a nucleic acid sequence by using nucleic acid sequence data acquired from the next-generation sequencer C13. The test information management device C11 also manages receipt of a sample, quality information of a sample and a test, a test progress status, and the like. The expert meeting terminal C15 of the test facility C1 is used by a clinical technologist and a bioinformatician who participate in an expert meeting, to display the graphical user interface UI outputted from the integrated data management device A and to participate in the expert meeting.

The system 1000 includes an expert meeting terminal SP11 installed in an external facility SP1. The expert meeting terminal SP11 of the external facility SP1 is communicably connected to the integrated data management device A via a wired or wireless network. The expert meeting terminal SP11 is used by a genetic counselor and a molecular genetics researcher who participate in an expert meeting, to display the graphical user interface UI outputted from the integrated data management device A and to participate in the expert meeting. There may be a plurality of external facilities. Here, a case of five external facilities is taken as an example, which are represented by external facilities SP1 to SP5. The expert meeting terminals installed in the external facilities are also represented by expert meeting terminals SP11 to SP15 of the external facilities. One of the expert meeting terminals of the external facilities, for example, the expert meeting terminal SP15, may be a terminal used by an expert meeting bureau that controls the expert meeting. The expert meeting terminal SP15 of the external facility is also called a bureau expert meeting terminal SP15. The expert meeting terminal SP15 is used to register a new expert meeting schedule slot in an expert meeting schedule database SDB.

The system 1000 may also include a drug information database (also simply referred to as a drug database or a drug DB) F11, a clinical trial information database (also simply referred to as a clinical trial database or a clinical trial DB) F21, and an article information database (also simply referred to as an article database or an article DB) F31 that are databases of an external institution. The drug information database F11, the clinical trial database F21, and the article database F31 are communicably connected to the integrated data management device A via a wired or wireless network.

Examples of the drug information database F11 include, for example, CanDL (https://candl.osu.edu/), Cancer Genome Interpreter (https://www.cancergenomeinterpreter.org/home), CIViC (https://civicdb.org/home), OncoKB (https://oncokb.org/), and the like. Examples of the clinical trial information database F21 include, for example, clinicaltrials.gov (https://clinicaltrials.gov/), and FAERS (https://www.fda.gov/Drugs/GuidanceComplianceRegulatory Information/Surveillance/ AdverseDrugEffects/ucm082193.htm). An example of the article information database F31 is PubMed (https://www.ncbi.nlm.nih.gov/pubmed/).

In the system 1000, since the integrated data management device A integrates test request information, attribute information, quality information, an expert meeting setting, and the like regarding gene panel testing, other device and terminal are sometimes called "other computers".

### 2. Integrated data management device

### 2-1. Hardware configuration of integrated data management device

Fig. 5 shows a hardware configuration of the integrated data management device A (also simply referred to as "management device A").

The integrated data management device A may be a general-purpose computer.

The integrated data management device A includes a control unit 100A, an input unit 106A, and an output unit 107A.

The control unit 100A includes a central processing unit (CPU) 101A that performs data processing described later, a memory 102A used as a temporary storage area for data processing, a storage device 103A that records a program and processing data described later, and a bus 104A that transmits data between individual units. The input unit 106A and the output unit 107A are connected to the control unit 100A. Exemplarily, the input unit 106A includes a keyboard, a mouse, a touch sensor, and the like. The output unit 107A includes a display, a printer, a speaker and the like. It is also possible to use a device having both functions of the input unit and the output unit, such as a touch panel in which a touch sensor and a display are integrated. An I/F unit 105A is an interface for the control unit 100A to communicate with an external device or a network. The control unit 100A may connect to a network 99 via the I/F unit 105A, to communicate with the clinical information management device B10, the expert meeting terminal B15 of the medical facility B1, the clinical information management device B20, the expert meeting terminal B25 of the medical facility B2, the clinical information management device B30, the expert meeting terminal B35 of the medical facility B3, the test information management device C11, the expert meeting terminal C15 of the test facility C1, the expert meeting terminal SP11 of an external facility, the expert meeting terminal SP15 of an external facility, the drug information database (drug information DB) F11, the clinical trial database (clinical trial DB) F21, and the article database (article DB) F31 that are databases of an external institution.

The storage device 103A has recorded, in advance, an operating system (OS), an application program to perform a process of each step shown in Figs. 19 to 22, 32, 33, 39, 42, 44, 48, 51, 55, and 57 below, and mail software, in the storage device 103A in an execution format, for example. The execution format is, for example, a format generated by converting from a programming language by a compiler. The control unit 100A uses each program recorded in the storage device 103A to perform each process shown in Figs. 19 to 22, 32, 33, 39, 42, 44, 48, 51, 55, and 57. In the storage device 103A, the master table M, and various databases to be used for processing described later such as the expert meeting schedule database SDB linked to the master table M are recorded. The control unit 100A updates information in the master table M on the basis of information transmitted from each clinical information management device, the test information management device, and the expert meeting terminal of each medical facility. The control unit 100A updates information in the expert meeting schedule database SDB on the basis of information transmitted from the expert meeting terminal of each medical facility, the expert meeting terminal of the test facility C1, and the expert meeting terminal of each external facility.

In the following description, unless otherwise noted, processing performed by the control unit 100A means processing performed by the CPU 101A on the basis of an application program stored in the storage device 103A or the memory 102A. The CPU 101A uses the memory 102A as a work area to temporarily store necessary data (intermediate data during processing, and the like) in a volatile manner. The CPU 101A appropriately stores data for long-term storage such as an analysis result in the storage device 103A in a non-volatile manner.

The application program may be downloaded from an external storage medium 98A such as a DVD or a USB memory, to be installed in the storage device 103A of the control unit 100A.

### 2-2. Functional configuration of control unit of integrated data management device

Fig. 6 shows a functional configuration of the control unit 100A of the integrated data management device A.

The control unit 100A of the integrated data management device A includes a test request reception unit A1, a test request transmission unit A3, a patient information reception unit A5, a test status management unit A7, a quality information management unit A11, an attribute information acquisition unit A15, a report acquisition unit A17, a test request list output unit A19, a schedule reception unit A20, a schedule output unit A21, a patient information output unit A23, a meeting content reception unit A25, a meeting content output unit A27, a master table update unit A50, a drug information DB access unit A31, a clinical trial DB access unit A33, an article DB access unit A35, and an integrated database OG. The integrated database OG stores the master table M, various data tables linked to the master table M, the expert meeting schedule database SDB, and the like.

Information in the master table update unit A50 and the master table M is updated by the test request reception unit A1, the patient information reception unit A5, the test status management unit A7, the quality information management unit A11, the attribute information acquisition unit A15, the report acquisition unit A17, the test request list output unit A19, the schedule reception unit A20, the patient information output unit A23, the meeting content reception unit A25, and the meeting content output unit A27. The schedule reception unit A20 stores a set date and time of an expert meeting received from the expert meeting terminals B15, B25, and B35 of individual medical facilities, in the expert meeting schedule database SDB.

The schedule output unit A21 transmits the set date and time of the expert meeting received from the expert meeting terminals B15, B25, and B35 of individual medical facilities, to the expert meeting terminals B15, B25, B35, C15, SP11, and SP15 of individual medical facilities by mail software or the like.

The drug information DB access unit A31, the clinical trial DB access unit A33, and the article DB access unit A35 are respectively connected to the drug information database F11, the clinical trial database F21, and the article database F31 via the I/F unit 105A.

### 2-3. Configuration of master table

Fig. 7 shows an example of the master table M.

The master table M includes an area for recording "patient ID" that is an identification label of a patient, an area for recording "sample ID" that is an identification label of a sample, an area for recording "test request ID" that is an identification label of a test request, an area for recording "gene panel ID" that is an identification label for gene panel testing, an area for recording "patient name", an area for recording "patient gender", an area for recording "patient date of birth", an area for recording "patient consent" that is informed consent information of the patient, an area for recording "test request date", an area for recording "medical person user ID" that is an identification label of a doctor in charge, an area for recording "medical person name" corresponding to the "medical person user ID", an area for recording "group ID" that is a label of a group in charge of an expert meeting, an area showing "test status" that is information indicating a test progress status, an area showing "patient information" that is information related to patient clinical information, an area for recording "test result" that is information regarding a result of gene panel testing, an area for recording "first attribute information" that is an outline of a test result and is information on the presence or absence of a gene mutation, an area for recording "second attribute information" that is an outline of a test result and is information regarding a type of a gene mutation, an area for recording "quality information" that is information regarding quality of a sample and a test, an area for recording "bureau facility ID" that is identification information of a bureau that leads the expert meeting, and an area for recording "holding date and time" of the expert meeting.

The master table M of Fig. 7 shows an example of using, in the gene panel testing, a first sample containing a tumor cell and a second sample containing a normal cell, as samples in one test of one patient. Since two types of samples are used in one test of one patient, in the master table M of Fig. 7, except for "sample ID", "test status", and "quality information", an individual test request display area UI3 in the first and second rows and an individual test request display area UI3 in the third and fourth rows have the same contents.

Each field in the master table M may be linked to another database or a data table. For example, a "patient information" field of the master table M of Fig. 7 is linked to a pathological image table PT shown in Fig. 8 that stores pathological image data corresponding to the sample ID. The pathological image data is one example of patient information transmitted from the clinical information management device B10 to the integrated data management device A, in step ST2 of Fig. 19 described later. The information stored in the pathological image table PT is transmitted from the clinical information management device B10 to the integrated data management device A. The pathological image table PT is recorded in the integrated database OG.

The field of "bureau facility ID" in the master table M in Fig. 7 is linked with the expert meeting group table GT shown in Fig. 9 including "group ID" of an expert meeting and "user ID included in group" in which identification information of a user included in the group is recorded. The "group ID" of the expert meeting group table GT corresponds to the "group ID" of the master table M. Two group IDs G01 and G02 correspond to the "facility ID" F01 in Fig. 9. Members identified by user IDs: U01, U02, U03, U04, U05... are registered in G01. Members identified by user IDs: U01, U04, U06, U10, U11... are registered in G01.

The field for recording the "medical person user ID" in the master table M of Fig. 7 is linked to a role table CT indicating a role of each medical person shown in Fig. 10A. Each medical person is identified in the system 1000 by a user ID. The role of each medical person is identified by the role ID shown in Fig. 10A. In Fig. 10A, a role ID "R01" is recorded for a user ID: U01, as identification information indicating the role. R02 is recorded as the "role ID" for the user IDs: U01 and U02. Depending on the role ID, information that can be displayed in the test request list described later may differ. A viewable information table AT shown in Fig. 10B is linked to the "role ID" shown in Fig. 10A. The viewable information table AT stores a list of information that can be displayed in a test request list described later in accordance with the role ID. For example, in the example shown in Fig. 10B, there are common items that can be viewed by all users of the system 1000 and additional items that can be additionally viewed in accordance with the role ID. In Fig. 10B, "test request date", "patient ID", and "attribute information" are exemplified as the common items. As additional items that can be viewed by a user having the role ID: R01, "meeting schedule information", "test status", "test result", and "patient information" are exemplified. As additional items that can be viewed by a user having the role ID: R02, "test status", "test result", "drug information", "clinical trial information", "article information", "patient IC information", and "patient information" are exemplified.

The expert meeting group table GT, the role table CT, and the viewable information table AT are generated in advance and recorded in the integrated database OG.

### 3. Clinical information management device

### 3-1. Hardware configuration of clinical information management device

Fig. 11 shows a hardware configuration of the clinical information management devices B10, B20, and B30. The clinical information management devices B10, B20, and B30 may be general-purpose computers. The hardware configuration of the clinical information management devices B10, B20, and B30 is basically similar to that of the integrated data management device A. In the clinical information management device B10, B20, and B30, the control unit 100A, the input unit 106A, the output unit 107A, the CPU 101A, the memory 102A, the storage device 103A, the bus 104A, and the I/F unit 105A in the integrated data management device A are to be replaced with a control unit 100B, an input unit 106B, an output unit 107B, a CPU 101B, a memory 102B, a storage device 103B, a bus 104B, and an I/F unit 105B.

The storage device 103B has recorded, in advance, an operating system (OS), a computer program to perform a process of each step shown in Fig. 19 and Fig. 53 below, a computer program to display an electronic medical record stored in the electronic medical record database (DB) B11, a computer program to display a test image stored in the test image database (DB) B 12, a computer program to make a test request in a hospital and the like, browser software to make a test request for gene panel testing, and browser software for display of display information and the like outputted from the integrated data management device A. The storage device 103B may store the electronic medical record database (DB) B11, the test image database (DB) B12, and the test request database (DB) B13.

The computer program and the browser software described above may be downloaded from an external storage medium 98B such as a DVD or a USB memory, to be installed in the storage device 103B.

The control unit 100B is connected to the network 99 via the I/F unit 105B to communicate with the integrated data management device A.

### 3-2. Functional configuration of control unit of clinical information management device

Fig. 12 shows a functional configuration of the control unit 100B of the clinical information management devices B10, B20, and B30.

The control unit 100B of the clinical information management device B10, B20, and B30 includes a test request information acquisition unit 1B, a test request information transmission unit 3B, a patient information transmission request reception unit 5B, a patient information transmission unit 7B, the electronic medical record database (DB) B11, the test image database (DB) B12, and the test request database (DB) B13. The electronic medical record database (DB) B11, the test image database (DB) B12, and the test request database (DB) B13 may be external to and communicatively connected to the clinical information management devices B10, B20, and B30.

### 4. Test information management device

### 4-1. Hardware configuration of test information management device

Fig. 13 shows a hardware configuration of the test information management device C11. The test information management device C11 may be a general-purpose computer.

The test information management device C11 includes a control unit 100, an input unit 106, and an output unit 107.

The control unit 100 includes a CPU 101 that performs data processing described later, a memory 102 used as a temporary storage area for data processing, a storage device 103 that records a program and processing data described later, a bus 104 that transmits data between individual units, and an I/F unit 105 that inputs and outputs data to and from an external device. The input unit 106 and the output unit 107 are connected to the control unit 100. Exemplarily, the input unit 106 includes a keyboard, a mouse, a touch sensor, and the like. The output unit 107 includes a display, a printer, a speaker and the like. It is also possible to use a device having both functions of the input unit and the output unit, such as a touch panel in which a touch sensor and a display are integrated. The I/F unit 105 is an interface for the control unit 100 to communicate with an external device.

The storage device 103 of the control unit 100 has recorded, in advance, an operating system, and an application program to perform a process of each step shown in Figs. 19, 20, 27, 28, 37, and 56 below, in the storage device 103 in an execution format, for example. The execution format is, for example, a format generated by converting from a programming language by a compiler. The control unit 100 uses the program recorded in the storage device 103 to perform a nucleic acid sequence analysis process, an attribute information acquisition process, and a report generation process.

In the following description, unless otherwise noted, processing performed by the control unit 100 means processing performed by the CPU 101 on the basis of a computer program stored in the storage device 103 or the memory 102. The CPU 101 uses the memory 102 as a work area to temporarily store necessary data (intermediate data during processing, and the like) in a volatile manner. The CPU 101 appropriately stores data for long-term storage such as an analysis result in the storage device 103 in a non-volatile manner.

The application program may be downloaded from an external storage medium 98 such as a DVD or a USB memory, to be installed in the storage device 103 of the control unit 100.

The test information management device C11 can be connected to a mutation information database 400 and a nucleic acid sequence data storage device 300 via the network 99.

The mutation information database 400 is an external public sequence information database, a public known mutation information database, or the like. Examples of the public sequence information database include NCBI RefSeq (web page, www.ncbi.nlm.nih.gov/refseq/), NCBI GenBank (web page, www.ncbi.nlm.nih.gov/genbank/), UCSC Genome Browser, and the like. Examples of the public known mutation information database include COSMIC database (web page, www.sanger.ac.uk/genetics/CGP/cosmic/), ClinVar database (web page, www.ncbi.nlm.nih.gov/clinvar/), dbSNP (web page, www.ncbi.nlm.nih.gov/SNP/), and the like. The mutation information database 400 may be a public known mutation information database containing frequency information for each race or animal category regarding a public known mutation. Examples of the public known mutation information database having such information include HapMap Genome Browser release #28, Human Genetic Variation Browser (web page, www.genome.med.kyoto-u.ac.jp/SnpDB/index.html), and 1000 Genomes (web page, www.1000genomes.org/). From these databases, for example, mutation frequency information and the like of Japanese can be obtained.

Examples of a sequencing technology applicable to the next-generation sequencer C13 include a sequencing technology such as ion semiconductor sequencing, pyrosequencing, sequencing-by-synthesis using a reversible dye terminator, sequencing-by-ligation, and sequencing by probe ligation of oligonucleotide, which can acquire multiple read sequences per run. The next-generation sequencer C13 sequences a nucleic acid sequence to acquire read sequence information as nucleic acid sequence information. A read sequence is a nucleic acid sequence obtained by sequencing. The next-generation sequencer C13 outputs read sequence information. The read sequence information may include a sequence name, a nucleic acid sequence, a sequencing quality score, and the like. Read sequence information acquired from a nucleic acid derived from a tumor cell is first nucleic acid sequence data, while read sequence information acquired from a nucleic acid derived from a non-tumor cell is second nucleic acid sequence data.

The nucleic acid sequence data storage device 300 is a computer that stores nucleic acid sequence data acquired by the next-generation sequencer C13.

### 4-2. Functional configuration of control unit of test information management device

Fig. 14 shows a functional configuration of the control unit 100 of the test information management device C11.

The control unit 100 of the test information management device C11 includes a test request information acquisition unit 11, a test status management unit 12, a read sequence information acquisition unit 1, a sequence determination unit 2, a mutation detection unit 3, an attribute information acquisition unit 4, a report generation unit 5, an information output unit 14, a form selection unit 9, a form database 17, a quality information acquisition unit 16, a test management database QCD, a reference sequence management unit 102a, a reference sequence generation unit 102b, a gene panel information database 121, a reference sequence database 6, and a mutation database 7.

The test request information acquisition unit 11 acquires information regarding a test request, from the integrated data management device A. The test status management unit 12 acquires sample receipt information, pretreatment information, a test progress status, and the like inputted by the clinical technologist T1 and the like, from the input unit 106. The quality information acquisition unit 16 acquires information regarding sample quality inputted by a clinical technologist T1 or the like from the input unit 106. Then, the quality information acquisition unit 16 records the information in a test management table L stored in the test management database QCD. The quality information acquisition unit 16 also acquires information regarding test quality such as sequencing acquired by the read sequence information acquisition unit 1. Then, the quality information acquisition unit 16 records the information in the test management database QCD.

### 5. Expert meeting terminal in medical facility

### 5-1. Hardware configuration of expert meeting terminal in medical facility

Fig. 15 shows a hardware configuration of the expert meeting terminals B15, B25, and B35 installed in the medical facilities B1, B2, and B3.

The expert meeting terminals B15, B25, and B35 installed in the medical facilities B1, B2, and B3 may be general-purpose computers. The hardware configuration of the expert meeting terminals B15, B25, and B35 is basically similar to that of the integrated data management device A. The control unit 100A, the input unit 106A, the output unit 107A, the CPU 101A, the memory 102A, the storage device 103A, the bus 104A, and the I/F unit 105A in the integrated data management device A are to be replaced with a control unit 100X, an input unit 106X, an output unit 107X, a CPU 101X, a memory 102X, a storage device 103X, a bus 104X, and an I/F unit 105X, in the expert meeting terminals B15, B25, and B35.

The storage device 103X stores, in advance, an operating system (OS), a computer program to perform a process of each step shown in Figs. 21, 22, 43, 23, 26, 30A to 30C, 31, 33, and 35 below, and browser software for display of display information and the like outputted from the integrated data management device A.

The browser software may be downloaded from an external storage medium 98X such as a DVD or a USB memory, to be installed in the storage device 103X.

The control unit 100X is connected to the network 99 via the I/F unit 105X to communicate with the integrated data management device A.

### 5-2. Functional configuration of control unit of expert meeting terminal of medical facility

Fig. 16 shows a functional configuration of the control unit 100X of the expert meeting terminals B15, B25, and B35 installed in the medical facilities B1, B2, and B3.

The control unit 100X of the expert meeting terminals B15, B25, and B35 installed in the medical facilities B1, B2, and B3 includes a schedule setting unit X1, a schedule reception unit X3, a test request list display request unit X5, a test request list output unit X7, a specific test request list output unit X9, a quality information output unit X11, an external database (DB) information output unit X13, an in-list link selection unit X15, a meeting content acquisition unit X17, and a meeting content output unit X19.

### 6. Expert meeting terminal of test facility and expert meeting terminal of external facility

### 6-1. Hardware configuration of expert meeting terminal of test facility and expert meeting terminal of external facility

Fig. 15 shows a hardware configuration of the expert meeting terminal C15 of the test facility C1 and the expert meeting terminal SP11 of the external facility SP1.

The expert meeting terminal C15 and the expert meeting terminal SP11 may be general-purpose computers. The hardware configuration of the expert meeting terminal C15 and the expert meeting terminal SP11 is basically similar to that of the integrated data management device A. The control unit 100A, the input unit 106A, the output unit 107A, the CPU 101A, the memory 102A, the storage device 103A, the bus 104A, and the I/F unit 105A in the integrated data management device A are to be replaced with a control unit 100Y, an input unit 106Y, an output unit 107Y, a CPU 101Y, a memory 102Y, a storage device 103Y, a bus 104Y, and an I/F unit 105Y, in the expert meeting terminal C15 of the test facility C1 and the expert meeting terminal SP11 of the external facility SP1.

The storage device 103Y stores, in advance, an operating system (OS), a computer program to perform a process of each step shown in Figs. 21, 22, 48, and 52 below, and browser software for display of display information and the like outputted from the integrated data management device A.

The browser software may be downloaded from an external storage medium 98Y such as a DVD or a USB memory, to be installed in the storage device 103Y.

The control unit 100Y is connected to the network 99 via the I/F unit 105Y to communicate with the integrated data management device A.

### 6-2. Functional configuration of control unit of expert meeting terminal of test facility and expert meeting terminal of external facility

Fig. 17 shows a functional configuration of the control unit 100Y of the expert meeting terminal C15 of the test facility C1, and the control unit 100Y of the expert meeting terminal SP11 of the external facility SP1.

The control unit 100Y of the expert meeting terminal C15 and the expert meeting terminal SP11 includes a schedule reception unit Y1, a test request list display request unit Y3, a test request list output unit Y5, a specific test request list output unit Y7, a quality information output unit Y9, an external database (DB) information output unit Y11, and an in-list link selection unit Y13.

### 7. Bureau expert meeting terminal

### 7-1. Hardware configuration of bureau expert meeting terminal

Fig. 15 shows a hardware configuration of the bureau expert meeting terminal SP15.

The bureau expert meeting terminal SP15 may be a general-purpose computer. The hardware configuration of the bureau expert meeting terminal SP15 is basically similar to that of the integrated data management device A. The control unit 100A, the input unit 106A, the output unit 107A, the CPU 101A, the memory 102A, the storage device 103A, the bus 104A, and the I/F unit 105A in the integrated data management device A are to be replaced with a control unit 100Z, an input unit 106Z, an output unit 107Z, a CPU 101Z, a memory 102Z, a storage device 103Z, a bus 104Z, and an I/F unit 105Z, in the bureau expert meeting terminal SP15.

The storage device 103Z stores, in advance, an operating system (OS), a computer program to perform a process of each step described in Figs. 21, 22, 48, 52, and 58 below, and browser software for display of display information and the like outputted from the integrated data management device A.

The browser software may be downloaded from an external storage medium 98Z such as a DVD or a USB memory, to be installed in the storage device 103Z.

The control unit 100Z is connected to the network 99 via the I/F unit 105Z to communicate with the integrated data management device A.

### 7-2. Functional configuration of control unit of bureau expert meeting terminal

Fig. 18 shows a functional configuration of the control unit 100Z of the bureau expert meeting terminal SP15.

The control unit 100Z of the bureau expert meeting terminal SP15 includes a schedule reception unit Z1, a test request list display request unit Z3, a test request list output unit Z5, a specific test request list output unit Z7, a quality information output unit Z9, an external database (DB) information output unit Z10, an in-list link selection unit Z11, a meeting content acquisition unit Z13, a meeting content output unit Z15, a new reservation slot registration unit Z17, and a schedule update unit Z19.

### 8. System operation

An operation of the management system 1000 for test request for gene panel testing will be described with reference to Figs. 19 to 22.

### 8-1. Flow of test request

In the system 1000, first, the medical facilities B1, B2, and B3 request gene panel testing of a patient having a tumor.

While there may be a plurality of medical facilities who participate in the system 1000, a description is given here to an operation with an example of the medical facility B1 with a case of using the clinical information management device B10 and the expert meeting terminal B 15 of the medical facility B1.

The control unit 100B of the clinical information management device B10 installed in the medical facility B1 (hereinafter, also simply referred to as a clinical information management device B10) receives, in step ST1 of Fig. 19, an input of a test request start by the doctor in charge H1a from the input unit 106B. At this time, the control unit 100B functions as the test request information acquisition unit 1B shown in Fig. 12. Processing of the test request information acquisition unit 1B will be described later.

The input of the test request is performed via a graphical user interface UIa shown in Fig. 23. The graphical user interface UIa may include a medical facility information input area UIa1 for input of information of a request source medical facility, a test request information input area UIa3 for input of test request information, and a request confirmation icon UIa7 for confirmation of a request. The medical facility information input area UIa1 is provided with an area UIa11 that displays a facility name, an area UIa13 for input of facility identification information (ID), an area UIa15 for input of an address of the facility, and an area UIa17 for input of facility contact information.

The test request information input area UIa3 is provided with an area UIa31 for input of a test type for specifying requested gene panel testing, an area UIa32 for input of a name of a doctor in charge of a patient for which the test is requested, an area UIa33 for input of identification information of the doctor in charge as a user in the gene panel testing, an area UIa34 for input of patient identification information (ID), an area UIa35 for input of information regarding patient's informed consent, an area UIa41 for input of a patient's name, an area UIa42 for input of patient's gender, an area UIa43 for input of a patient's date of birth, an area UIa44 for input of a test facility name to which the gene panel testing is requested, an area UIa51 for input of a test request date, an area UIa52 for input of a name of a facility serving as a bureau that leads an expert meeting, an area UIa53 for input of identification information (ID) of the facility serving as the bureau, an area UIa57 for input of an ID of a first sample containing a nucleic acid derived from a tumor cell, and an area UIa58 for input of an ID of a second sample containing a nucleic acid derived from a non-tumor cell.

When the doctor in charge H1a makes input in some or all of individual areas of the graphical user interface UIa from the input unit 106B of the clinical information management device B10, and selects the request confirmation icon UIa7, the clinical information management device B10 transmits a content inputted to the graphical user interface UIa, to the integrated data management device A as information related to the test request. At this time, the control unit 100B of the clinical information management device B10 functions as the test request information transmission unit 3B.

The control unit 100A of the integrated data management device A (hereinafter, simply referred to as an integrated data management device A) receives, in step ST21 of Fig. 19, the test request information transmitted from the clinical information management device B10 via the I/F unit 105A. At this time, the control unit 100A functions as the test request reception unit A1.

Subsequently, in step ST22, the integrated data management device A records the test request information in the master table M, to update the master table M. At this time, the control unit 100A functions as the master table update unit A50.

In the update process of the master table M in step ST22 of Fig. 19, information regarding the test request inputted from the graphical user interface UIa may be reflected in the master table M in the following correspondence relationship, for example.
A column indicating "patient ID" in the master table M, and the patient ID input area UIa34
A column indicating "sample ID" of the master table M, and the first sample ID input area UIa57 and the second sample ID input area UIa58
A "gene panel ID" area of the master table M, and the test type input area UIa31
A "patient name" area of the master table M, and the patient name input area UIa41
A "patient gender" area of the master table M, and the patient gender input area UIa42
A "patient date of birth" area of the master table M, and the patient date-of-birth input area UIa43
A "patient consent" area of the master table M, and the patient informed consent information input area UIa35
A "test request date" area of the master table M, and the test request date input area UIa51,
A "medical person user ID" area of the master table M, and a doctor-in-charge user ID input area UIa33
A "medical person name" area of the master table M, and a doctor-in-charge name input area UIa32
A "bureau facility" area of the master table M, and the bureau facility name input area UIa52.

Information regarding a test request other than the above, an input area of which is not shown in Fig. 7, is also stored in a predetermined area of the master table M.

Information inputted in the "test request ID" area of the master table M is, for example, given to the master table M in advance. When the integrated data management device A acquires information regarding a new test request, fields of the same row other than the "test request ID" area are automatically blank. By inputting acquired information regarding the new test request in fields of a row whose test request ID is the smallest, the information regarding the new test request can be associated with the test request ID in the master table M.

Next, in step ST23 of Fig. 19, the integrated data management device A transmits test request information acquired in step ST22 to the test information management device C11 via the I/F unit 105A. At this time, in addition to the acquired test request information, the test request ID given in step ST22 may be included in the test request information and transmitted to the test information management device C11. In step ST23, the control unit 100A of the integrated data management device A functions as the test request transmission unit A3. The transmission in step ST23 of Fig. 19 may be triggered by the integrated data management device A updating the master table M. An operator who operates the integrated data management device A may input a transmission request via the input unit 106A.

The control unit 100 of the test information management device C11 (hereinafter, also simply referred to as a test information management device C11) acquires, in step ST61, the test request information transmitted from the integrated data management device A via the I/F unit 105. The test information management device C11 stores the acquired test request information in the storage device 103. At this time, the control unit 100 of the test information management device C11 functions as the test request information acquisition unit 11. Fig. 24 shows an example of the test management table L to store data that is acquired by the test information management device C11 and is stored in the test information management device C11.

The information regarding the test request acquired in step ST61 of Fig. 19 may be reflected in the test management table L in the following correspondence relationship, for example.
A column indicating "patient ID" in the test management table L, and the patient ID input area UIa34
A column indicating "sample ID" of the test management table L, and the first sample ID input area UIa57 and the second sample ID input area UIa58
A "gene panel ID" area of the test management table L, and the test type input area UIa31
A "patient name" area of the test management table L, and the patient name input area UIa41
A "patient gender" area of the test management table L, and the patient gender input area UIa42
A "patient date of birth" area of the test management table L, and the patient date-of-birth input area UIa43
A "patient consent" area of the test management table L, and the patient informed consent information input area UIa35
A "test request date" area of the test management table L, and the test request date input area UIa51,
A "medical person user ID" of the test management table L, and the doctor-in-charge user ID input area UIa33
A "medical person name" area of the test management table L and the doctor-in-charge name input area UIa32

The test management table L may have the same configuration as that of the master table M, but an expert meeting bureau, a holding date and time, a group ID, patient information, and the like may not be present at a test stage.

Next, in step ST2 of Fig. 19, the clinical information management device B10 calls patient information of the patient for which the test request is made, from the electronic medical record database B11 or the test image database B12. Then, the clinical information management device B10 transmits the patient information to the integrated data management device A. At this time, the control unit 100B of the clinical information management device B10 functions as the patient information transmission unit 7B. The information transmitted in step ST2 of Fig. 19 may be included in test request information and transmitted when the test request information is transmitted in step ST1. The doctor in charge H1a may also input a transmission request from the input unit 106B to the control unit 100B. For example, the patient information may be transmitted by the doctor in charge H1a in step ST83 of Fig. 21 described later. At this time, the control unit 100B of the clinical information management device B10 functions as the patient information transmission request reception unit 5B (Fig. 12).

The integrated data management device A receives the patient information via the I/F unit 105A in step ST25 of Fig. 19. At this time, the control unit 100 of the integrated data management device A functions as the patient information reception unit A5. In step ST26 of Fig. 19, the integrated data management device A records the patient information in the master table M, to update the master table M. The patient information is stored in the "patient information" area of the master table M. At this time, the control unit 100 of the integrated data management device A functions as the master table update unit A50 (Fig. 6).

The processes of step ST2, step ST25, and step ST26 of Fig. 19 may be performed before step ST1 of Fig. 19. The processes above are simply required to be performed before an end of setting of the expert meeting (ST83 in Fig. 21) described later.

### 8-2. Flow of gene panel testing

Gene panel testing is started when the test information management device C11 receives test request information. When a sample of a patient collected at the medical facility B1 is carried into the test facility C1, for example, the clinical technologist T1 of the test facility C1 inputs a label of sample receipt in a test status area of the test management table L stored in the storage device 103, via the input unit 106 of the test information management device C11. A list of labels indicating a test status is shown in Fig. 25. The label of the test status may be appropriately inputted by the clinical technologist T1 along with progress of the test. This input is received by the test information management device C11. At this time, the control unit 100 of the test information management device C11 functions as the test status management unit 12 (Fig. 14).

In step ST63 of Fig. 19, the test information management device C11 transmits, as sample receipt information, the fact that a test status of the test management table L is updated to "sample receipt" in step ST62, to the integrated data management device A. At this time, the control unit 100 of the test information management device C11 functions as the test status management unit 12 (Fig. 14).

The integrated data management device A receives, in step ST27, the sample receipt information transmitted by the test information management device C11 in step ST63. At this time, the control unit 100A of the integrated data management device A functions as the test status management unit A7. The integrated data management device A updates the "test status" area of the master table M in step ST28, on the basis of the content received in step ST27. At this time, the control unit 100A of the integrated data management device A functions as the master table update unit A50 (Fig. 6). The information in the test management table L and the information in the master table M can be linked by, for example, the test request ID, the sample ID, the patient ID, and the test request date. The information transmission in step ST63 of Fig. 19 may be automatically performed when the test management table L is updated. Further, the information transmission may be performed by the clinical technologist T1 or the like inputting a transmission request from the input unit 106.

The sample carried into the test facility C1 is subjected to pretreatment such as nucleic acid extraction treatment and a nucleic acid quality test. When pretreatment of the sample is completed, in step ST64 of Fig. 19, via the input unit 106 of the test information management device C11, the clinical technologist T1 inputs a label indicating the completion of the pretreatment process shown in Fig. 25, in the test status area of the test management table L stored in the storage device 103. This input is received by the test information management device C11. At this time, the control unit 100 of the test information management device C11 functions as the test status management unit 12 (Fig. 14).

The test information management device C11 updates the test status of the test management table L to "pretreatment process completed" in step ST64. Then, the test information management device C11 transmits the information on the test status to the integrated data management device A in step ST65 of Fig. 19. The control unit 100 of the test information management device C11 functions as the test status management unit 12 (Fig. 14).

The integrated data management device A updates the "test status" area of the master table M on the basis of the test status information transmitted by the test information management device C11, in step ST29 of Fig. 20. At this time, the control unit 100A of the integrated data management device A functions as the master table update unit A50 (Fig. 6). The information in the test management table L and the information in the master table M can be linked by, for example, the test request ID, the sample ID, the patient ID, and the test request date. The information transmission in step ST65 may be automatically performed when the test management table L is updated. Further, the information transmission may be performed by the clinical technologist T1 or the like inputting a transmission request from the input unit 106.

The clinical technologist T1 then registers information regarding sample quality in "quality information" of the test management table L stored in the storage device 103, via the input unit 106 of the test information management device C11. The field of the "quality information" is linked to a sample quality information input table Q for input of sample quality information. Fig. 26 shows an example of the sample quality information input table Q. The sample quality information input table Q shown in Fig. 26 is provided with at least an extraction date, a test request ID, an input field of "extracted nucleic acid amount" for input of an absolute amount of a nucleic acid extracted for a first sample, an input field of "electrophoresis result" evaluated by electrophoresis as to whether the extracted nucleic acid is not excessively decomposed, an input field for input of "extracted nucleic acid amount" for a second sample, and an input field for input of "electrophoresis result". The extraction date is a date on which a nucleic acid has been extracted. The "sample request ID" of the sample quality information input table Q corresponds to the "test request ID" of the test management table L and the master table M. Samples with "test request ID" T01 and T02 shown in the sample quality information input table Q can be said to have sufficient quality as a nucleic acid sample to be used for sequencing, since an amount of the extracted nucleic acid is sufficient for both the first sample and the second sample, and the electrophoresis result is also favorable. However, for a nucleic acid sample derived from the first sample with the "test request ID" of T03, a nucleic acid amount is a detection limit or less, and a sufficient nucleic acid sample has not been obtained. In such a case, even if no mutation is detected by sequencing, it is not possible to adopt the test result. Therefore, it is necessary to collect the sample again.

In step ST66 of Fig. 20, the test information management device C11 receives information inputted by the clinical technologist T1 to a sample quality information table via the input unit 106. Then, the test information management device C11 records the information in the storage device 103. At this time, the control unit 100 of the test information management device C11 functions as the quality information acquisition unit 16 (Fig. 14).

Next, in step ST67 of Fig. 20, the test information management device C11 transmits, as sample quality information, the content recorded in the sample quality information table in step ST66, to the integrated data management device A. The control unit 100 of the test information management device C11 functions as the information output unit 14 (Fig. 14).

In step ST31, the integrated data management device A receives sample quality information transmitted by the test information management device C11 in step ST67. At this time, the control unit 100A of the integrated data management device A functions as the quality information management unit A11 (Fig. 6). The integrated data management device A updates the "quality information" area of the master table M in step ST32. At this time, the control unit 100A of the integrated data management device A functions as the master table update unit A50 (Fig. 6). The information in the test management table L and the information in the master table M can be linked with, for example, the test request ID and the like. The information transmission in step ST67 may be automatically performed when the test management table L is updated. Further, the information transmission may be performed by the clinical technologist T1 or the like inputting a transmission request from the input unit 106.

Next, in step ST68 of Fig. 20, the test information management device C11 performs sequencing of a nucleic acid sample extracted from the first sample and a nucleic acid sample extracted from the second sample by the next-generation sequencer C13, to perform mutation analysis of the nucleic acid sequence. An outline of a nucleic acid sequence mutation analysis process will be described later.

In step ST68 of Fig. 20, when the sequencing is performed, quality of sequencing analysis is acquired for each test batch (one sequencing is one batch, and samples of a plurality of patients are subjected to sequencing for each batch) by the next-generation sequencer C13, and a quality control report (also called QC report) of the test is recorded in the test management database QCD of the storage device 103.

In step ST69 of Fig. 20, the test information management device C11 registers test quality information by storing or associating the QC report corresponding to each sample subjected to the sequencing, in a field of the quality information corresponding to each sample of the test management table L. In steps ST68 and ST69 of Fig. 20, the control unit 100 of the test information management device C11 functions as the quality information acquisition unit 16 (Fig. 14).

When quality information of a new test is registered in the "quality information" field in step ST69, the test information management device C11 transmits, in step ST70 of Fig. 20, the content recorded in the test management table L to the integrated data management device A as test quality information. At this time, the control unit 100 of the test information management device C11 functions as the information output unit 14 (Fig. 14).

In step ST33, the integrated data management device A receives the test quality information transmitted in step ST70 of Fig. 20. In step ST34, the integrated data management device A records the test quality information in the "quality information" area of the master table M, to update the master table M. At this time, the control unit 100A of the integrated data management device A functions as the master table update unit A50 (Fig. 6). The information in the test management table L and the information in the master table M can be linked with, for example, the test request ID and the like. The information transmission in step ST70 of Fig. 20 may be automatically performed when the test management table L is updated. Further, the information transmission may be performed by the clinical technologist T1 or the like inputting a transmission request from the input unit 106.

Next, in step ST71 of Fig. 20, the test information management device C11 acquires attribute information indicating an outline of a test result of the gene panel testing, on the basis of a mutation analysis result detected in step ST68. Details of the attribute information acquisition process will be described later. The test information management device C11 records the acquired attribute information in the attribute information field of the test management table L. In step ST71, the control unit 100 of the test information management device C11 functions as the attribute information acquisition unit 4 (Fig. 14).

When new attribute information is registered in the "attribute information" field in step ST71, the test information management device C11 transmits, in step ST72 of Fig. 20, the content recorded in the test management table L to the integrated data management device A, as attribute information. At this time, the control unit 100 of the test information management device C11 functions as the information output unit 14 (Fig. 14).

In step ST35, the integrated data management device A receives the attribute information transmitted in step ST72 of Fig. 20. In step ST36, the integrated data management device A records the attribute information in the "attribute information" area of the master table M, to update the master table M. The information in the test management table L and the information in the master table M can be linked with, for example, the test request ID and the like. The information transmission in step ST72 of Fig. 20 may be automatically performed when the test management table L is updated. Further, the information transmission may be performed by the clinical technologist T1 or the like inputting a transmission request from the input unit 106.

Next, in step ST73 of Fig. 20, the test information management device C11 performs a report generation process of a test result of the gene panel testing on the basis of the mutation analysis result detected in step ST68. At this time, the control unit 100 of the test information management device C11 functions as the report generation unit 5 (Fig. 14). Details of the report generation process will be described later. At this time, the control unit 100 of the test information management device C11 functions as the information output unit 14 (Fig. 14).

The test information management device C11 registers a test result report by storing the report generated in step ST73 of Fig. 20 in a test result field corresponding to each test request ID in the test management table L, or providing a link.

When a new test result is registered in the "test result" field in step ST73, the test information management device C11 transmits, in step ST74 of Fig. 20, the content recorded in the test management table L to the integrated data management device A, as test result information.

In step ST37, the integrated data management device A receives the test result information transmitted in step ST74 of Fig. 20. In step ST38, the integrated data management device A records the test quality information in the "test result" area of the master table M, to update the master table M. The information in the test management table L and the information in the master table M can be linked with, for example, the test request ID and the like. The information transmission in step ST74 of Fig. 20 may be automatically performed when the test management table L is updated. Further, the information transmission may be performed by the clinical technologist T1 or the like inputting a transmission request from the input unit 106.

The gene panel testing report generated by the test information management device C11 in step ST73 of Fig. 20 is outputted from the output unit 107 such as a printer, to be sent to the medical facility B 1 as a paper medium.

In Fig. 20, through the processing from steps ST21 to ST28 shown in Fig. 19 and steps ST29 to ST38 shown in Fig. 20, the integrated data management device A acquires information regarding the test request from the clinical information management device B10, or the clinical information management device B10, B20, or B30 of a first group, which is another computer different from the integrated data management device A. Further, the integrated data management device A acquires attribute information, a test result, sample and test quality management information from the test information management device C11, which is different from the integrated data management device A and the clinical information management devices B10, B20, and B30. The integrated data management device A has integrated these pieces of information, to record individual information in the master table M shown in Fig. 7 and a table linked to the master table.

Information recorded in the master table M and information recorded in a table linked to the master table M can be displayed as a test request list or as a link from a test list, from the expert meeting terminals B15, B25, and B35 of the medical facilities B1, B2, and B3, the expert meeting terminal C15 of the test facility C1, the expert meeting terminal SP11 of the external facility SP1, and the bureau expert meeting terminal SP15, which are shown in Fig. 4. That is, the integrated data management device A can output the information recorded in the master table M to each expert meeting terminal as a test request list. Information recorded in a table linked to the master table M can also be outputted via a link provided in a corresponding item of the test request list.

Each expert meeting terminal can display a test request list and information linked to the test request list via browser software stored in a storage device of each terminal.

An example of the test request list is as shown in Figs. 2A and 2B and 3.

### (1) Mutation analysis process

An outline of mutation analysis will be described below with reference to Figs. 27 and 28. Details of the mutation analysis is according to the method described in U. S. Patent Application Publication No. 2019/156914.

The test information management device C11 acquires a read (tumor read sequence) of a nucleic acid sequence derived from a tumor cell from a nucleic acid sample acquired from the first sample and acquires a read of a nucleic acid sequence derived from a normal cell (normal read sequence) from a nucleic acid sample acquired from the second sample, to use for the mutation analysis.

The test information management device C11 determines whether or not a tumor carried by a patient has a somatic mutation on the basis of the tumor read sequence and the normal read sequence. The normal read sequence is also used to determine whether the patient carries a germline mutation.

Detection of a somatic mutation and a germline mutation can be performed by comparing reference sequence data reported as a general sequence, with the tumor read sequence and the normal read sequence. For example, when comparing the reference sequence data and the first nucleic acid sequence data, a mutation in the tumor read sequence can be detected by detecting a sequence in the tumor read sequence different from a sequence in the reference sequence data. Similarly, when comparing the reference sequence data and the normal read sequence, a mutation in the normal read sequence can be detected by detecting a sequence in the normal read sequence different from a sequence in the reference sequence data. Instead of the reference sequence data, the mutation reference sequence data described in U.S. Patent Application Publication No. 2019-156914 may be used to detect a mutation.

Information regarding a germline mutation is not limited as long as the information is related to a germline mutation carried by the patient for which the nucleic acid sequence is analyzed. For example, the information regarding a germline mutation may include at least a label indicating a name of a gene in which the mutation has been detected. Preferably, the information regarding a germline mutation may include a label indicating a name of a gene in which the mutation has been detected, information on the detected nucleic acid sequence, and/or information on an amino acid sequence generated by the mutation. As described in the section of I. Outline of embodiment, locus information of the gene in which the mutation has been detected, reference sequence information, and information on a mutation sequence held by the patient may be included. The information regarding a germline mutation is not limited to the information on detection as to whether or not there is a mutation, but may be information implying possibility of a germline mutation (for example, a mosaic mutation).

### (1-1) Detection of somatic mutation

With reference to Figs. 13, 14, and 27, a description will be given to an example of an operation of the control unit 100 for the test information management device C11 to detect a somatic mutation.

In step ST201 of Fig. 27, the control unit 100 of the test information management device C11 (hereinafter, simply referred to as a test information management device C11) acquires a read sequence from the nucleic acid sequence data storage device 300 shown in Fig. 13. At this time, the control unit 100 of the test information management device C11 functions as the read sequence information acquisition unit 1 shown in Fig. 14. The acquired read sequence includes a normal read sequence and a tumor read sequence.

In step ST202 of Fig. 27, the test information management device C11 aligns each of the normal read sequence and the tumor read sequence with the reference sequence. At this time, the control unit 100 of the test information management device C11 functions as the sequence determination unit 2 shown in Fig. 14.

In step ST203 of Fig. 27, the test information management device C11 determines whether or not a mismatch with the reference sequence is present in the tumor read. When a mismatch with the reference sequence is present in the tumor read (when "Yes"), the process proceeds to ST204. Then, it is determined whether or not a mismatch with the reference sequence is absent in the normal read. When a mismatch with the reference sequence is absent in the normal read (when "Yes"), the process proceeds to step ST205. Then, the mutation existing in the tumor read is determined to be a somatic mutation. The test information management device C11 identifies a gene name, locus, and a mismatch site of the reference sequence corresponding to the read sequence having the mismatch.

In step ST206 of Fig. 27, the test information management device C11 searches the mutation database 7 on the basis of the detected mutation. The mutation database 7 in Fig. 14 is constructed based on the external mutation information database 400 such as COSMIC or ClinVar, for example. In one aspect, each piece of mutation information in the database may be assigned with metadata of information regarding a gene panel.

Next, in step ST207 of Fig. 27, the test information management device C11 assigns an annotation to the detected mutation on the basis of a search result of step ST206. Fig. 29 shows an example of the search result and the annotation. Fig. 29 includes information of, from the left, "mutation ID" showing identification information of a mutation, "CHROM" indicating a chromosome number including a mutation site, "POS" indicating a position number of the mutation site, "REF" indicating a nucleotide sequence of the reference sequence, "ALT" indicating a mutation sequence, and "Annotation" specifically indicating what kind of mutation it is. Assignment of the Annotation can be omitted.

When the test information management device C11 determines that the tumor read has no mismatch with the reference sequence ("No") in step ST203, the test information management device C11 determines in step ST208 that there is no somatic mutation. Then, the test information management device C11 ends the process.

After step ST207 in Fig. 27, the result shown in Fig. 29 may be outputted. In steps ST203 to ST207 shown in Fig. 27, the control unit 100 of the test information management device C11 functions as the mutation detection unit 3 shown in Fig. 14.

### (1-2) Detection of germline mutation

With reference to Figs. 13, 14, and 28, a description will be given to an example of an operation of the control unit 100 for the test information management device C1 to detect a germline mutation.

In step ST301 of Fig. 28, the test information management device C11 acquires a read sequence from the nucleic acid sequence data storage device 300 shown in Fig. 13. At this time, the control unit 100 of the test information management device C11 functions as the read sequence information acquisition unit 1 in Fig. 14. The acquired read sequence includes a normal read sequence.

In step ST302 of Fig. 28, the test information management device C11 aligns the normal read sequence with the reference sequence. At this time, the control unit 100 of the test information management device C11 functions as the sequence determination unit 2 shown in Fig. 14.

In step ST303 of Fig. 28, the test information management device C11 determines whether or not the normal read has a mismatch with the reference sequence. When there is a mismatch with the reference sequence in the normal read (when "Yes"), the mutation detection unit 3 proceeds to ST304. Then, the mutation detection unit 3 determines that the mutation existing in the normal read is a germline mutation. The test information management device C11 identifies a gene name, locus, and a mismatch site of the reference sequence corresponding to the read sequence having the mismatch.

In step ST305 of Fig. 28, the test information management device C11 searches the mutation database 7 shown in Fig. 14 on the basis of the identified mutation.

Next, in step ST306 of Fig. 28, the test information management device C11 assigns an annotation to the detected mutation on the basis of a search result of step ST305. This step is similar to step ST207 in Fig. 27.

When the test information management device C11 determines that the normal read has no mismatch with the reference sequence ("No") in step ST303 of Fig. 28, the test information management device C11 determines that there is no germline mutation in step ST307. Then, the test information management device C11 ends the process. In steps ST303 to ST306 shown in Fig. 28, the control unit 100 of the test information management device C11 functions as the mutation detection unit 3 shown in Fig. 14.

### (2) Attribute information acquisition process

With reference to Figs. 13, 14, and 30A to 30C to 33, an operation for the test information management device C11 to acquire attribute information will be described.

In a test item of gene panel testing, mutation analysis of multiple genes is included in one panel. The first attribute information and the second attribute information are included in the gene panel testing. Therefore, the first attribute information is given with a "mutation present" label when a mutation is found in at least one gene to be tested (also referred to as a predetermined gene) included in the gene panel testing. When a mutation is found in at least one gene to be tested (also referred to as a predetermined gene) included in the gene panel testing, the second attribute information is also given with a label indicating a mutation type. For example, if both an actionable mutation and a germline mutation are found in single gene panel testing, labels of both will be given.

### (2-1) First acquisition mode

A first acquisition mode of attribute information is a method in which the clinical technologist T1 or the bioinformatician T2 determines an attribute indicating an outline of a test result on the basis of the test result, and inputs a determination result from the input unit 106 of the test information management device C11 shown in Fig. 13, and accordingly the test information management device C11 receives this input.

Figs. 30A to 30C show an example of a graphical user interface for the clinical technologist T1 or the like to input attribute information to the test information management device C11, and an input example thereof. Fig. 30A shows a graphical user interface UIc in a case where a germline mutation has been detected in a test target gene of the gene panel testing. The graphical user interface UIc includes, as the first attribute information, a selection area UIc1 for selection as to whether or not a mutation has been detected in the test target gene. The graphical user interface UIc also includes, as the second attribute information, a selection area UIc2 for selection as to whether or not an actionable mutation has been detected, whether or not a germline mutation has been detected, and whether an other mutation has been detected.

Fig. 30B shows a graphical user interface UId in a case where a germline mutation has not been detected in a test target gene of the gene panel testing. A selection area UId1 in Fig. 30B is similar to the selection area UIc1 in Fig. 30A. A selection area UId2 in Fig. 30B is similar to the selection area UIc2 in Fig. 30A except that there is no input field for a germline mutation. The control unit 100 of the test information management device C11 determines whether to display the graphical user interface UIc or the graphical user interface UId on the basis of the test result. If the patient does not consent to disclosure of a germline mutation, the graphical user interface UId may be displayed even if the germline mutation is detected.

The clinical technologist T1 or the like selects the corresponding check box in each selection area by using a mouse or the like, which is the input unit 106 shown in Fig. 13.

An input from the input unit 106 shown in Fig. 13 is recorded in fields of the first attribute information and the second attribute information of the test management table shown in Fig. 30C.

Input of attribute information from the input unit 106 shown in Fig. 13 may be performed by selecting corresponding attribute information from options of a label indicating an attribute in a list format. As selection of the list format, a pull-down type graphical user interface UIe shown in Fig. 31 can be exemplified.

An actionable mutation is intended to, for example, a mutation that can be expected to have therapeutic efficacy of 3A or more of evidence level classification shown in the "Clinical practice guidance for next-generation sequencing in cancer diagnosis and treatment". The evidence level classification of therapeutic efficacy is classified into seven stages of 1A, 1B, 2A, 2B, 3A, 3B and 4. "3A or more" is intended to be a mutation classified into any of 1A, 1B, 2A, 2B, and 3A. Information regarding what kind of gene mutation is classified at what evidence level can be acquired from "Table 2. Evidence Levels of Gene Panel Testing Results" (as of August 21, 2017) in "Clinical practice guidance for next-generation sequencing in cancer diagnosis and treatment".

### (2-2) Second acquisition mode

A second acquisition mode of attribute information is a method for acquiring the attribute information on the basis of a mutation detected by the test information management device C11 through the processing shown in Figs. 27 and 28. With reference to Figs. 32 to 34A and 34b, a process in which the test information management device C11 acquires attribute information will be described.

In step ST501 of Fig. 32, the test information management device C11 acquires mutation information acquired through the processing shown in Figs. 27 and 28. The mutation information is recorded in the storage device 103 as a detection result table G as shown in Fig. 34A, for example. For example, in Fig. 34A, the detection result table G includes a "data ID" field showing identification information of data acquired from the next-generation sequencer C13, a "test request ID" field, a "link to result report" field with a link to a folder that stores a test result, a "detected mutation" field showing a name of a gene in which a mutation has been detected, and a "panel ID" field showing a gene panel ID.

In step ST502 of Fig. 32, the test information management device C11 determines whether or not a mutation is absent in the result, on the basis of the mutation information acquired in step ST501. For example, the storage device 103 of the test information management device C11 has recorded a determination table shown in Fig. 34B in advance.

The determination table H in Fig. 34B is created for each gene panel ID. An example in which the gene panel ID is P001 is shown here. In the determination table H of Fig. 34B, a name of a gene in which a mutation can be detected in a gene panel of P001 is recorded in a "mutation targeted for first attribute information" field. The first attribute information of a gene mutation a, a gene mutation b, a gene mutation c,... a gene mutation i, and a gene mutation z, that is, the presence or absence of a mutation can be detected. A gene targeted for second attribute information is recorded separately for "actionable mutation", "germline mutation", and "other mutation". A gene mutation a and a gene mutation b are recorded in the "actionable mutation" field, a gene mutation h and a gene mutation i are recorded in the "germline mutation" field, and a gene mutation v, a gene mutation w, and a gene mutation x are recorded in the "other mutation" field.

In step ST502 of Fig. 32, the test information management device C11 determines whether or not the gene mutation a and the gene mutation i recorded in step ST501 and stored in the "detected mutation" field of the detection result table G shown in Fig. 34A are absent in the "mutation targeted for first attribute information" field of the determination table H of Fig. 34B.

When the "mutation targeted for first attribute information" field of the determination table H of Fig. 34B has no gene mutation stored in the "detected mutation" field of the detection result table G, step ST502 in Fig. 32 is to be YES. In this case, the test information management device C11 proceeds to step ST503 in Fig. 32. Then, the test information management device C11 assigns and records a label of "no mutation" in a first attribute information field of the test management table L shown in Fig. 30C.

In the example of Figs. 34A and 34B, since there are the gene mutation a and the gene mutation i in the "mutation targeted for first attribute information" field of the determination table H, step ST502 of Fig. 32 is to be NO. In this case, the test information management device C11 proceeds to step ST504 in Fig. 32. Then, the test information management device C11 assigns and records a label of "mutation present" in the first attribute information field of the test management table L shown in Fig. 30C. Next, the test information management device C11 proceeds to step ST601 in Fig. 33.

In Fig. 33, the second attribute information, that is, a mutation type is determined.

Next, in step ST601 of Fig. 33, the test information management device C11 identifies a gene in which a mutation has been found. For the identification of the gene having a mutation, a search is performed to determine, in the "mutation targeted for second attribute information" field of the determination table H of Fig. 34B, which mutation field has the gene mutation a and the gene mutation i recorded in step ST501 of Fig. 32 and stored in the "detected mutation" field of the detection result table G shown in Fig. 34A.

When the gene stored in the "detected mutation" field of the detection result table G shown in Fig. 34A is in the "actionable mutation" field of the determination table H of Fig. 34B, step ST602 of Fig. 33 is to be YES, and the test information management device C11 proceeds to step ST603 in Fig. 33. Then, a label indicating that there is "actionable mutation" is given and recorded in the "second attribute information" field of the test management table shown in Fig. 30C.

When a gene stored in the "detected mutation" field of the detection result table G shown in Fig. 30A is not in the "actionable mutation" field of the determination table H of Fig. 34B (when step ST602 of Fig. 33 is NO) and after step ST603 in Fig. 33, the test information management device C11 proceeds to step ST604 in Fig. 33.

When the gene is in the "germline mutation" field of the determination table H in Fig. 34B, step ST604 in Fig. 33 is to be YES, and the test information management device C11 proceeds to step ST605 in Fig. 33. Then, a label indicating that there is "germline mutation" is given and recorded in the "second attribute information" field of the test management table shown in Fig. 30C.

When the gene stored in the "detected mutation" field of the detection result table G shown in Fig. 34A is not in the "germline mutation" field of the determination table H of Fig. 34B (when step ST604 of Fig. 33 is NO), the test information management device C11 proceeds to step ST606 of Fig. 33. Then, in step ST607 of Fig. 33, a label indicating that there is "other mutation" is given and recorded in the "second attribute information" field of the test management table shown in Fig. 30C.

The attribute information recorded in the test management table L by the attribute information acquisition process is transmitted to the integrated data management device A in step ST70 of Fig. 20.

In steps ST501 to ST504 shown in Fig. 32 and steps ST601 to ST607 shown in Fig. 33, the control unit 100 of the test information management device C11 functions as the attribute information acquisition unit 4 shown in Fig. 14.

### (3) Report generation process

The test information management device C11 generates a test report (report) of a test result acquired by the processing shown in Figs. 27 and 28. In gene panel testing, a germline mutation is a mutation that is found incidentally or additionally. A mutation in a nucleic acid sequence other than for a testing purpose may be sometimes referred to as an incidental finding in the present specification. It is necessary to carefully consider whether or not to inform the patient about the incidental finding or additional information, based on a type of the gene in which the mutation has been detected, the mutation type, severity of a disease that develops associated with the mutation, possibility of treatment, an intention of the patient and a relative of the patient regarding being informed of information about a germline mutation, and the like. Therefore, in the gene panel testing, informed consent (IC) for confirming the intention of the patient and a relative of the patient regarding being informed of information about a germline mutation is usually obtained before the test.

In the present embodiment, a format of the report can be changed according to IC information of the patient. For example, when a germline mutation is detected, it is possible to select whether to generate an analysis report in a format of a normal report R1 or to generate a report in a format exemplified in a confidential report R2.

An example of the format of the normal report R1 will be described with reference to Fig. 35. The format of the normal report R1 is an example including an area S of a summary report, which is a first area (hereinafter, also referred to as "summary report area S"), and an area DT of a detailed report, which is a second area (hereinafter, also referred to as "detailed report area DT"). The summary report area S further includes an area S1 showing a part of test request information indicating information regarding a patient or a test content (hereinafter, also referred to as "request information area S1"), and an area S2 showing a list of all detected gene mutations (hereinafter, also referred to as "mutation list area S2"). The detailed report area DT includes an area DT1 showing detailed information of a gene and a mutation thereof detected in a nucleic acid sequence derived from a first sample (containing a tumor cell) (hereinafter, also referred to as "gene mutation information area DT1"), and an area DT2 showing detailed information of a gene and a mutation thereof in which a germline mutation has been detected in a nucleic acid sequence derived from a second sample (containing a non-tumor cell) (hereinafter, also referred to as "germline mutation information area DT2").

In Fig. 35, an attribute information area S1 may display information for identification of a patient such as a patient identifier (ID), a name of a doctor in charge, and a name of a medical facility, and information indicating a test item such as a gene panel. The gene mutation list area S2 may display all detected gene mutations regardless of being a somatic mutation or a germline mutation. In the example of the gene mutation list area S2 shown in Fig. 35, EGFR, BRAF, and BRCA1 represent gene names, and L858R, V600E, and K1183R represent mutation sites. Therefore, EGFR_L858R indicates that a codon at the 858th amino acid of the EGFR gene is mutated from a nucleic acid sequence encoding leucine (L) to a nucleic acid sequence encoding arginine (R).

The summary report area S is an area that may be displayed to the patient, the doctor in charge, an expert in gene analysis, and the like.

The gene mutation information area DT1 may include information such as a name of a gene in which a mutation has been detected, a mutation identifier (ID), a locus number of a gene in which a mutation has been detected (including chromosome number: CROM and mutation position: POS), a nucleic acid sequence of a reference sequence (REF), a detected mutation sequence (ALT), and an annotation when showing a detected mutation in an analysis report.

The germline mutation information area DT2 may include information such as a name of a gene in which a mutation has been detected, a mutation identifier (ID), a locus number of a gene in which a mutation has been detected (including chromosome number: CROM and mutation position: POS), a nucleic acid sequence of a reference sequence (REF), a detected mutation sequence (ALT), and an annotation when showing a detected mutation in an analysis report.

The detailed report area DT can be presented to at least an expert in gene analysis. The detailed report area DT may not be necessarily presented to the patient or the doctor in charge.

In the example of Fig. 35, the germline mutation information area DT2 shows that there is a germline mutation "BRCA1_K1183R" in the BRCA1 gene, and "BRCA1_K1183R" is also shown in the gene mutation list area S2 of the summary report area S. In other words, the normal report R1 shown in Fig. 35 presents the germline mutation "BRCA1_K1183R" shown in the gene mutation list area S2 to the patient.

Fig. 36 shows an example of the format of the confidential report R2. The confidential report R2 includes a summary report area S and a detailed report area DT, similarly to the normal report R1. The confidential report R2 is an example in which, even if a germline mutation is detected, the summary report area S and the detailed report area DT do not show information regarding the germline mutation. For example, in the format of the confidential report R2, no information regarding the germline mutation "BRCA1_K1183R" is described in the gene mutation list area S2. Further, detailed information on "BRCA1_K1183R", which is detailed information on a germline mutation, is not described in the detailed report area DT. The confidential report R2 is an example in which information regarding a germline mutation is not presented in both the summary report area S and the detailed report area DT. The confidential report may be a form in which the information regarding the germline mutation is not presented in the summary report area S exclusively.

In step ST141 of Fig. 37, the test information management device C11 receives a report output request inputted from the input unit 106 of Fig. 13 by the clinical technologist T1 or the bioinformatician T2. In step ST142 of Fig. 37, when it is determined that there is a germline mutation in step ST304 of Fig. 28 (when step ST142 of Fig. 37 is "Yes"), the test information management device C11 proceeds to step ST143 of Fig. 37. In step ST143 of Fig. 37, the test information management device C11 refers to a "patient consent" field of the test management table L shown in Fig. 24. Then, when the patient does not consent to disclosure of a germline mutation, the test information management device C11 determines that confidentiality of information regarding the germline mutation is "necessary" (step ST143 in Fig. 37 is "Yes"). When the patient consents to disclosure of a germline mutation in the "patient consent" field of the test management table L shown in Fig. 24, the test information management device C11 determines that the confidentiality of the information regarding the germline mutation is "unnecessary" (step ST143 in Fig. 37 is "No").

When step ST143 of Fig. 37 is "Yes", the test information management device C11 proceeds to step ST144. Then, the test information management device C11 calls the format of the confidential report R2 shown in Fig. 36 from the form database 17 via the form selection unit 9, to generate a report.

When step ST143 of Fig. 37 is "No", the test information management device C11 proceeds to step ST145. Then, the test information management device C11 calls the format of the normal report R1 from the form database 17 shown in Fig. 35 via the form selection unit 9, to generate a report.

After generating the report, the test information management device C11 proceeds to step ST74 of Fig. 20. In steps ST141 to ST145 shown in Fig. 37, the control unit 100 of the test information management device C11 functions as the report generation unit 5 shown in Fig. 14.

### 8-3. Report output process from test request list

In step S38 of Fig. 20, after recording the test result in the master table M, the integrated data management device A may select and output a report format from a link provided in the test request list.

Fig. 38 shows an example of the test request list area UI1 in which a link for report format selection is displayed. For example, for a test T02 in the test request list area UI1 shown in Fig. 38, "mutation present" and "germline mutation" are displayed as result attribute information. As described above, when a germline mutation is confirmed, it is necessary to output a report based on the patient IC information. Therefore, a "registered" label indicating that the patient IC information is registered is displayed in the "IC information" area of the test request list area UI1. In the "patient Information" area, a "format selection" label for selection of the report form is displayed.

When outputting a report from the integrated data management device A, the form of each report is stored in the integrated database OG.

Fig. 39 shows a flow of a report generation process by the control unit 100A of the integrated data management device A.

In step ST151, the control unit 100A determines whether or not a "germline mutation" label is recorded as the second attribute information in a field corresponding to a test request of the patient for which the report is to be generated, in the master table M. When the "germline mutation" label is recorded ("Yes" in step ST151), the process proceeds to step ST152.

In step ST152, the control unit 100A determines whether or not the patient IC information has been recorded in a field corresponding to the test request of the patient for which the report is to be generated, in the master table M. When the patient IC information is recorded ("Yes" in step ST152), the process proceeds to step ST153.

In step ST153, the control unit 100A outputs a link to the patient IC information in the field corresponding to the test request of the patient for which the report is to be generated, in the test request list area UI1 shown in Fig. 38.

In step ST154 of Fig. 39, the control unit 100A outputs a link to a report format selection screen to the field corresponding to the test request of the patient for which the report is to be generated, in the test request list area UI1 shown in Fig. 38.

The user can display the IC information of the patient for which the report is to be generated from the IC information link, and can know whether the patient wants to know a result of an incidental finding. The user can also select the report form in accordance with the patient IC information.

In step ST155 of Fig. 39, the control unit 100A receives user's selection of a link to the format selection screen. Then, the control unit 100A outputs form selection dialog shown in Fig. 40.

When the user selects a "No" icon W23 in the dialog of Fig. 40, the format of the confidential report R2 shown in Fig. 36 is selected. When the user selects a "Yes" icon W24 in the dialog of Fig. 40, the format of the normal report R1 shown in Fig. 35 is selected.

In step ST156 of Fig. 39, the control unit 100A receives the user's selection of the "No" icon W23 or the "Yes" icon W24 shown in Fig. 40. Then, the control unit 100A outputs the report in the selected form, in step ST157.

### 8-4. Display of test request list and expert meeting setting

Returning to Fig. 21, a continuation of the operation of the system 1000 will be described.

The operation of the system shown in Figs. 21 and 22 is to be communication of the integrated data management device A between with the expert meeting terminals B15, B25, and B35 of the medical facilities B1, B2, and B3, the expert meeting terminal C15 of the test facility C1, the expert meeting terminal SP11, and the bureau expert meeting terminal SP15.

With reference to Figs. 13 to 18, 20, and 21, a display process for a test request list and an expert meeting setting process in the system 1000 will be described. Since the expert meeting terminal B15 of the medical facility B1, the expert meeting terminal B25 of the medical facility B2, and the expert meeting terminal B35 of the medical facility B3 have the same processing content, processing will be described using the expert meeting terminal B15 of the medical facility B1.

When the doctor in charge H1a displays a test result of a patient for which gene panel testing is requested, the doctor in charge H1a accesses the integrated data management device A via browser software, from the expert meeting terminal B15 of the medical facility B1 provided in the medical facility B1. In step ST81 of Fig. 21, the control unit 100X of the expert meeting terminal B15 of the medical facility B1 shown in Fig. 15 (hereinafter, simply referred to as "expert meeting terminal B15 of the medical facility B1") receives a display request for the test request list area UI1 shown in Fig. 3, from the doctor in charge H1a via the input unit 106X. Then, the display request for the test request list area UI1 is transmitted to the integrated data management device A via the I/F unit 105X. At this time, the control unit 100X functions as the test request list display request unit X5 shown in Fig. 16.

In step ST39 shown in Fig. 21, the integrated data management device A receives the display request from the expert meeting terminal B15 of the medical facility B1. Subsequently, the integrated data management device A outputs the test request list area UI1 shown in Fig. 3 in step ST40 shown in Fig. 21. At this time, the control unit 100A functions as the test request list output unit A19 shown in Fig. 6.

In step ST82, the expert meeting terminal B15 of the medical facility B1 displays the test request list area UI1 shown in Fig. 3 outputted from the integrated data management device A, on the output unit 107X such as a display via browser software. At this time, the control unit 100X functions as the test request list output unit X7 shown in Fig. 16.

The doctor in charge H1a sets a schedule of an expert meeting from the expert meeting terminal B15 of the medical facility B1. In step ST83, the expert meeting terminal B15 of the medical facility B1 receives a setting of a meeting schedule by the doctor in charge H1a from the input unit 106X shown in Fig. 15. At this time, the control unit 100X functions as the schedule setting unit X1 shown in Fig. 16. Details of the setting process for the expert meeting will be described later.

The integrated data management device A performs a schedule setting process in ST41 shown in Fig. 21. Details of the schedule setting process will be described later. At this time, the control unit 100A functions as the schedule reception unit A20 shown in Fig. 6.

Subsequently, the integrated data management device A outputs the set expert meeting schedule in step ST42 of Fig. 21. This output is, for example, transmission of the expert meeting schedule to each participant in the expert meeting, by using mail software. At this time, the control unit 100A functions as the schedule output unit A21 shown in Fig. 6.

The mail of the expert meeting schedule transmitted from the integrated data management device A is received by mail software of the expert meeting terminal B15 of the medical facility B 1 in step ST84 of Fig. 21. At this time, the control unit 100X functions as the schedule reception unit X3 shown in Fig. 16. In step ST101 of Fig. 21, mail software of the expert meeting terminal SP11 also receives the mail of the expert meeting schedule. At this time, the control unit 100Y shown in Fig. 15 functions as the schedule reception unit Y1 shown in Fig. 17.

Prior to the expert meeting, each participant in the expert meeting can display a test request list of a patient to be examined in the expert meeting on each expert meeting terminal.

In the following, the expert meeting terminal B 15 of the medical facility B 1 and the expert meeting terminal SP11 can display the test request list by similar processing.

An example of communication between the expert meeting terminal B 15 of the medical facility B1 and the integrated data management device A will be described first.

The doctor in charge H1a accesses the integrated data management device A via browser software, from the expert meeting terminal B15 of the medical facility B 1 provided in the medical facility B 1.

In step ST85 of Fig. 21, the expert meeting terminal B 15 of the medical facility B1 shown in Fig. 15 receives a display request for the test request list area UI1 shown in Fig. 3, from the doctor in charge H1a from the input unit 106X. Then, the display request for the test request list area UI1 is transmitted to the integrated data management device A via the I/F unit 105X. At this time, the control unit 100X functions as the test request list display request unit X5 shown in Fig. 16.

In step ST43 shown in Fig. 21, the integrated data management device A receives the display request from the expert meeting terminal B 15 of the medical facility B 1. Then, the integrated data management device A outputs the test request list area UI1 shown in Fig. 3. At this time, the control unit 100A functions as the test request list output unit A19 shown in Fig. 6.

In step ST86, the expert meeting terminal B 15 of the medical facility B1 displays the test request list area UI1 shown in Fig. 3 outputted from the integrated data management device A, on the output unit 107X such as a display via browser software. At this time, the control unit 100X functions as the test request list output unit X7 shown in Fig. 16.

Each participant can request display for a specific test request assigned to the participant in the expert meeting handled by the participant. For example, by providing a sorting function and an extraction function in the test request list, the participant can rearrange the test request list in accordance with the "holding date and time" of the expert meeting, and extract the test request assigned to the participant with the "group ID" of the expert meeting participated by the participant. Step ST88 shown in Fig. 22 is a step in which the expert meeting terminal B15 of the medical facility B 1 makes a display request for such a specific test request. In this step, the control unit 100X functions as the test request list display request unit X5.

In step ST45 shown in Fig. 22, the integrated data management device A receives a display request from the expert meeting terminal B 15 of the medical facility B1. Then, the integrated data management device A outputs a specific test request from the test request list area UI1 shown in Fig. 3. At this time, the control unit 100A functions as the test request list output unit A19 shown in Fig. 6. The output process for the specific test request will be described later.

In step ST89, the expert meeting terminal B 15 of the medical facility B1 displays the test request list area UI1 shown in Fig. 3 outputted from the integrated data management device A, on the output unit 107X such as a display via browser software. At this time, the control unit 100X functions as the specific test request list output unit X9 shown in Fig. 16.

The expert meeting terminal B15 of the medical facility B1 receives an input of a meeting content by the doctor in charge H1a from the input unit 106X shown in Fig. 15 in the expert meeting (step ST90). At this time, the control unit 100X functions as the meeting content acquisition unit X17 shown in Fig. 16. Subsequently, in step ST91, the expert meeting terminal B 15 of the medical facility B 1 transmits the meeting content received in step ST90 to the integrated data management device A. At this time, the control unit 100X functions as the meeting content output unit X19 shown in Fig. 16.

In step ST46, the integrated data management device A receives the meeting content transmitted from the expert meeting terminal B 15 of the medical facility B 1. In step ST47, the integrated data management device A records the meeting content in the integrated database OG in association with the test request ID and the patient information, to update the master table.

The expert meeting terminal C15 of the test facility C1, the expert meeting terminal SP11 of the external facility SP1, and the bureau expert meeting terminal SP15 perform, in steps ST101 to ST107 of Figs. 21 and 22, similar processing to that of steps ST84 to ST91 performed by the expert meeting terminal B15 of the medical facility B1.

Regarding the processing performed by the expert meeting terminal C15 of the test facility C1 and the expert meeting terminal SP11 of the external facility SP1, the control unit 100X, the I/F unit 105X, the input unit 106X, and the output unit 107X of the expert meeting terminal B 15 of the medical facility B1 shown in Fig. 15 are to be replaced with the control unit 100Y, the I/F unit 105Y, the input unit 106Y, and the output unit 107Y of the expert meeting terminal C15 of the test facility C1 or the expert meeting terminal SP11 shown in Fig. 15. The schedule reception unit X3, the test request list display request unit X5, the test request list output unit X7, and the specific test request list output unit X9 showing functions of the control unit 100X of the expert meeting terminal B 15 of the medical facility B 1 shown in Fig. 16 are to be replaced with the schedule reception unit Y1, the test request list display request unit Y3, the test request list output unit Y5, and the specific test request list output unit Y7 shown in Fig. 17, respectively.

Regarding the processing performed by the bureau expert meeting terminal SP15, the control unit 100X, the I/F unit 105X, the input unit 106X, and the output unit 107X of the expert meeting terminal B 15 of the medical facility B 1 shown in Fig. 15 are to be replaced with the control unit 100Z, the I/F unit 105Z, the input unit 106Z, and the output unit 107Z of the bureau expert meeting terminal SP15 shown in Fig. 15. The schedule reception unit X3, the test request list display request unit X5, the test request list output unit X7, the specific test request list output unit X9, the meeting content acquisition unit X17, and the meeting content output unit X19 showing functions of the control unit 100X of the expert meeting terminal B 15 of the medical facility B 1 shown in Fig. 16 are to be replaced with the schedule reception unit Z1, the test request list display request unit Z3, the test request list output unit Z5, the specific test request list output unit Z7, the meeting content acquisition unit Z13, and the meeting content output unit Z15 shown in Fig. 18.

### (1) Expert meeting setting

With reference to Fig. 41 to Fig. 46, a description is given to details of the graphical user interface UI that is for setting of an expert meeting, a test request list output process of step ST40 shown in Fig. 21, an expert meeting setting process in step ST83, and the schedule setting process in step ST41.

### (1-1) Graphical user interface

Fig. 41 shows an example of the graphical user interface UI that enables setting of an expert meeting. A "setting status" area of the test request list area UI1 displays a label of "set" or "unset" indicating whether or not a schedule of an expert meeting has been set. When the schedule of the expert meeting has been set, the set date and time is displayed in a "holding date and time" area. When the schedule of the expert meeting has not been set, "-" is shown in the "holding date and time" area.

### (1-2) Dialog display

Fig. 42 shows a process in which the integrated data management device A displays a link to dialog for setting an expert meeting in the "setting status" field of the graphical user interface UI shown in Fig. 41.

In step ST221 of Fig. 42, the integrated data management device A determines whether or not the setting status of the expert meeting is unset. This determination can be made based on whether or not a holding date and time has been inputted in the "holding date and time" field of each test request ID in the master table M shown in Fig. 7.

When the holding date and time is not recorded in the "holding date and time" field of the master table M shown in Fig. 7 (when step ST221 is "Yes"), the integrated data management device A outputs the "unset" label to the "setting status" field of the graphical user interface UI shown in Fig. 41, in step ST222 of Fig. 42. The "unset" label has a link for outputting dialog UI5 shown in Fig. 45 or dialog UI7 shown in Fig. 46.

The dialog UI5 shown in Fig. 45 may display a date and time of a vacant slot of the meeting schedule stored in advance in the meeting schedule database SDB, the number of entries that can be set within the date and time of the vacant slot, and a check box that can be selected by the doctor in charge H1a from the input unit 106X of the expert meeting terminal B 15 of the medical facility B1 shown in Fig. 15. The dialog UI7 shown in Fig. 46 may display an area UI71 that displays patient identification information for identification of the patient, such as a patient name, gender, a date of birth, and the like, an area UI73 that displays a list of members who participate in the expert meeting, an area UI75 for schedule setting of the expert meeting, and a meeting notification icon UI77 that receives selection by the doctor in charge H1a to confirm the setting and notify each participant in the meeting by mail that the meeting has been set.

The patient name, the gender, the date of birth, and the like are read from a corresponding area in the master table M shown in Fig. 7. The list of members who participate in the expert meeting is read from the expert meeting group table GT (Fig. 9) linked to the area of "group ID" of the master table M shown in Fig. 7. The area UI75 for schedule setting of the expert meeting corresponds to Fig. 45.

### (1-3) Medical facility expert meeting terminal side

A specific step of the expert meeting setting process in step ST83 shown in Fig. 21 will be described with reference to Fig. 43.

In step ST231 shown in Fig. 43, the expert meeting terminal B 15 of the medical facility B 1 uses browser software to display the "unset" label including a link to expert meeting setting dialog outputted by the integrated data management device A in step ST222 of Fig. 42.

The expert meeting terminal B15 of the medical facility B1 determines, in step ST232 shown in Fig. 43, whether selection of the link by the doctor in charge H1a from the input unit 106X has been received.

When the selection of the link to the expert meeting setting dialog is received (when "Yes") in step ST232 shown in Fig. 43, the expert meeting terminal B15 of the medical facility B1 proceeds to step ST233. Then, the expert meeting terminal B15 displays the dialog UI5 shown in Fig. 45 or the dialog UI7 shown in Fig. 46 on the output unit 107X such as a display of the expert meeting terminal B 15 of the medical facility B 1. In step ST232 shown in Fig. 43, when the selection of the link to setting dialog for the expert meeting is not received (when "No"), the process waits.

In step ST234 shown in Fig. 43, the expert meeting terminal B 15 of the medical facility B 1 receives selection of a schedule by the doctor in charge H1a from the input unit 106X. The reception of the selection the schedule is performed when the doctor in charge H1a checks a check box for selection of a date and time for which the expert meeting is desired to be started from the input unit 106X, and this is received by the expert meeting terminal B 15 of the medical facility B 1.

In step ST235 shown in Fig. 43, the expert meeting terminal B15 of the medical facility B 1 transmits information received in step ST234 to the integrated data management device A. For the transmission in step ST235 of the information received in step ST234, at a time when the doctor in charge H1a selects the check box shown in Fig. 45, the expert meeting terminal B15 of the medical facility B 1 may receive this, and simultaneously transmit the information to the integrated data management A. The expert meeting terminal B15 of the medical facility B1 may also transmit the information to the integrated data management device A at a time when the doctor in charge H1a selects the check box shown in Fig. 46, and the doctor in charge H1a further selects the meeting notification icon UI77.

### (1-4) Integrated data management device side

Next, a specific step of the schedule setting process of step ST41 shown in Fig. 21 will be described with reference to Fig. 44.

In step ST241 of Fig. 44, the integrated data management device A determines whether or not selection of a link to the setting dialog for the expert meeting is received from a browser of the expert meeting terminal B15 of the medical facility B 1. When the selection of the link is received from the expert meeting terminal B15 of the medical facility B 1 (when step ST241 of Fig. 44 is "Yes"), the integrated data management device A proceeds to step ST242, and acquires a schedule having a vacant slot from the meeting schedule database SDB. Next, the integrated data management device A proceeds to step ST243. Then, the integrated data management device A outputs the dialog UI5 shown in Fig. 45 or the dialog UI7 shown in Fig. 46 including vacant slot information of the schedule, to the expert meeting terminal B15 of the medical facility B1.

In step ST244 of Fig. 44, the integrated data management device A receives information on the schedule that is transmitted by the expert meeting terminal B15 of the medical facility B1 and selected by the doctor in charge H1a. In step ST245, the integrated data management device A records the information in the meeting schedule database SDB. When the expert meeting is set, the integrated data management device A displays the "set" label in the "setting status" field of the test request list area UI1 shown in Fig. 41. The schedule of the expert meeting set in the "holding date and time" field is displayed.

### (2) Link to quality information

The integrated data management device A may output quality information to the test request list area UI1 as shown in Fig. 47, in the test request list output process of step ST43 shown in Fig. 21. When no mutation is detected in gene panel testing, it is necessary to evaluate, at the expert meeting, as to whether or not the determination of no somatic mutation in step ST208 shown in Fig. 27, or the determination of no germline mutation in step ST307 shown in Fig. 28 has been appropriate, after considering information regarding sample quality such as whether a nucleic acid has been properly obtained from a sample, or information regarding test quality such as whether there has been no problem in reaction in sequencing. A display example of the graphical user interface UI shown in Fig. 47 is useful for examining suitability of a sample and a test at the expert meeting.

Fig. 48 shows a display process for the graphical user interface UI of Fig. 47 in the test request list output process of step ST43 shown in Fig. 21.

### (2-1) Integrated data management device side

In step ST251 shown in Fig. 48, in the master table M corresponding to a test request ID of a patient to be examined in the expert meeting, the integrated data management device A determines whether or not there is a "no mutation" label indicating that there is no mutation in the "first attribute information" area of the master table M shown in Fig. 7.

In step ST251 shown in Fig. 48, when the integrated data management device A determines that there is the "no mutation" label (when "Yes"), the integrated data management device A proceeds to step ST252.

Next, in step ST252 of Fig. 48, the integrated data management device A confirms whether quality information of the test and the sample has been acquired, and the quality information of the test and the sample has been recorded in the "quality information" field of the master table M shown in Fig. 7.

When the quality information of the test and the sample has been acquired (when "Yes") in step ST252 of Fig. 48, the integrated data management device A proceeds to step ST253.

In step ST253, the integrated data management device A generates a link to the "quality information" field of the master table M shown in Fig. 7, in the "registered" label indicating that the quality information is registered, which is outputted in the "quality information" field of the test request list area UI1 shown in Fig. 47. Association between the "quality information" field of the test request list area UI1 and the "quality information" field of the master table M can be made with the test request ID or the sample ID corresponding to the test request ID.

In step ST254 of Fig. 48, the integrated data management device A outputs the "registered" label provided with the link generated in step ST253.

The integrated data management device A waits for access to the label provided with the link outputted in step ST254 from the expert meeting terminal C15 of the test facility C1 described later. Then, the integrated data management device A outputs quality information of the link destination in step ST255 of Fig. 48.

### (2-2) Expert meeting terminal side

At the expert meeting, quality of the gene panel testing is evaluated on the basis of the quality information, mainly by the clinical technologist T1 and the bioinformatician T2 at the test facility C1.

In step ST103 shown in Fig. 21, when the clinical technologist T1 and the bioinformatician T2 who participate in the expert meeting display quality information from the test request list area UI1, in step ST261 shown in Fig. 49, the expert meeting terminal C15 of the test facility C1 uses browser software to display a "registered" label provided with the link outputted by the integrated data management device A in step ST254 of Fig. 48, in the quality information field of the test request list area UI1 displayed on the output unit 107Y such as a display shown in Fig. 15.

In step ST262 shown in Fig. 49, the expert meeting terminal C15 of the test facility C1 receives selection of the label displayed in step ST254 of Fig. 48 from the input unit 106Y shown in Fig. 15. Input from the input unit 106Y is made by the clinical technologist T1 and the bioinformatician T2.

In step ST263 shown in Fig. 49, the expert meeting terminal C15 of the test facility C1 displays quality information of the link destination outputted by the integrated data management device A in step ST255 of Fig. 48.

### (3) Link to external database

When the second attribute information of "actionable mutation" or "other mutation" is given as a result of gene panel testing, information regarding a treatment method or the like may be examined from an external database, in an expert meeting, in accordance with a type of a gene in which a mutation has been detected, a site of the mutation, and the like.

In the test request list output process of step ST43 shown in Fig. 21, as shown in Fig. 50, the integrated data management device A may output fields of "result registration" indicating that the test result of the test request list area UI1 has been registered, and of "information DB" displaying a link to an external information database (DB), in accordance with the second attribute information.

The "result registration" field indicates that the test result has been registered in the "test result" field of the master table M shown in Fig. 7. The "result registration" field displays the label "registered" provided with a link to the "test result" field of the master table M. The "result registration" field of the test request list area UI1 shown in Fig. 50 and the "test result" field of the master table M shown in Fig. 7 are associated by the test request ID or the sample ID corresponding to the test request ID.

The "Information DB" field may display a "drug DB" label provided with a link to the drug information database server F11, a "clinical trial DB" label provided with a link to the clinical trial database server F21, and an "article DB" label provided with a link to the article database server F31, in accordance with the second attribute information.

By displaying such a link to an information database, a participant in the expert meeting can access information regarding mutation information of a gene of a patient to be examined from the test request list area UI1, which improves convenience.

### (3-1) Integrated data management device side

Fig. 51 shows a generation process for a link to an external database and an output process for the link, in the integrated data management device A.

In step ST271 of Fig. 51, the integrated data management device A determines whether or not the test result has been recorded in the "test result" field of the master table M shown in Fig. 7.

When the "test result" is not recorded in step ST271 of Fig. 51 (when "No"), the integrated data management device A waits until the "test result" is recorded in the master table M. This is because, since information search in the external database is performed based on a specific gene name or mutation site, it is not possible to perform the information search before a detailed test result is registered even if a link to an external database is displayed.

When the "test result" is recorded (when "Yes") in step ST271 of Fig. 51, the integrated data management device A proceeds to step ST272. Then, the integrated data management device A outputs the "registered" label to the "result registration" field of the test request list area UI1 in Fig. 50.

Subsequently, the integrated data management device A proceeds to step ST273 of Fig. 51. Then, the integrated data management device A refers to the "first attribute information" field of the master table M shown in Fig. 7. When the "mutation present" label is included in the "first attribute information field" (when step ST273 in Fig. 51 is "YES"), the integrated data management device A proceeds to step ST274 in Fig. 51. When the "mutation present" label is not included in the "first attribute information field" (when step ST273 in Fig. 51 is "NO"), the integrated data management device A ends the process.

In step ST274 of Fig. 51, the integrated data management device A refers to the "second attribute information" of the master table M shown in Fig. 7. Then, the integrated data management device A determines whether or not there is a label of "actionable mutation" and/or "germline mutation" in the "second attribute information" field. In step ST274 of Fig. 51, when there is the label of "actionable mutation" and/or "germline mutation" (when "Yes"), the integrated data management device A proceeds to step ST275 in Fig. 51. Then, the integrated data management device A outputs labels of "drug DB" and "clinical trial DB". When there is no label of "actionable mutation" and/or "germline mutation" (when "No") in step ST274 of Fig. 51, a label of "other mutation" is given to the "second attribute information" field. In this case, the integrated data management device A proceeds to step ST276 in Fig. 51, to output the "article DB" label.

To the "drug DB", the "clinical trial DB", and the "article DB", URLs for accessing the corresponding servers are linked. The link of each database is stored in the integrated database OG of the integrated data management device A shown in Fig. 4.

The integrated data management device A waits for access from each expert meeting terminal to the "information DB" field of the test request list area UI1 shown in Fig. 50. Then, the integrated data management device A receives selection of any of the "drug DB" label, the "clinical trial DB" label, or the "article DB" label in step ST277 shown in Fig. 51.

Subsequently, the integrated data management device A outputs a link destination of the selected label in step ST278 shown in Fig. 51.

### (3-3) Expert meeting terminal side

At the expert meeting, each participant refers to an external database.

In steps ST86 and ST103 shown in Fig. 21, each participant at the expert meeting accesses the external database from the test request list area UI1 shown in Fig. 50.

In step ST281 shown in Fig. 52, each of the expert meeting terminals B15, B25, B23, C15, SP11, and SP15 uses browser software to display a "registered" label outputted by the integrated data management device A in step ST272 of Fig. 51, in the "result registration" field of the test request list area UI1 displayed on the output units 107X, 107Y, and 107Z such as a display shown in Fig. 15.

In step ST282 shown in Fig. 52, each of the expert meeting terminals B15, B25, B23, C15, SP11, and SP15 uses the browser software to display the test request list area UI1 including the label of "drug DB", "clinical trial DB", or "article DB" outputted in step ST275 or step ST276 in Fig. 51.

In step ST283 shown in Fig. 52, each of the expert meeting terminals B15, B25, B23, C15, SP11, and SP15 receives selection of the label displayed in step ST282 of Fig. 52, from the input unit 106X, 106Y, and 106Z shown in Fig. 15. Input from the input units 106X, 106Y, and 106Z is made by each participant in the expert meeting.

In step ST284 shown in Fig. 52, each of the expert meeting terminals B15, B25, B23, C15, SP11, and SP15 displays the external database of the link destination outputted by the integrated data management device A in step ST278 of Fig. 51.

### 9. Computer program

The following steps may be executed on a computer as a computer program for managing a test request for gene panel testing: step ST21 to step ST38 shown in Fig. 19 and Fig. 20, steps ST501 to ST504 shown in Fig. 32, steps ST601 to ST607 shown in Fig. 33, and steps ST321 to ST338 shown in Fig. 55; or steps ST21 to ST47 shown in Figs. 19 to 22, steps ST501 to ST504 shown in Fig. 32, and steps ST601 to ST607 shown in Fig. 33, steps ST221 and ST222 shown in Fig. 42, steps ST241 to ST245 shown in Fig. 44. steps ST251 to ST255 shown in Fig. 48, steps ST271 to ST278 shown in Fig. 51, and steps ST321 to ST338 shown in Fig. 55.

The computer program can be provided as a program product such as a storage medium. The computer program is stored in a storage medium such as a hard disk, a semiconductor memory device such as a flash memory, or an optical disk. A storage format of the program in the storage medium is not limited as long as the control unit can read the program. The storage in the storage medium is desirably non-volatile.

### III. Other processing and modified example

### (1) User authentication process at test request

A description is given to an example of the test request process of step ST1 of Fig. 19 performed by the test request information acquisition unit 1B of the clinical information management device B10 shown in Fig. 12 mentioned in "Flow of test request" in 8-1 above. Fig. 53 shows a flow of the user authentication process of step ST1 of Fig. 19.

In step ST111 of Fig. 53, when the doctor in charge H1a makes a test request for gene panel testing with the clinical information management device B10, the doctor in charge H1a logs in to access the graphical user interface UIa that is shown in Fig. 23 and stored in the integrated database OG of the integrated data management device A. The clinical information management device B10 receives, from the input unit 106B shown in Fig. 11, a processing start command by the doctor in charge H1a, that is, for example, selection of a URL of a gene panel testing request. Then, the clinical information management device B10 uses browser software to display a login screen on the output unit 107B such as a display shown in Fig. 11.

Subsequently, in step ST112 shown in Fig. 53, the clinical information management device B10 receives input of a user ID and a password by the doctor in charge H1a, from the input unit 106B shown in Fig. 11. Then, the clinical information management device B10 transmits the user ID and the password to the integrated data management device A.

The integrated database OG shown in Fig. 4 stores a login information table in which a user ID and a login password shown in Fig. 54 are recorded. The integrated data management device A collates recorded information of this login information table with the information inputted in step ST112 of Fig. 53. When the collation is successful, the graphical user interface UIa shown in Fig. 23 is outputted via the browser software.

In step ST113 in Fig. 53, the clinical information management device B10 receives the input of the test request information in step ST1 of Fig. 19 described in 8-1 above. Then, the clinical information management device B10 transmits the received test request information to the integrated data management device A.

### (2) Modified example of test request screen

In inputting the test request information in step ST1 of Fig. 19, information on a request source facility, information related to a doctor in charge, information regarding a bureau that leads the expert meeting, and the like may be recorded in advance in the integrated database OG. In step ST113 of Fig. 53, it is possible to output, to the corresponding area, information related to the information on the request source facility, the information regarding the doctor in charge, the information regarding the bureau that leads the expert meeting, and the like recorded in advance in the graphical user interface UIa shown in Fig. 23.

### (3) Modified example of test request ID assignment

In the master table M shown in Fig. 7, the "test request ID" may be individually issued by a numbering system owned by a facility that has received the test request, or may be issued at will of a person in charge of the facility that has received the test request.

### (4) Test status display process

In 8-2. above, a test progress status is described with, as an example, "sample receipt" and "pretreatment process completed" in steps ST61 to ST65 shown in Fig. 19, step ST27 shown in Fig. 19, step ST28 shown in Fig. 19, step ST29 shown in Fig. 20, and step ST30 shown in Fig. 20. However, the label inputted in the "test status" field shown in Fig. 3 can be updated at any time in accordance with progress of the test at the test facility C1 in Figs. 55 and 56. Labels that can be inputted in the "test status" field are shown in Fig. 25.

Fig. 55 shows an update process of the "test status" field of the master table M shown in Fig. 7, in the integrated data management A. Fig. 56 shows an update process of the "test status" field of the test management table L shown in Fig. 24, in the test information management device C11.

In step ST321 of Fig. 55, when the integrated data management A receives the test request information transmitted from the clinical information management device B10 (corresponding to step ST21 in Fig. 19), the integrated data management A updates the "test status" field of the master table M corresponding to the received test request to a "preparing sample" label, in step ST322 of Fig. 55.

In step ST323 of Fig. 55, when the integrated data management A receives information on sample shipment completion transmitted from the clinical information management device B10, the integrated data management A updates the "test status" field of the master table M corresponding to the received sample information to a "transporting sample" label, in step ST324 of Fig. 55. The information on sample shipment completion from the clinical information management device B10 may be inputted by the pathologist H1b or the clinical technologist H3 from the clinical information management device B10, from the input unit 106b of the clinical information management device B10 shown in Fig. 11.

When the sample is received at the test facility C1, the clinical technologist T1 inputs the information to the test information management device C11 via the input unit 106 in Fig. 13. In step ST421 of Fig. 56, the test information management device C11 receives, as the sample receipt information, input of a "sample receipt" label from the input unit 106 in Fig. 13 by the clinical technologist T1 (corresponding to step ST62 in Fig. 19). In step ST422 of Fig. 56, the test information management device C11 updates the "test status" of the test management table L shown in Fig. 24 to the "sample receipt" label (corresponding to step ST62 of Fig. 19). Subsequently, the test information management device C11 transmits the updated content to the integrated data management device A in step ST423 of Fig. 56 (corresponding to step ST63 of Fig. 19).

In step ST325 of Fig. 55, the integrated data management A receives the update information for the "sample receipt" label transmitted from the test information management device C11 as the sample receipt information (corresponding to step ST27 in Fig. 19). In step ST326 of Fig. 55, the integrated data management A updates the "test status" field of the master table M corresponding to the received sample information to the "sample receipt" label (corresponding to step ST28 of Fig. 19).

When pretreatment of the sample is started, the clinical technologist T1 inputs the information to the test information management device C11 via the input unit 106 in Fig. 13. In step ST424 of Fig. 56, the test information management device C11 receives the input from the input unit 106 of Fig. 13 by the clinical technologist T1 as sample pretreatment start information. In step ST425 of Fig. 56, the test information management device C11 updates the "test status" of the test management table L shown in Fig. 24 to a "pretreatment in progress" label. Subsequently, the test information management device C11 transmits the updated content to the integrated data management device A in step ST426 of Fig. 56.

In step ST327 of Fig. 55, the integrated data management A receives the update information for the "pretreatment in progress" label transmitted from the test information management device C11 as the pretreatment start information. In step ST328 of Fig. 55, the integrated data management A updates the "test status" field of the master table M corresponding to the received sample information to the "pretreatment in progress" label.

When the pretreatment of the sample is completed, the clinical technologist T1 inputs the information to the test information management device C11 via the input unit 106 in Fig. 13. In step ST427 of Fig. 56, the test information management device C11 receives the input from the input unit 106 of Fig. 13 by the clinical technologist T1 as sample pretreatment completion information (corresponding to step ST64 of Fig. 19). In step ST428 of Fig. 56, the test information management device C11 updates the "test status" of the test management table L shown in Fig. 24 to a "pretreatment completed" label (corresponding to step ST64 of Fig. 19). Subsequently, the test information management device C11 transmits the updated content to the integrated data management device A in step ST429 of Fig. 56 (corresponding to step ST65 of Fig. 19).

In step ST329 of Fig. 55, the integrated data management A receives the update information for the "pretreatment completed" label transmitted from the test information management device C11 as the pretreatment completion information (corresponding to ST29 of Fig. 20). In step ST330 of Fig. 55, the integrated data management A updates the "test status" field of the master table M corresponding to the received sample information to the "pretreatment completed" label (corresponding to ST30 of Fig. 20).

When sequencing is started, the clinical technologist T1 inputs the information to the test information management device C11 via the input unit 106 in Fig. 13. In step ST430 of Fig. 56, the test information management device C11 receives the input from the input unit 106 of Fig. 13 by the clinical technologist T1 as sequencing start information. In step ST431 of Fig. 56, the test information management device C11 updates the "test status" of the test management table L shown in Fig. 24 to a "sequencing in progress" label. Subsequently, the test information management device C11 transmits the updated content to the integrated data management device A in step ST432 of Fig. 56.

In step ST331 of Fig. 55, the integrated data management A receives the update information for the "sequencing in progress" label transmitted from the test information management device C11, as the sequencing start information. In step ST332 of Fig. 55, the integrated data management A updates the "test status" field of the master table M corresponding to the received sample information to the "sequencing in progress" label.

When the sequencing is completed, the clinical technologist T1 inputs the information to the test information management device C11 via the input unit 106 in Fig. 13. In step ST433 of Fig. 56, the test information management device C11 receives the input from the input unit 106 of Fig. 13 by the clinical technologist T1 as sequencing completion information. In step ST434 of Fig. 56, the test information management device C11 updates the "test status" of the test management table L shown in Fig. 24 to a "sequencing completed" label. Subsequently, the test information management device C11 transmits the updated content to the integrated data management device A in step ST435 of Fig. 56.

In step ST333 of Fig. 55, the integrated data management A receives the update information for the "sequencing completed" label transmitted from the test information management device C11, as the sequencing completion information. In step ST334 of Fig. 55, the integrated data management A updates the "test status" field of the master table M corresponding to the received sample information to the "sequencing completed" label.

When the sequencing is completed, the clinical technologist T1 inputs the information to the test information management device C11 via the input unit 106 in Fig. 13. In step ST433 of Fig. 56, the test information management device C11 receives the input from the input unit 106 of Fig. 13 by the clinical technologist T1 as sequencing completion information. In step ST434 of Fig. 56, the test information management device C11 updates the "test status" of the test management table L shown in Fig. 24 to a "sequencing completed" label. Subsequently, the test information management device C11 transmits the updated content to the integrated data management device A in step ST435 of Fig. 56.

In step ST333 of Fig. 55, the integrated data management A receives the update information for the "sequencing completed" label transmitted from the test information management device C11, as the sequencing completion information. In step ST334 of Fig. 55, the integrated data management A updates the "test status" field of the master table M corresponding to the received sample information to the "sequencing completed" label.

When the sequencing is completed, the test information management device C11 receives sequence information obtained by the sequencing from the next-generation sequencer C13. Then, the test information management device C11 starts mutation analysis. The clinical technologist T1 inputs the information to the test information management device C11 via the input unit 106 in Fig. 13. In step ST436 of Fig. 56, the test information management device C11 receives the input from the input unit 106 of Fig. 13 by the clinical technologist T1 as mutation analysis start information. In step ST437 of Fig. 56, the test information management device C11 updates the "test status" of the test management table L shown in Fig. 24 to a "mutation analyzing" label. Subsequently, the test information management device C11 transmits the updated content to the integrated data management device A in step ST438 of Fig. 56.

In step ST335 of Fig. 55, the integrated data management A receives the update information for the "mutation analyzing" label transmitted from the test information management device C11 as the mutation analysis start information. In step ST336 of Fig. 55, the integrated data management A updates the "test status" field of the master table M corresponding to the received sample information to the "mutation analyzing" label.

When the mutation analysis is completed, The clinical technologist T1 inputs the information to the test information management device C11 via the input unit 106 in Fig. 13. In step ST439 of Fig. 56, the test information management device C11 receives the input from the input unit 106 of Fig. 13 by the clinical technologist T1 as mutation analysis completion information. In step ST440 of Fig. 56, the test information management device C11 updates the "test status" of the test management table L shown in Fig. 24 to a "mutation analysis completed" label. Subsequently, the test information management device C11 transmits the updated content to the integrated data management device A in step ST441 of Fig. 56.

In step ST337 of Fig. 55, the integrated data management A receives the update information for the "mutation analysis completed" label transmitted from the test information management device C11 as the mutation analysis completion information. In step ST338 of Fig. 55, the integrated data management A updates the "test status" field of the master table M corresponding to the received sample information to the "mutation analysis completed" label.

If the clinical technologist T1 determines that quality of the nucleic acid extracted from the sample is poor, information indicating that it is necessary to recollect the sample, for example, a label of "sample reacquisition" may be recorded in the "test status" field of the master table M and the test management table L, in step ST427 of Fig. 56 (step ST64 of Fig. 19).

If for some reason the gene panel testing is stopped, information indicating that the test is stopped, for example, a label of "test stopped" may be recorded in the "test status" field of the master table M and the test management table L.

### (5) Output process for specific test request

In step ST45 shown in Fig. 22, the integrated data management device A can set access authority to the test request list area UI1 shown in Fig. 3 according to a user ID, a group ID, and a role ID, to limit display of the individual test request display area UI3 stored in the master table M in accordance with the ID. For one individual test request UI3, display of each area displayed in the test request list area UI1 can also be changed in accordance with the access authority.

For example, as shown in Fig. 10A, a group for holding an expert meeting is determined for each user, and each user has a predetermined role in the group.

An output process for a specific test request will be described with reference to Figs. 7, 10A and 10B, and 57.

In step ST701 of Fig. 57, the integrated data management device A receives a display request from each expert meeting terminal to the test request list area UI1 shown in Fig. 3.

In step ST702 of Fig. 57, the integrated data management device A identifies the user ID that has logged in to the system or the role ID of the user, from the role table CT that is shown in Fig. 10A and stored in the integrated database OG. As shown in the viewable information table AT in Fig. 10B, what information to display in the test request list is predetermined for each role ID.

In step ST703 of Fig. 57, the integrated data management device A refers to the master table M shown in Fig. 7. Then, the integrated data management device A identifies the group ID of the expert meeting to which the user belongs, based on the user ID.

In step ST704 of Fig. 57, the integrated data management device A refers to the master table M shown in Fig. 7. Then, the integrated data management device A identifies one or more individual test requests corresponding to the group ID, based on the group ID.

In step ST705 of Fig. 57, the integrated data management device A determines a common item and an additional item recorded in the viewable information table AT of Fig. 10B, based on the ID corresponding to the role identified in step ST702 of Fig. 57.

In step ST706 of Fig. 57, the integrated data management device A outputs, for each individual test request identified in step ST704 of Fig. 57, an item corresponding to the role ID corresponding to each user ID to the test request list area UI1.

Such a setting making it possible to avoid unnecessary display of information regarding a highly confidential gene mutation.

Whereas, when the "germline mutation" label is recorded in the second attribute information field, a medical person (such as a gene counselor or a doctor in charge) who participates in the expert meeting can access the patient IC information from the test request list area UI1 to check the patient IC information, a patient medical history, a family history, and the like. This enables an appropriate disclose method for a test result and information provision to the patient and the family of the patient when a germline mutation is detected.

### (6) Modified example of test request list

In the test request list shown in Fig. 3, the attribute information may be discernibly displayed for every attribute information. The "discernible" may be represented by changing a display color of a label for every attribute information. The attribute information may be represented by an identification symbol such as an alphabet such as "A", "G", and "O". In a display example, a reference symbol SM is represented in Fig. 3.

Specific attribute information (such as no mutation) may be displayed with a common symbol (for example, "!") for calling attention.

The identification by the common symbol for calling attention and the color of the label may be displayed when a label different from progress of a normal test is given to the "test status" of the test request list, for example, when "sample reacquisition", "test stop", or the like is recorded.

The test request list may be displayed in accordance with priority of the attribute information, by setting the priority in advance for every attribute information in the test request list.

### (7) Record of new expert meeting schedule slot

The bureau expert meeting terminal SP15 records a new schedule slot to be displayed in the dialog shown in Figs. 45 and 46, in the meeting schedule database SDB.

A staff of a facility that leads the expert meeting inputs a new meeting schedule slot from the input unit 106Z of the bureau expert meeting terminal SP15 shown in Fig. 15.

In step ST801 of Fig. 58, the bureau expert meeting terminal SP15 receives the input from the input unit 106Z shown in Fig. 15. At this time, the control unit 100Z of the bureau expert meeting terminal SP15 functions as the new reservation slot registration unit Z17 shown in Fig. 18.

In step ST802 of Fig. 58, the bureau expert meeting terminal SP15 transmits the information inputted in step ST801 of Fig. 58 to the integrated data management device A. At this time, the control unit 100Z of the bureau expert meeting terminal SP15 functions as the schedule update unit Z19 shown in Fig. 18. The transmitted information is recorded in the meeting schedule database SDB by the integrated data management device A.

### (8) Temporary reservation for meeting

In step ST235 shown in Fig. 43, the example has been described in which the setting of the expert meeting is confirmed by the doctor in charge H1a selecting the schedule or selecting the meeting notification icon UI77. However, as another embodiment, for example, the doctor in charge H1a may temporarily reserve the setting of the expert meeting by using the dialog UI5 shown in Fig. 45 before a result of the gene panel testing is obtained. With the temporary reservation, a label indicating "temporarily reserved" may be displayed in the "setting status" field of the test request list area UI1 in Fig. 59. The "temporarily reserved" label has a link to dialog UI7 shown in Fig. 46. When the doctor in charge H1a selects the "temporarily reserved" label, the dialog UI7 shown in Fig. 46 is displayed on the output unit 107X of the expert meeting terminal B 15 of the medical facility B 1. For confirming the reservation, the expert meeting is regularly set by the doctor in charge H1a selecting the meeting notification icon UI77, and the "set" label is displayed in the "setting status" field of the test request list area UI1. With the regular setting, the schedule of the expert meeting can be transmitted by mail to each participant in the expert meeting.

### (9) Modified example 1 of system 1000

In the above-mentioned embodiment, the integrated data management device A acquires test request information from the clinical information management devices B10, B20, and B30, and acquires the attribute information from the test information management device C11. However, these pieces of information may be acquired from a same computer. For example, the clinical information management devices B10, B20, and B30 may transmit the test request information to the test information management device C11, and the test information management device C11 may transmit the test request information and the attribute information to the integrated data management device A.

### (10) Modified example 2 of system 1000

The integrated data management device A may be a web server that provides cloud computing. The clinical information management device B10, the expert meeting terminal B 15, the clinical information management device B20, the expert meeting terminal B25, the clinical information management device B30, and the expert meeting terminal B35 may access the integrated data management device A, which is a web server, via a web browser. That is, the system 1000 may be a cloud computing system that does not require a dedicated application to access the integrated data management device A.

## Claims

1. A method for managing a test request for gene panel testing by a computer, the method comprising:
acquiring, for each of a plurality of test requests, information regarding the test request and attribute information of a test result in the gene panel testing; and
outputting display information for displaying a plurality of the test requests and the attribute information in association with each other.

2. The method according to claim 1, wherein
the acquiring comprises selecting the attribute information from a plurality of attribute candidates stored in advance.

3. The method according to claim 1 or 2, wherein
the acquiring comprises acquiring the information regarding the test request and the attribute information from at least one of other computers different from the computer that executes the method for managing, and
the outputting comprises integrating the plurality of the test requests and the attribute information that have been received from the other computer.

4. The method according to claim 3, wherein
the other computer comprises a first computer and a second computer,
the acquiring comprises acquiring the information regarding the test request from the first computer and acquiring the attribute information from the second computer.

5. The method according to claim 4, wherein
the first computer comprises a plurality of computers, and
the acquiring comprises acquiring the information regarding the test request, from each of the plurality of computers of the first computer.

6. The method according to claim 1 or 2, wherein
the computer that executes the method for managing comprises a web server that provides cloud computing, and
through a web browser of a computer that accesses the web server, acquisition of the information regarding the test request and the attribute information and outputting of the display information are executed.

7. The method according to any one of claims 1 to 6, wherein
the attribute information comprises at least one selected from information regarding presence or absence of a predetermined gene mutation in the test result, and information regarding a type of a predetermined gene mutation.

8. The method according to claim 7, wherein
the information regarding presence or absence of the predetermined gene mutation comprises information as to whether or not a mutation is detected, for at least one selected from a plurality of genes comprised in a test item of the gene panel testing.

9. The method according to claim 7 or 8, wherein
the information regarding the type of the predetermined gene mutation comprises information as to whether or not a gene mutation of a predetermined type is detected, for at least one selected from a plurality of genes comprised in a test item of the gene panel testing.

10. The method according to claim 9, wherein
the gene mutation of the predetermined type comprises at least one selected from an actionable mutation and a germline mutation.

11. The method according to any one of claims 1 to 10, wherein
the display information comprises information for displaying the attribute information in a display format that enables identification of a type of the attribute information.

12. The method according to claim 11, wherein
the display format that enables identification is a display format that is for identification with a color according to a type of attribute information, a common symbol, or a label that attracts viewer's attention.

13. The method according to any one of claims 1 to 12, wherein
the display information comprises the attribute information to be subjected to a list display corresponding to the test request.

14. The method according to claim 13, further comprising:
receiving a display format setting based on the attribute information, and wherein
the outputting comprises outputting display change information for change of the list display based on the received setting.

15. The method according to claim 14, wherein
the display format setting based on the attribute information is a setting for rearrangement of a plurality of test requests in accordance with a type of the attribute information.

16. The method according to claim 15, wherein
the display format setting based on the attribute information is a setting for extraction and display of attribute information of a predetermined type.

17. The method according to any one of claims 1 to 15, further comprising
acquiring test result information corresponding to the test request, and wherein
the display information comprises link information for reception of a display request for the test result information.

18. The method according to any one of claims 1 to 17, wherein
the display information comprises link information for reception of a display request of at least one information database selected from a drug information database, a clinical trial information database, and an article information database.

19. The method according to any one of claims 1 to 18, further comprising
acquiring quality information of a sample and/or a test related to the test request, and wherein
the display information comprises link information for reception of a display request for the acquired quality information.

20. The method according to any one of claims 1 to 19, further comprising
acquiring informed consent information of a patient, the informed consent information comprising whether or not to desire to be informed of germline mutation information and being related to the test request, and wherein
the display information comprises link information for reception of a display request for the acquired informed consent information.

21. The method according to claim 20, wherein
the display information comprises link information for reception of a change request for display of germline mutation information in the attribute information.

22. The method according to claim 20 or 21, further comprising
acquiring test result information related to the test request, and wherein
the display information comprises link information for reception of a selection request for a presentation format of information regarding the germline mutation.

23. The method according to any one of claims 1 to 22, further comprising
acquiring progress information of a test related to the test request, and wherein
the display information comprises link information for reception of a display request for the acquired progress information.

24. The method according to any one of claims 1 to 23, further comprising
acquiring information regarding an expert meeting for interpretation of genetic information by a plurality of medical persons, the information being related to the test request, and wherein
the display information comprises link information for reception of a display request for the acquired information regarding the expert meeting.

25. The method according to claim 23, wherein
the display information comprises link information for reception of a request for schedule setting of the expert meeting.

26. A management device for managing a test request for gene panel testing, the management device comprising:
a control unit, wherein
the control unit
acquires, for each of a plurality of test requests, information regarding the test request and attribute information of a test result in the gene panel testing; and
outputs display information for displaying a plurality of the test requests and the attribute information in association with each other.

27. The management device according to claim 26, wherein
the management device is a web server that provides cloud computing, and
through a web browser of a computer that accesses the web server, acquisition of information regarding the test request and the attribute information and outputting of the display information are executed.

28. A non-transitory computer-readable storage medium storing a computer program for managing a test request for gene panel testing, the computer program, which when read and executed, causes a computer to perform operations comprising:
acquiring, for each of a plurality of test requests, information regarding the test request and attribute information of a test result in gene panel testing corresponding to the test request, and
outputting display information for displaying the plurality of the test requests and the attribute information in association with each other.

29. A management system for managing a test request for gene panel testing, the management system comprising:
a first computer comprising a control unit;
a second computer comprising a control unit; and
a management device comprising a control unit,
wherein
the control unit of the first computer transmits information regarding a plurality of test requests to the management device,
the control unit of the second computer transmits attribute information of a test result in the gene panel testing to the management device, and
the control unit of the management device outputs display information for displaying a plurality of the test requests and the attribute information that have been received, in association with each other.

30. The management system according to claim 29, wherein
the control unit of the second computer comprises a storage unit that stores a plurality of attribute candidates, and
the attribute information selected from a plurality of the stored attribute candidates is transmitted to the management device.

31. The management system according to claim 29 or 30, wherein
the first computer comprises a plurality of computers, and
the control unit of the management device acquires information regarding the test request from each of the plurality of computers of the first computer.
